# EUROPEAN PATENT SPECIFICATION

(11) **EP 1 224 186 B1**
(45) Date of publication and mention of the grant of the patent: **24.09.2003**
(21) Application number: 00974002.8
(22) Date of filing: 27.10.2000
(51) Int. Cl.: C07D 471/10, A61K 31/438, A61P 9/00, C07D 413/14, C07D 401/04

(54) **PYRIDYL-CONTAINING SPIROCYCLIC COMPOUNDS AS INHIBITORS OF FIBRINOGEN-DEPENDENT PLATELET AGGREGATION**
PYRIDYL ENTHALTENDE SPIROCYCLISCHE VERBINDUNGEN ALS INHIBITOREN DER FIBRINOGEN-ABHÄNGIGEN BLUTPLÄTTCHEN AGGREGATION
COMPOSES SPIROCYCLIQUES PYRIDILES INHIBITEURS DE L'AGREGATION PLAQUETTAIRE DEPENDANT DES FIBRINOGENES

(30) Priority: 27.10.1999 US 161825 P
(43) Date of publication of application: 24.07.2002
(73) Proprietor: Millennium Pharmaceuticals, Inc., Cambridge, Massachusetts 02139 (US)
(72) Inventor: SCARBOROUGH, Robert, M., Half Moon Bay, CA 94019 (US); MEHROTA, Mukund, South San Francisco, CA 94080 (US); PANDEY, Anjali, Fremont, CA 94555 (US); SMYTH, Mark, Foster City, CA 94404 (US)
(74) Representative: Leidescher, Thomas, Dr.
(86) International application number: US0029804
(87) International publication number: WO01030780

(56) References cited:
- WO-A-97/11940

## Description

This invention relates to novel substituted or unsubstituted pyridyl-containing spirocyclic compounds useful as glycoprotein IIb/IIIa antagonists for the prevention of thrombosis.

### Background of the Invention

The most prevalent vascular disease states are related to platelet dependent narrowing of the blood supply such as atherosclerosis and arteriosclerosis, acute myocardial infarction, chronic stable angina, unstable angina, transient ischemic attacks and strokes, peripheral vascular disease, arterial thrombosis, preeclampsia, embolism, restenosis following angioplasty, carotid endarterectomy, anastomosis of vascular grafts, and etc. These conditions represent a variety of disorders thought to be initiated by platelet activation on vessel walls.

Platelet adhesion and aggregation is believed to be an important part of thrombus formation. This activity is mediated by a number of platelet adhesive glycoproteins. The binding sites for fibrinogen, fibronectin and other clotting factors have been located on the platelet membrane glycoprotein complex IIb/IIIa. When a platelet is activated by an agonist such as thrombin the GPIIb/IIIa binding site becomes available to fibrinogen, eventually resulting in platelet aggregation and clot formation.

Heretofore it has been proposed to block these thrombus formation sites by the use of various therapeutic agents.

There is a need in the area of cardiovascular and cerebrovascular therapeutics for new agents which can be used in the prevention and treatment of thrombi.

It is a discovery of this invention that certain novel spirocyclic compounds block the GPIIb/IIIa fibrinogen receptor, thereby inhibiting platelet aggregation and subsequent thrombus formation. Pharmaceutical formulations containing the spirocyclic compounds of this invention inhibit aggregation and are useful for the prophylaxis and treatment of thrombogenic diseases, such as myocardial infarction, angina, stroke, peripheral arterial disease, disseminated intravascular coagulation and venous thrombosis.

WO 97/11940 discloses inhibitors of fibrinogen-dependent platelet aggregation which are spiro compounds carrying an acidic side chain. Furthermore, the bicyclic spiro core structure may be substituted by a pyridinyl group which is attached via an alkylene spacer.

### Summary of the Invention

The present invention covers novel spirocyclic compounds having a spirocyclic nucleus formed from two fused rings sharing a common central carbon atom, which are shown in rings A and B in the formula (I), as hereinafter defined, and all pharmaceutically-acceptable salts, solvates and prodrug derivatives thereof: 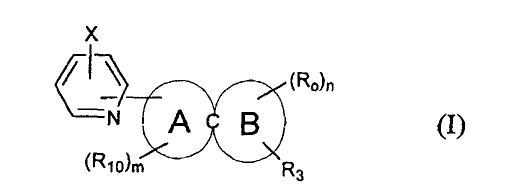 having substituents and subscripts; X, R₁₀, m, n, R₀ and R₃, as hereinafter defined.

Another aspect of the invention is a pharmaceutical formulation containing a novel spirocyclic compound of the invention.

Another aspect of the invention is a method of inhibiting platelet aggregation, inhibiting fibrinogen binding, or preventing thrombosis by administering to a mammal the novel spirocyclic compounds of the invention.

Another aspect of this invention is a method of treating a human to alleviate the pathological effects of atherosclerosis and arteriosclerosis, acute myocardial infarction, chronic stable angina, unstable angina, transient ischemic attacks and strokes, peripheral vascular disease, arterial thrombosis, preeclampsia, embolism, restenosis following angioplasty, carotid endarterectomy, and anastomosis of vascular grafts; wherein the method comprises administering to said human a therapeutically-effective amount of a novel spirocyclic compound of this invention.

### Detailed Description of the Invention

### Definitions:

The term "spirocyclic" refers to a compound consisting of two rings having only one carbon atom in common. Spiropentane is an exemplary compound having a spirocyclic system. Spirocyclic systems exclude other bicyclic compounds such as naphthalene which have two or more carbon atoms in common.

The term "alkyl" used herein refers to a monovalent straight or branched chain radical of from one to ten carbon atoms, including, but not limited to methyl, ethyl, n-propyl, isopropyl, n-butyl, isobutyl, tert-butyl, n-hexyl, and the like.

The term "halosubstituted alkyl" as used herein refers to an alkyl group as just defined, substituted by one, two or three halogen atoms selected from fluorine, chlorine, bromine, and iodine. Examples of such groups include chloromethyl, bromoethyl, trifluoromethyl, and the like.

The term "aryl" when used alone means a homocyclic aromatic radical whether or not fused. Aryl groups preferably comprise five to eighteen carbons and preferred aryl groups include phenyl, naphthyl, biphenyl, phenanthrenyl, naphthacenyl, and the like.

The term "substituted aryl" denotes an aryl group substituted with one, two, or three substituents chosen from halogen, hydroxy, protected hydroxy, cyano, nitro, C₁-C₁₀ alkyl, C₁-C₁₀ alkoxy, trifluoromethyl, amino, aminomethyl, and the like. Examples of such groups are 4-chlorophenyl, 2-methylphenyl, 3-methyl-4-hydroxyphenyl, and 3-ethoxyphenyl,

The term "arylalkyl" means one, two or three aryl groups having the number of carbon atoms designated, appended to an alkyl radical having the number of carbon atoms designated. A typical arylalkyl group is the benzyl group.

The term "alkenyl" as used herein refers to a monovalent straight or branched chain radical of from two to six carbon atoms containing a carbon double bond including, but not limited to, 1-propenyl, 2-propenyl, 2-methyl-1-propenyl, 1-butenyl, 2-butenyl and the like.

The term "alkylene" as used herein refers to a divalent straight or branched chain group of from one to ten carbon atoms, including but not limited to, -CH₂-, -(CH₂)₂-. -(CH₂)₃-, -CH(CH₃)-, -CH(C₂H₅)-, -CH(CH₃)CH₂-, and the like.

The term "alkenylene" as used herein refers to a divalent straight or branched chain group of from two to ten carbon atoms containing a carbon-carbon double bond, including but not limited to, -CH=CH-, -C(CH₃)=CH-, CH=CH-CH₂-, -CH=C(CH₃)-CH₂-, -CH₂CH(CH=CH₂)CH₂, and the like.

The term "alkynylene" as used herein refers to a divalent straight or branched chain group of from two to ten carbon atoms containing a carbon-carbon triple bond, including but not limited to, 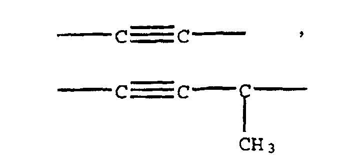 and the like.

The term "alkoxy" used herein refers to a monovalent straight or branched chain radical of from one to six carbon atoms linked through an oxygen atom, including, but not limited to methoxy, ethoxy, n-propoxy, isopropoxy, n-butoxy, isobutoxy, tert-butoxy, and the like.

The term "cycloalkyl" used herein refers to a non-aromatic ring structure of from three to eight carbon atoms, including, but not limited to cyclobutyl, cyclopentyl, cyclohexyl, and the like.

The term "alkynyl" refers to a monovalent straight or branched chain group of from two to six carbon atoms containing a carbon-carbon triple bond, including but not limited to, -C≡CH, -CH₂-CH₂-C=CH, -CH₂-C≡C-CH₂-CH₃, and the like.

The term "aralkoxy" refers to a monovalent radical in which an aryl or substituted aryl group, as defined above, is linked through an oxygen atom, including, but not limited to phenoxy, naphthoxy, -O-(C₆H₄)-CH₃, and the like.

The term "substituted amino" as used herein refers to an amino group in which one or more hydrogens are substituted with alkyl, halosubstituted alkyl, aryl, substituted aryl, alkenyl, or alkynyl groups, as these groups are defined above.

The term "carbamoyl" refers to an aminocarbonyl group, also called carbamyl, in which the amino portion is either substituted amino or unsubstituted amino.

The term "acyl" refers to a group having the structure: 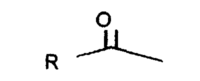 where R is alkyl, halosubstituted alkyl, aryl, substituted aryl, alkenyl, or alkynyl, as defined above.

The term "acid radical" refers to an organic radical which is a proton donor. Illustrative acid radicals include; 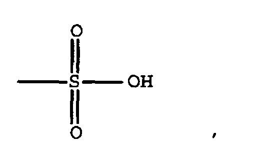 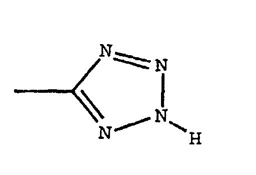 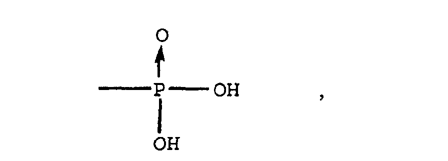 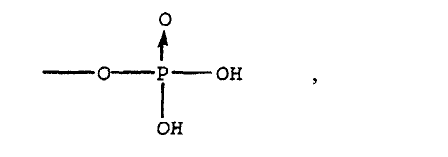 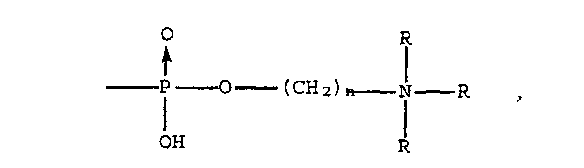 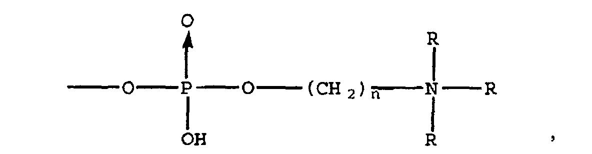 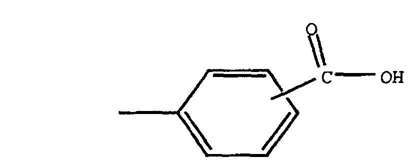 and.

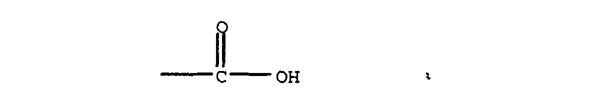

The term "acidic group" is an organic group containing one or more acid radicals. An acidic group may comprise only an acid radical.

The term "non-interfering substituent" refers to an organic radical which does not significantly reduce the therapeutic effectiveness of a compound.

### Compounds of the Invention:

This invention provides compounds of the general formula (I), or a pharmaceutically-acceptable salt, solvate or or prodrug thereof: 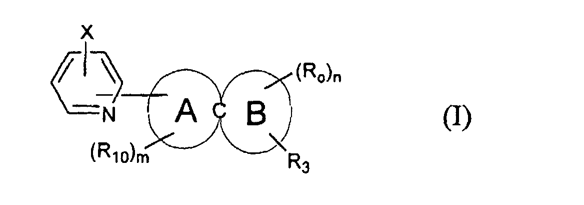 wherein;
the spirocycle nucleus A/B is a member selected from the group consisting of; 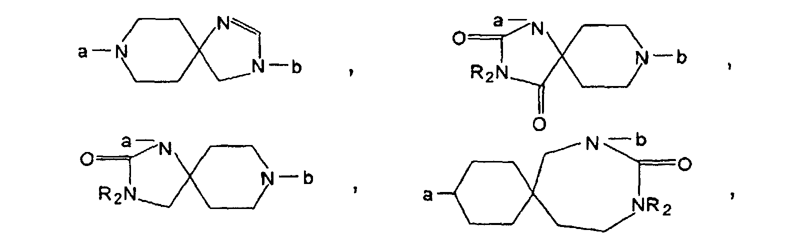 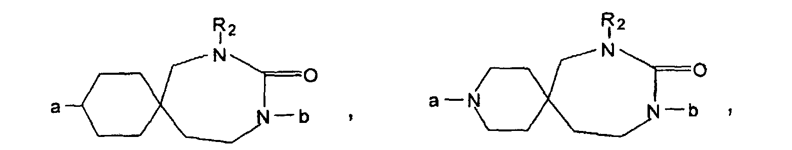 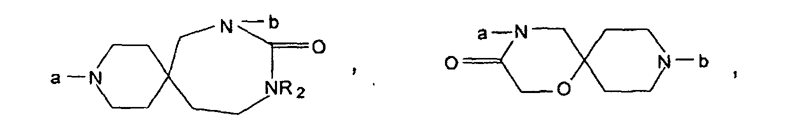 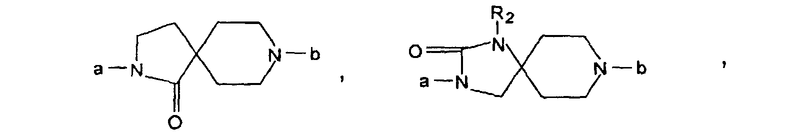 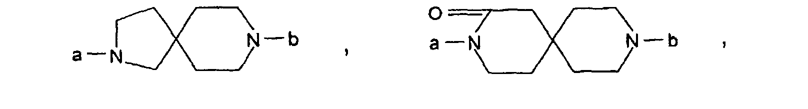 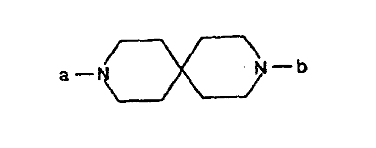 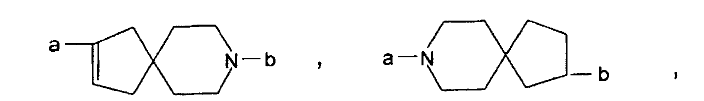 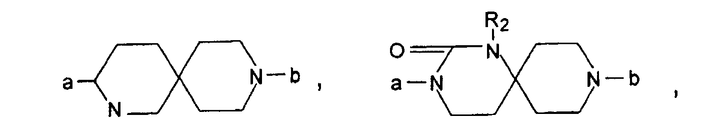 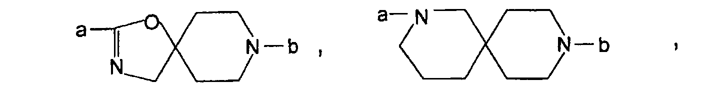 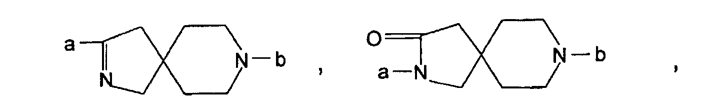 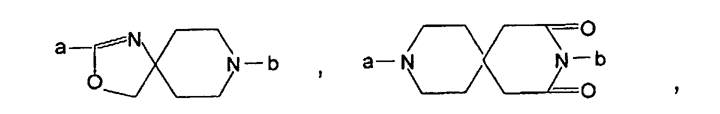 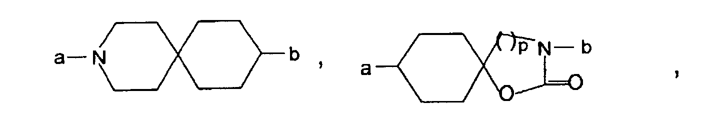 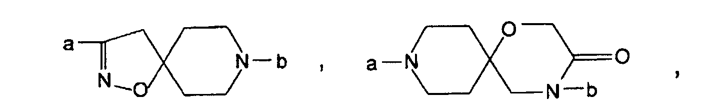 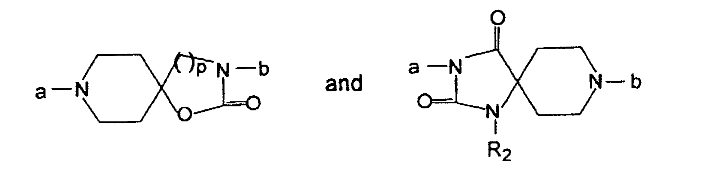 wherein
p is the number from 1 to 2,
one of the "a" and "b" attachment points of the spirocyclic nucleus is attached to a carbon of the pyridyl group while the other is attached to the R₃ group, and
R₂ is a R₁₀ group when the "a" attachment point is attached to the pyridyl group, otherwise R₂ is a R₀ group when the "b" attachment point of the spirocyclic nucleus is attached to the pyridyl group;
R₁₀ is the same or different and is a non-interfering substituent independently selected from hydrogen, alkyl, halosubstituted alkyl, alkenyl, alkynyl, cycloalkyl, aryl, arylalkyl, hydroxy, alkoxy, arylalkoxy, amino, substituted amino, carbamoyl, carboxy, acyl, cyano, halo, nitro, sulfo, =O, or =S, with the proviso that only one R₁₀ may be =O or =S;
m is a number from zero to 9;
R₀ is the same or different and is a non-interfering substituent independently selected from hydrogen, alkyl, halosubstituted alkyl, alkenyl, alkynyl, cycloalkyl, aryl, arylalkyl, hydroxy, alkoxy, arylalkoxy, amino, substituted amino, carbamoyl, carboxy, acyl, cyano, halo, nitro, sulfo, =O, or =S, with the proviso that only one R₀ may be =O or = S;
n is a number from zero to 9;
X is a substituent selected from the group consisting of hydrogen, halo, -C₁₋₆alkyl, C₁₋₆alkoxy, C₂₋₆alkenyl, C₂₋₆alkynyl, C₃₋₈cycloalkyl, C₀₋₄alkylC₃₋₈cycloalkyl, -CN, -NO₂, -(CH₂)ⱼ-N(-R^{a},-R^{b}), -C(=O)-N(-R^{a},-R^{b}), -S(=O)₂-N(-R^{a},-R^{b}), -S(=O)₂-R², -CF₃, and -(CH₂)ⱼ-O-R^{a}; wherein
R^{a} and R^{b} are independently selected from the group consisting of H, -C₁₋₆alkyl, C₁₋₆alkoxy, C₂₋₆alkenyl, C₂₋₆alkynyl, C₃₋₈cycloalkyl, C₀₋₆alkylC₃₋₈cycloalkyl, and -C₀₋₆alkyl-(carbocyclic aryl), wherein from 0-4 hydrogen atoms on the ring atoms of the carbocyclic aryl moiety may be independently replaced with a member selected from the group consisting of halo, C₁₋₄alkyl, C₂₋₆alkenyl, C₂₋₆alkynyl, C₃₋₈cycloalkyl, C₀₋₄alkylC₃₋₈cycloalkyl, -CN, -CF₃ and -NO₂; and
j is an integer of 0-2; and
R₃ is an acidic group containing one or more acid radicals.

A preferred group of the above compounds have the following spirocylic nuclei: 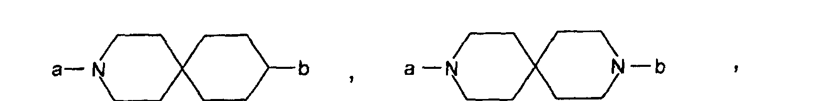 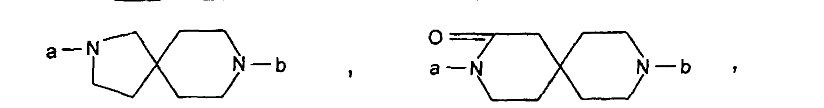 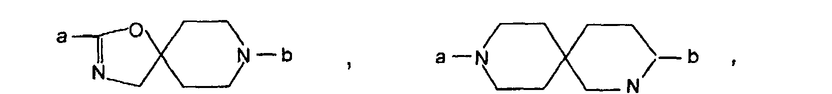 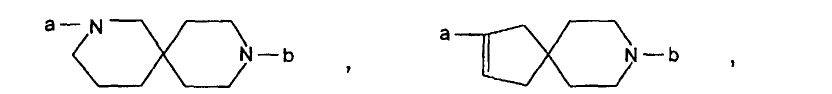 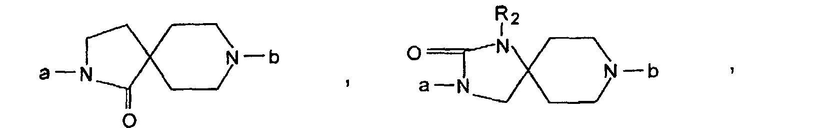 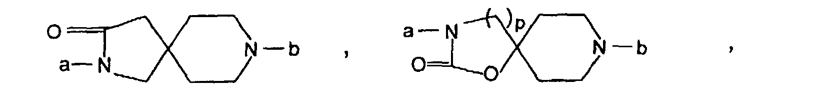 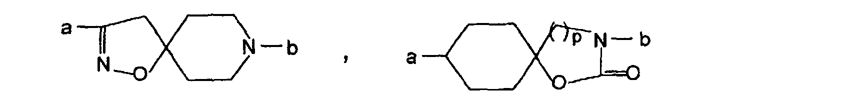 and 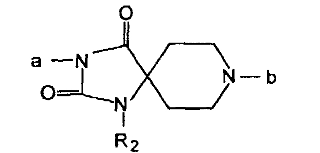 wherein
p is the number from 1 to 2,
one of the "a" and "b" attachment points of the spirocyclic nucleus is attached to the pyridyl group while the other is attached to the R₃ group, and
R₂ represents either a R₁₀ or a R₀ group depending on whether the "a" attachment point or the "b" attachment point of the spirocyclic nucleus is attached to the pyridyl group.

A second preferred group of the above compounds have the following spirocylic nuclei: 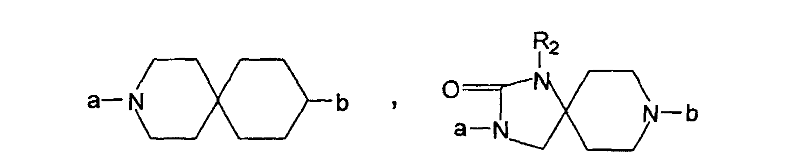 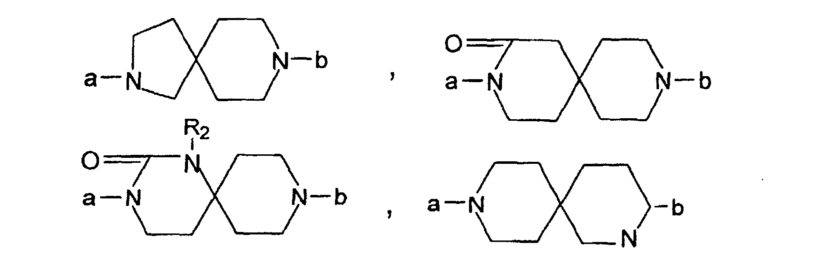 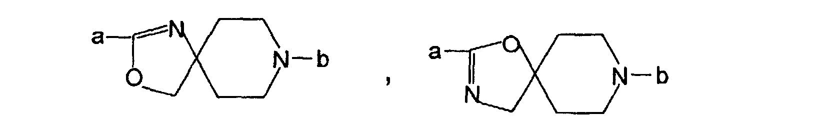 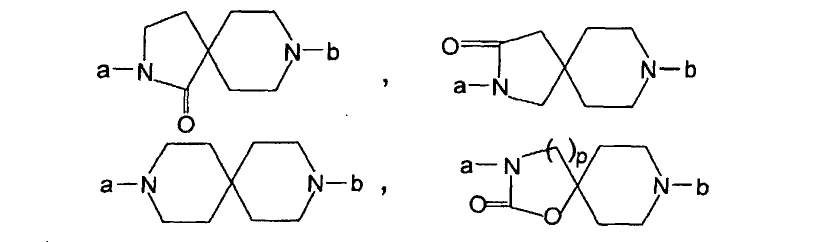 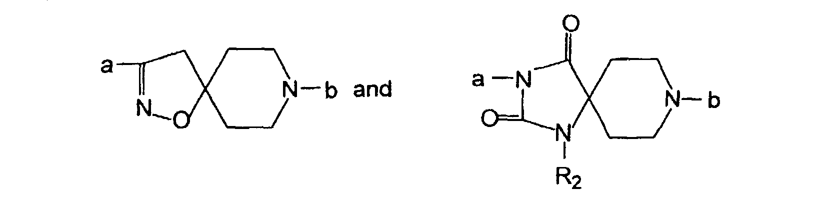 p is the number from 1 to 2,
one of the "a" and "b" attachment points of the spirocyclic nucleus is attached to the pyridyl group while the other is attached to the R₃ group, and
R₂ represents either a R₁₀ or a R₀ group depending on whether the "a" attachment point or the "b" attachment point of the spirocyclic nucleus is attached to the pyridyl group.

Further such preferred compounds have the following spirocyclic nuclei: 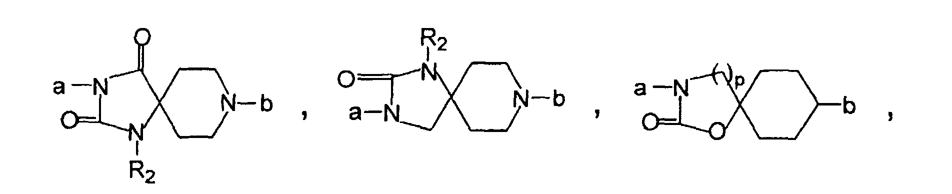 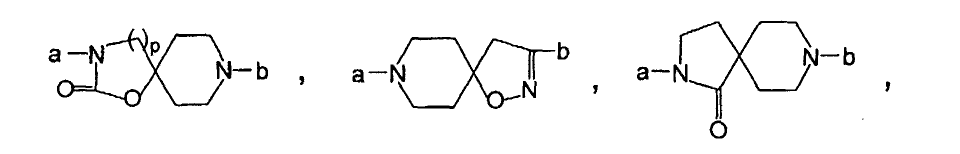 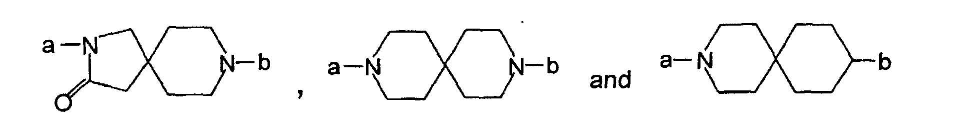 wherein the substituents and attachments are as discussed above. In one most preferred embodiment, the "a" attachment point is attached to the pyridyl group. In a second most preferred embodiment, the "b" attachment point is attached to the pyridyl group.

### The Pyridyl Substituents:

In a preferred embodiment a bond which directly links a carbon of the pyridyl substituent to a nitrogen atom of the spirocyclic nucleus.

A preferred pyridyl subsituent of formula (I) is a group of the following formula: 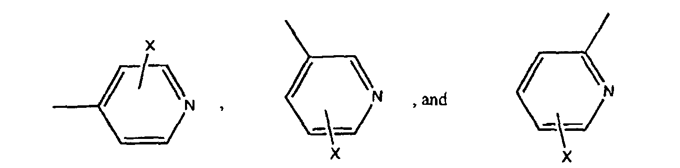 wherein X is a member selected from the group consisting of hydrogen, lower alkyl, halogen and trihalomethyl.

In an especially preferred embodiment, the pyridyl subsituent of formula (I) is of the formula below and is linked directly to a nitrogen atom on the spirocylic nucleus: 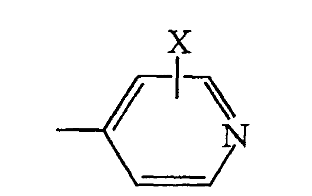 wherein X is a member selected from the group consisting of hydrogen, lower alkyl, halogen and trihalomethyl. Even more preferred is such a pyridyl radical wherein X is a hydrogen atom. For example in compounds having a 4-(4-pyridyl)diazaspiro-1-yl, moiety the ring nitrogen in the diazaspiro nucleus is believed to contribute to the ability of the nitrogen atom in the pyridyl group to function as a base as shown below:

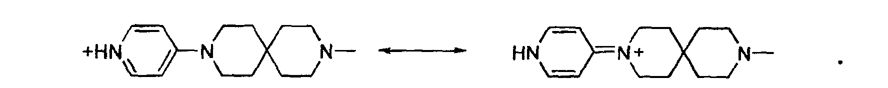

Thus, such compounds of the invention are preferred wherein the spirocyclic nucleus is a member selected from the group consisting of 6,6-diaza, 6,6-aza, 6,5-diaza and 5,6-diaza, and the like.

### The Acidic Substituent R₃

The substituent R₃ of formula (I) is an acidic group. An acidic group contains one or more acidic radicals. Suitable acidic radicals contain one or more proton donors, and include groups such as sulfonic acids, tetrazoles, phosphonic acids, carboxylic acids, and the like. The acidic radical may be bound to an aryl group, such as phenyl or substitued phenyl, or bound to alkyl chains, such as methylene. These groups may also be bound to the spirocyclic nucleus through alkyl chains having heteroatoms, such as S, O, or N, and amide (CONH) or carbonyl (CO) groups. The acidic substituent may also comprise an α-sulfonamido carboxylic acid group of the formula:

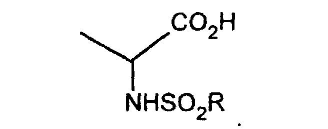

In preferred embodiments, R₃ is CO₂R₅, (C₁-C₆ alkyl)CO₂R₅, CO(C₁-C₆ alkyl)CO₂R₅, or CONH(C₁-C₆ alkyl)CO₂R₅, (C₁-C₆ alkyl)CH(NHR₄)CO₂R₅, CO(C₁-C₆ alkyl)CH(NHR₄)CO₂R₅, or CONH(C₁-C₆ alkyl)CH(NHR₄)CO₂R₅, wherein R₄ is SO₂(C₁-C₆ alkyl), SO₂ aryl, or SO₂ substituted aryl, and R₅ is hydrogen, C₁-C₆ alkyl, aryl, or substituted aryl.

### Preferred Spirocyclic Compounds:

General formulae and compound species for a preferred embodiment according to the present invention include the following pyridyl substituted spirocyclic compound formulae: 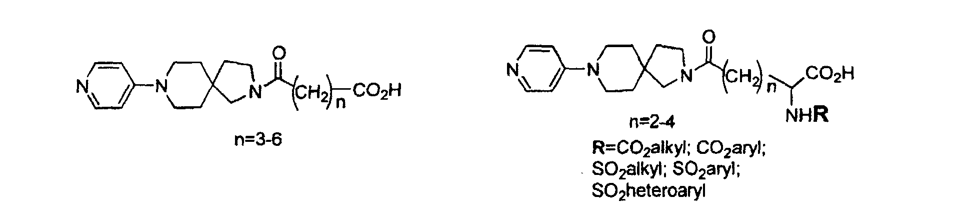 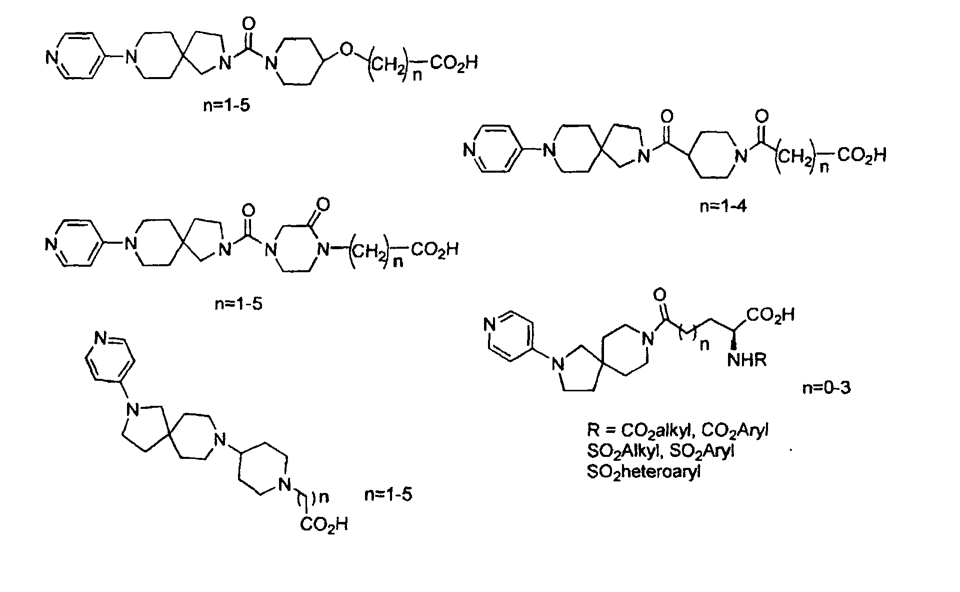 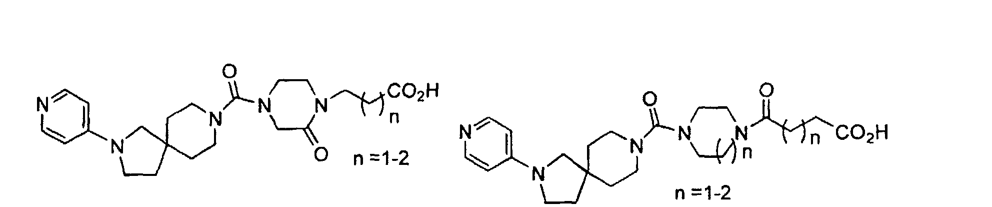 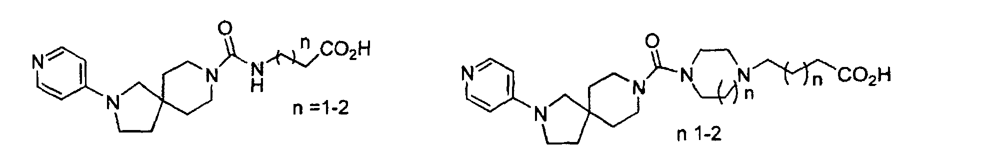 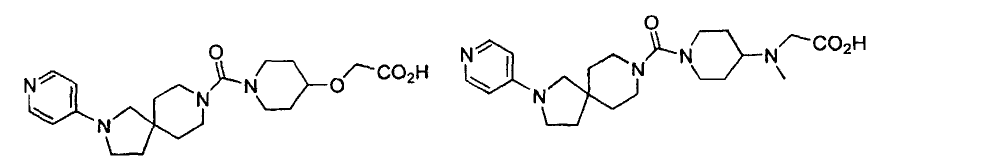 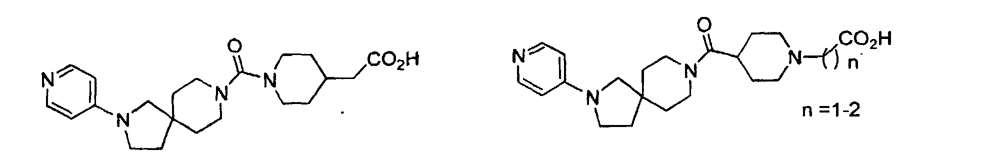 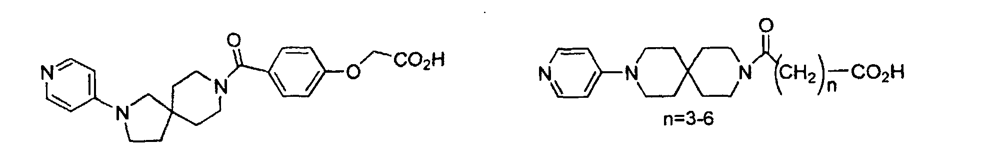 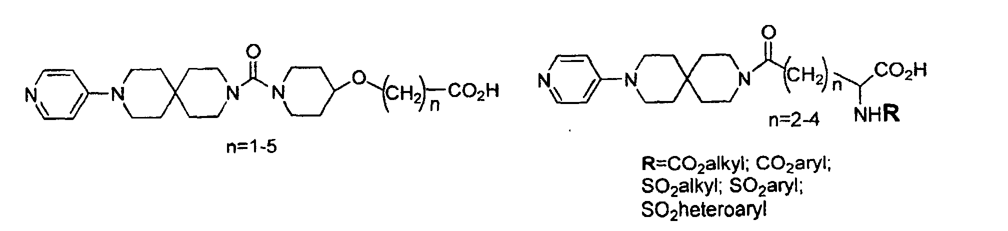 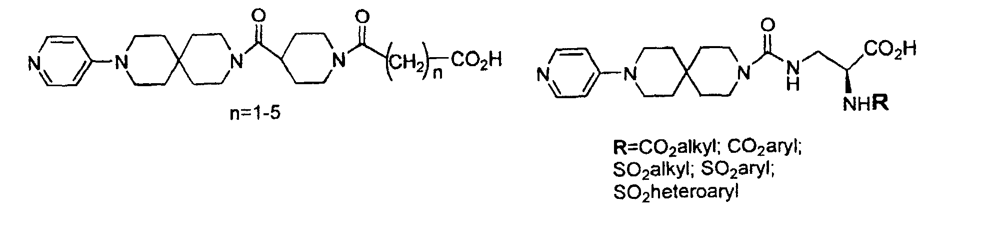 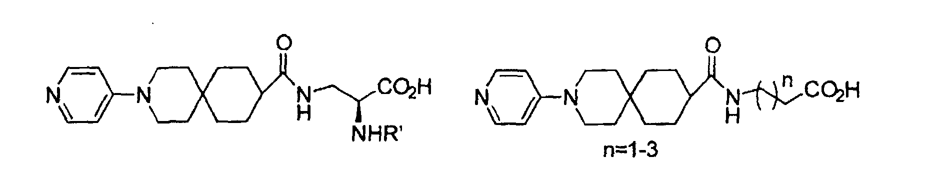 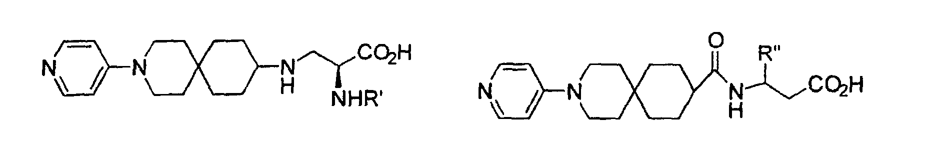
R'=CO₂alkyl; CO₂aryl;
SO₂alkyl; SO₂aryl;
SO₂heteroaryl
R"= akyl; aryl; heteroaryl

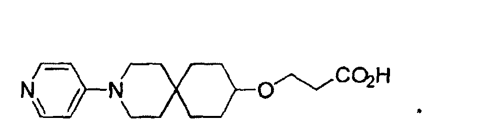

Also contemplated for such structures are their corresponding pharmaceutically acceptable salts, solvates, prodrug derivatives, and pharmaceutical compositions comprising such compounds in combination with at least one pharmaceutically acceptable carriers or excipient.

The compounds of the invention possess at least one acidic functional substituent (viz., R₃ of Formula I) and, as such, are capable of forming salts. Representative pharmaceutically-acceptable salts include, but are not limited to, salts with alkali and alkaline earth metals such as lithium, sodium, potassium, calcium, magnesium, aluminum and the like. Salts are conveniently prepared from the free acid by treating the acid in solution with a base or by exposing the acid to an anion exchange resin on the salt cycle.

Included within the definition of pharmaceutically-acceptable salts are the relatively non-toxic, inorganic and organic base addition salts of compounds of the present invention, for example, ammonium, quaternary ammonium, and amine actions, derived from nitrogenous bases of sufficient basicity to form salts with the compounds of this invention (see, for example, S. M. Berge, et. al., "Pharmaceutical Salts," J. Phar. Sci., 66: 1-19(1977)).

The basic portion of the compounds of the invention (viz., part Q of formula I or II) may be reacted with suitable organic or inorganic acids to form salts of the invention. Representative salts include those selected from the group comprising; acetate, benzenesulfonate, benzoate, bicarbonate, bisulfate, bitartrate, borate, camsylate, carbonate, chloride, clavulanate, dihydrochloride, edetate, edisylate, estolate, esylate, fumarate, gluceptate, gluconate, glutamate, glycollylarsanllate, hexylresorcinate, hydrabamine, hydrobromide, hydrochloride, hydroxynaphthoate, iodide, isothionate, lactate, lactobionate, laurate, malate, malseate, mandelate, mesylate, methylbromide, methylnitrate, methylsulfate, mucate, napsylate, nitrate, oleate, oxalate, palmitate, pantothenate, phosphate, polygalacturonate, salicylate, stearate, subacetate, succinate, tannate, tartrate, tosylate, trifluoroacetate, trifluoromethane sulfonate, and valerate.

The compounds of the formula (I) or (II) can also be in the form of zwitterions, since they contain both acidic and basic functionality and are capable of self-protonation.

Certain compounds of the invention possess one or more chiral centers and may thus exist in optically active forms, or as mixtures of diastereomers. Likewise, when the compounds contain an alkenyl or alkenylene group there exists the possibility of *cis-* and *trans-* isomeric forms of the compounds. The *R-* and *S*- isomers and mixtures thereof, including racemic mixtures as well as mixtures of *cis-* and *trans-* isomers, are contemplated by this invention. Additional asymmetric carbon atoms can be present in a substituent group such as an alkyl group. All such isomers as well as the mixtures thereof are intended to be included in the invention. If a particular stereoisomer is desired, it can be prepared by methods well known in the art by using stereospecific reactions with starting materials which contain the asymmetric centers and are already resolved or, alternatively by methods which lead to mixtures of the stereoisomers and subsequent resolution by known methods.

### Prodrug Derivatives of Compounds of the Invention:

Prodrugs are derivatives of the compounds of the invention which have metabolically cleavable groups and become by solvolysis or under physiological conditions the compounds of the invention which are pharmaceutically active *in* vivo. For example, ester derivatives of compounds of this invention are often active in vivo, but not in vitro. Other derivatives of the compounds of this invention have activity in both their acid and acid derivative forms, but the acid derivative form often offers advantages of solubility, tissue compatibility, or delayed release in the mammalian organism (see, Bundgard, H., Design of Prodrugs, pp. 7-9, 21-24, Elsevier, Amsterdam 1985). Prodrugs include acid derivatives well known to practitioners of the art, such as, for example, esters prepared by reaction of the parent acid with a suitable alcohol, or amides prepared by reaction of the parent acid compound with an amine. Simple aliphatic or aromatic esters derived from acidic groups pendant on the compounds of this invention are preferred prodrugs. In some cases it is desirable to prepare double ester type prodrugs such as (acyloxy)alkyl esters or ((alkoxycarbonyl)oxy)alkyl esters.

Preferred are the C₁-C₈ alkyl, C₂-C₈ alkenyl, aryl, C₇-C₁₂ substituted aryl, and C₇-C₁₂ arylalkyl esters of the compounds of the invention (per formula I or II) Particularly preferred are the C₁-C₄ alkyl esters, for example, where the R₃ acidic group has been esterified to form a group represented by one of the following formulae:

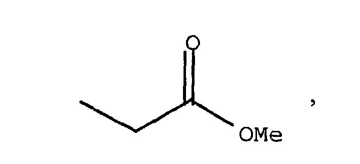

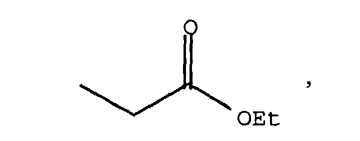

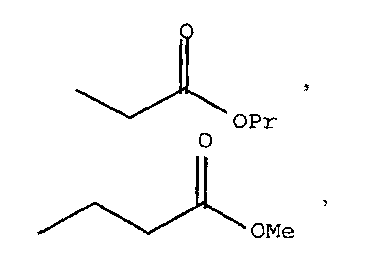

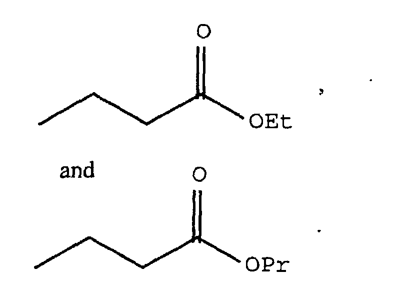

### Preparation of Spirocyclic Compounds:

The synthesis of spirocyclic compounds covered by the invention is described in Scheme 1 thru Scheme 25, in which the following terms are used:
P means a general protective group for amines like benzyl, tert.-butoxycarbonyl, benzyloxycarbonyl, or ethoxycarbonyl.
X, when present, is a spacer typically consisting of a chain of up to three carbon atoms, e.g. methylene, dimethylene, or trimethylene.

### Scheme Nomenclature:

The substituent R is a non-interfering substituent illustrated by an alkyl group selected from ethyl, methyl, or tert.-butyl forming esters containing the group COOR, which are cleaved to the corresponding carboxylic acids (R = H).

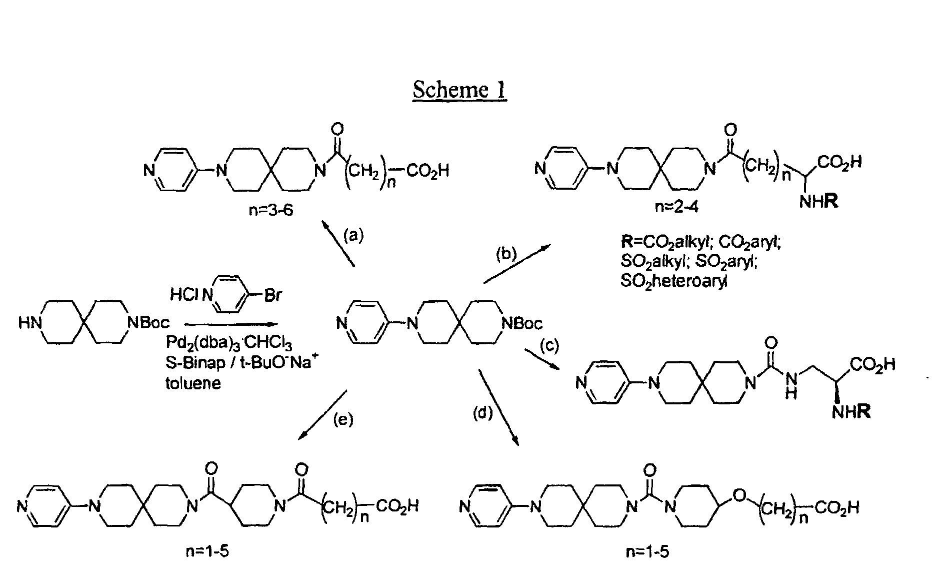

Scheme 1 describes the synthesis of 9-(4-pyridyl)-3,9-diazaspiro[5.5]undecane containing compounds derived from *tert*-butyl 3,9-diazaspiro[5.5]undecane-3-carboxylate which is prepared according to a published procedure (US Patent #5,451,578). The coupling of the diazaspirocycle to 4-bromopyridine is accomplished utilizing a Pd-catalyzed reaction that is well known in the literature (Buckwald, S.L., *et al.* J. Org. Chem. **1996**, 61, 7240). Subsequent coupling of this key intermediate as described in Paths a-c affords a variety of different targets. Path "a" demonstrates targets derived from removal of the Boc-group followed by acylation with an appropriate ester-acid chloride such as ethyl glutaryl chloride. Subsequent mild acid or base hydrolysis of the ester affords the desired targets. Path "b" demonstrates targets containing a sulfonylated or carbamoylated amino group α to the terminal acid functionality. Coupling of an appropriate acid-α-amino-ester side chain to the spirocyclic template is accomplished using standard peptide coupling agents such as HBTU or BOP-Cl. Mild acid or base hydrolysis affords the desired targets. Path "c" demonstrates targets containing a urea-linkage between the spirocycle and an appropriately substituted 2,3-diaminopropionic acid derivative. The coupling is accomplished by removing the Boc-group from the spirocycle intermediate and reacting that with a p-nitro-phenylcarbamate of a 2,3-diaminopropionic acid ester. Mild acid or base hydrolysis affords the desired targets. Path "d" demonstrates targets derived from the coupling of a p-nitro-phenylcarbamate derivative of an O-substituted 4-hydroxypiperidine with the deprotected spirocyclic intermediate, thus affording a urea linkage. Mild acid or base hydrolysis provides the desired targets. Path "e" demonstrates targets derived from a peptide-like coupling (utilizing HBTU, BOP-Cl, or similar reagent) between the deprotected spirocyclic intermediate and an isonipecotic acid derivative in which the nitrogen has been acylated with an appropriate ester-acid chloride similar to Path "a". Mild acid or base hydrolysis affords the desired targets.

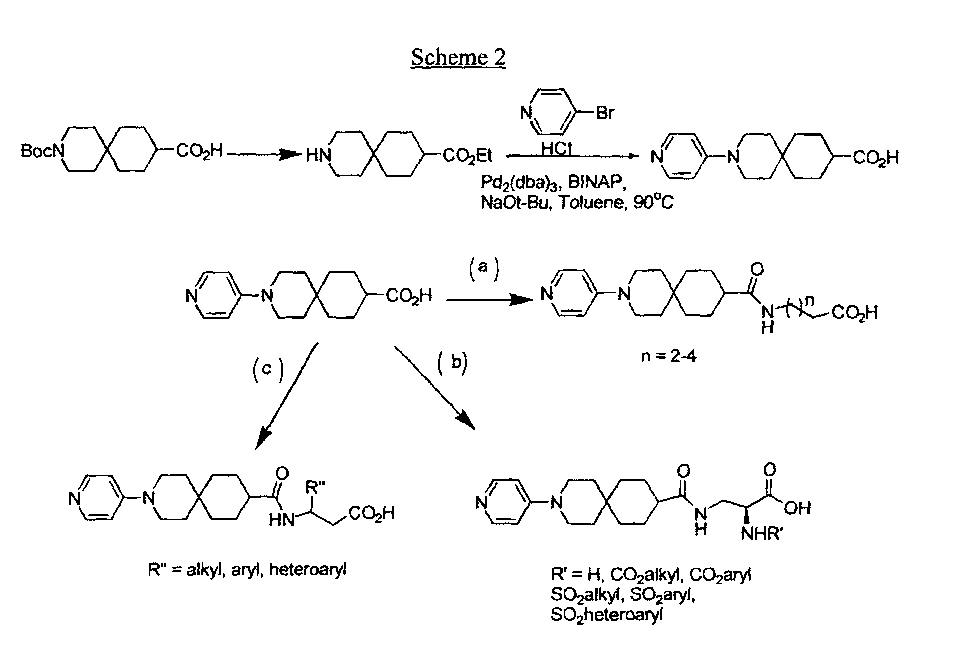

Scheme 2 describes the synthesis of (3-aza-spiro[5.5]undec-9-yl)formic acid derivatives. The synthesis of the spirocyclic nucleus is achieved following the patent procedure (see US Patent No. 5,451,578). The ester formation and deprotection of Boc is achieved in one step by treating with EtOH/HCl(g). The coupling of the azaspirocycle to 4-bromopyridine is accomplished utilizing a Pd-catalyzed reaction that is well known in the literature (Stephen L. Buckwald and Seble Wagaw, J. Org. Chem, 1996, 61, 7240). Subsequent coupling of this key intermediate as described in Paths a-c affords a variety of different targets. Coupling of the resultant spirocyclic acid template with appropriately substituted α or β-amino ester and unsubstituted aminoesters is accomplished using standard peptide coupling reagents such as EDC, HOBt. The mild acid or base hydrolysis of the ester affords the desired targets.

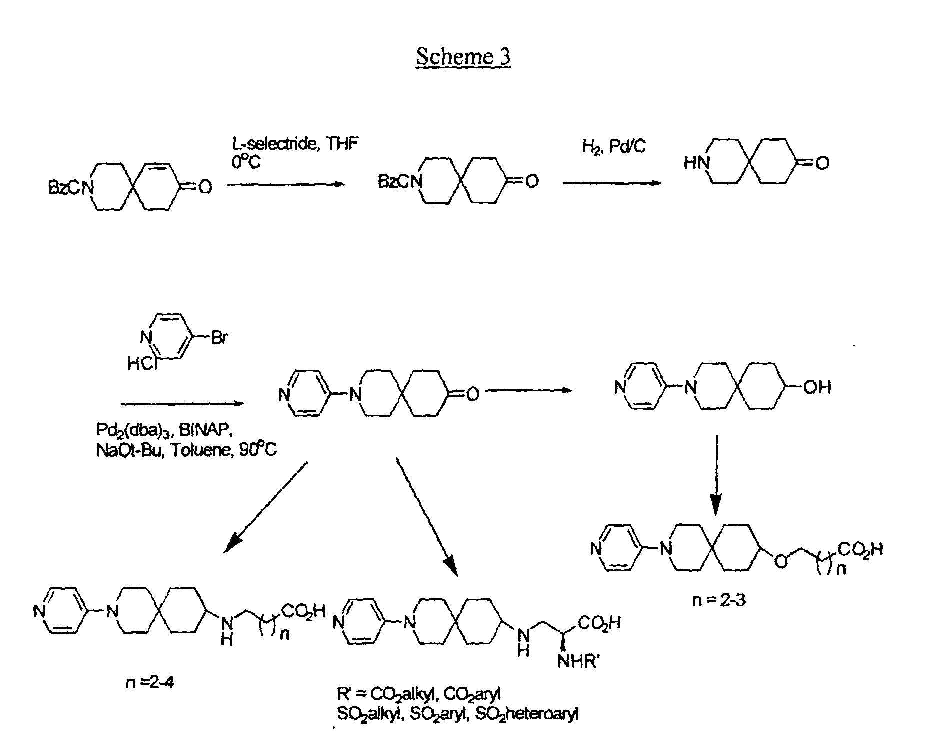

Scheme 3 describes the synthesis of the (3-aza-spiro[5.5]undec-9-yl)acetic acid derivative, a key spirocyclic intermediate that will be utilized in the synthesis of various targets as described herein. The spirocyclic enone is synthesized following the procedure as described in the Step A of Example 1 (WO 97/11940). The enone is reduced to ketone with L-selectride, followed by deprotection of carbobenzyloxy under standard hydrogenolysis conditions. The pyridyl group is then coupled to the spirocyclic template using a Pd-catalyzed coupling to 4-bromopyridine, thus affording the key spirocyclic intermediate. This ketone intermediate is subsequently treated under reductive amination conditions with several aminoesters and ∀-substituted aminoesters. In other series of derivatives, the intermediate ketone is reduced to the corresponding alcohol with sodium borohydride and then alkylated with bromoalkylesters. The mild acid or base hydrolysis of the esters affords the desired targets.

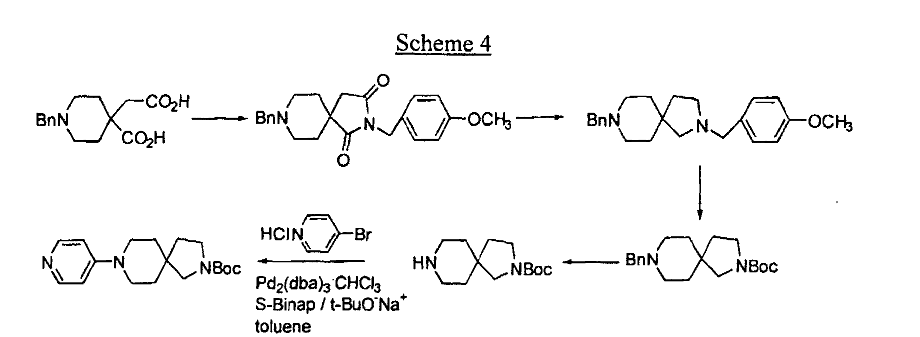

Scheme 4 describes the synthesis of *tert*-butyl 2,8-diaza-8-(4-pyridyl)spiro[4.5]decane-2-carboxylate, a key spirocyclic intermediate that will be utilized in the synthesis of various targets as described herein. The known diacid is treated with DCC followed by opening of the cyclic anhydride with 4-methoxybenzyl amine. This intermediate is then cyclized to the imide using sodium acetate in acetic anhydride. The carbonyl groups of the imide are then reduced using BH₃-THF complex. Removal of the 4-methoxy benzyl group is accomplished utilizing ceric ammonium nitrate. The Boc group is then added followed by removal of the benzyl group using standard hydrogenolysis over Pearlman's catalyst. The pyridyl group is then coupled to the spirocyclic template using a Pd-catalyzed coupling to 4-Br-pyridine (Buckwald, S.L., *et al. J.* Org. Chem. **1996**, 61, 7240), thus affording the key spirocyclic intermediate.

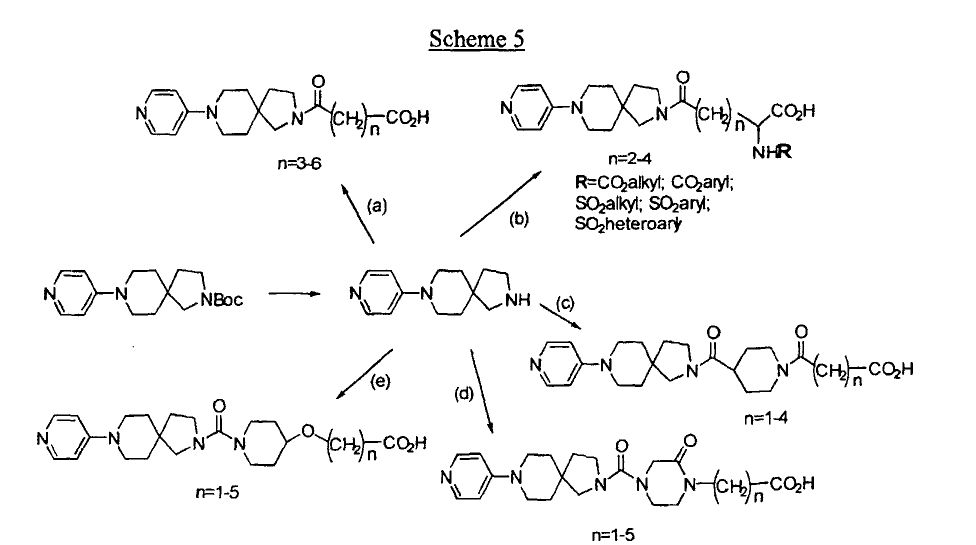

Scheme 5 describes the synthesis of 2,8-diaza-8-(4-pyridyl)spiro[4.5)decane containing compounds derived from *tert*-butyl 2,8-diaza-8-(4-pyridyl)spiro[4.5]decane-2-carboxylate which was obtained as described in Scheme 4. Removal of the Boc group with TFA provides the key intermediate from which all of the targets in this series were derived. Path "a" demonstrates targets obtained by the acylation of the key intermediate with a variety of ester-acid chlorides such as ethyl glutaryl chloride. Mild acid or base hydrolysis of the ester then affords the desired targets. Path "b" demonstrates targets containing a sulfonylated or carbamoylated amino group α to the terminal acid functionality. The coupling of an appropriate acid-α-amino-ester side chain is accomplished using standard peptide coupling agents such as HBTU or BOP-Cl. Mild acid or base hydrolysis of the ester then affords the desired targets. Path "c" demonstrates targets derived from a peptide-like coupling (utilizing HBTU, BOP-Cl, or similar reagent) between the key spirocyclic intermediate and an isonipecotic acid derivative in which the nitrogen has been acylated with an appropriate ester-acid chloride similar to Path "a". Mild acid or base hydrolysis of the ester then affords the desired targets. Path "d" demonstrates targets containing a urea linkage between the spirocycle and an appropriately derivatized piperazinone. Reacting the spirocycle with a p-nitro-phenylcarbamate of a functionalized piperazinone generates this urea linkage. Mild acid or base hydrolysis of the ester then affords the desired targets. Path "e" demonstrates targets derived from the coupling of a p-nitro-phenylcarbamate derivative of an O-substituted 4-hydroxypiperidine with the key spirocyclic intermediate, thus affording the urea linkage. Mild acid or base hydrolysis of the ester then provides the desired targets.

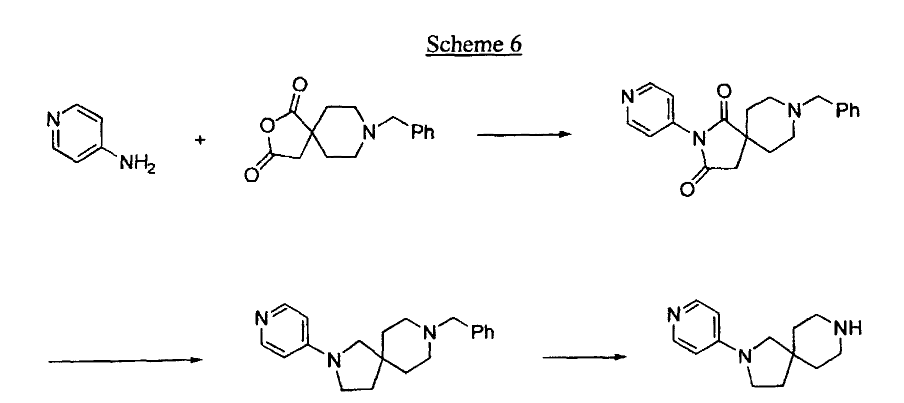

Scheme 6 describes the synthesis of a key spirocyclic intermediate 2-(4-pyridyl)-2,8-diazaspiro[4.5]decane. The spirocyclic anhydride (see WO 97/11940) is reacted with 4-amino pyridine to yield the imide. This is then reduced with BH₃-THF complex to furnish the amine. De-benzylation under hydrogenolysis conditions provided the desired spirocyclic template.

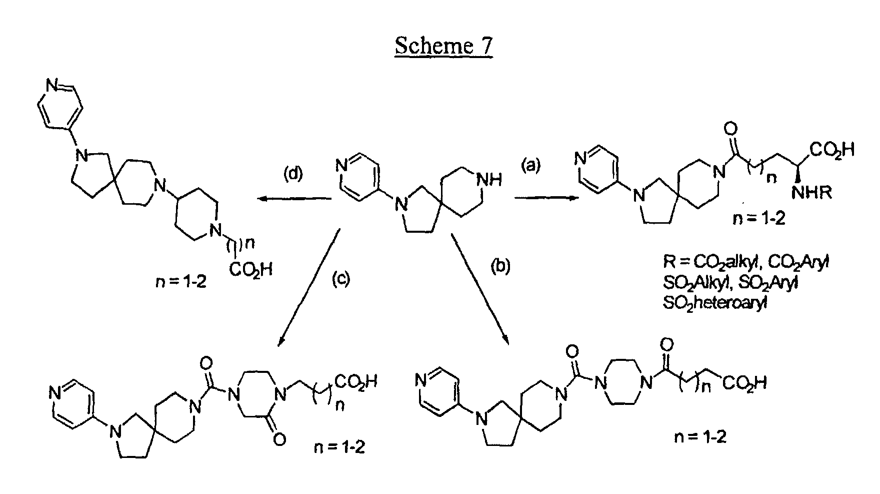

Scheme 7 describes the synthesis of 2-(4-pyridyl)-2,8-diazaspiro[4.5]decane containing compounds derived from the key intermediate which is obtained as described in Scheme 6. Path "a" demonstrates targets containing a sulfonylated or carbamoylated amino group α to the terminal acid functionality. The coupling of the appropriate acid-α-amino-ester side chain is accomplished using standard coupling agents such as HATU or HBTU. Mild acid or base hydrolysis of the ester then affords the desired targets. Path "b" demonstrates targets containing a urea linkage between the spirocycle and an appropriately derivatized piperazine. Reacting the spirocycle with a p-nitrophenyl-carbamate of a functionalized piperazine generates this urea linkage. The acid hydrolysis of the ester than affords the desired targets. Path "c' demonstrates the target containing a urea linkage between spirocycle and an appropriately derivatized piperazinone. The targets are synthesized as described for Path "b". Path "d" describes the targets containing a direct linkage between spirocycle and an appropriately derivatized piperidine. Reacting the spirocycle with a functionalized 4-piperidone under reductive amination conditions generates this linkage. Mild acid or base hydrolysis of the ester then affords the desired targets.

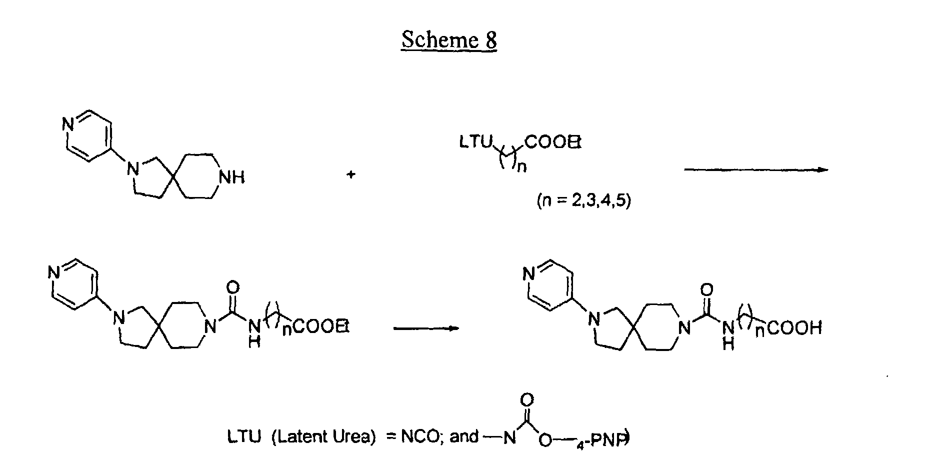

Scheme 8 describes the synthesis of a series of targets having alkyl-urea appendages attached to 2-(4-pyridyl)-2,8-diazaspiro[4.5]decane template. The spirocyclic piperidine is condensed with either isocyanates or with 4-nitrophenyl carbamates of the functionalized amines to afford the intermediate urea-esters. These esters are then hydrolyzed to the desired targets under mild acidic conditions.

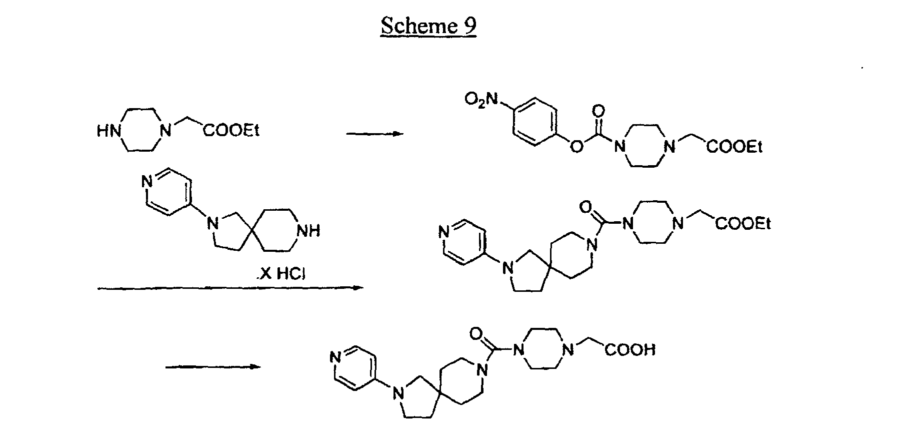

Scheme 9 describes the synthesis of 2-(4-{(2-([4-pyridyl)-2,8-diazaspiro[4.5]dec-8-yl]carbonyl}piperazino)acetic acid. Piperazine ethyl acetate derivative is reacted with 4-nitrophenyl chloroformate to give rise to the 4-nitrophenyl carbamate ester. This was reacted with the spirocyclic piperidine to afford the urea-ester. Treatment of this ester with 2N HCl provided the desired target.

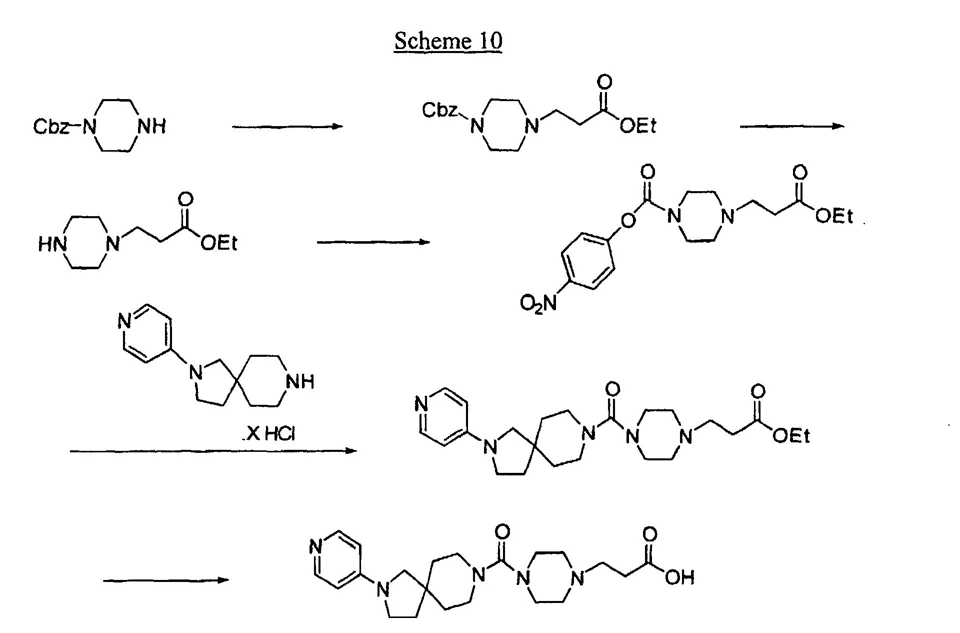

Scheme 10 describes the synthesis of 3-(4- { [2-(4-pyridyl)-2,8-diazaspiro[4.5]dec-8-yl]carbonyl}piperazino)propanoic acid. Benzyl-1-piperazine carboxylate is alkylated with ethyl bromopropionate, and the product is hydrogenolyzed. This amine is then reacted with 4-nitrophenyl chloroformate to give rise to the 4-nitrophenyl carbamate derivative. The resultant carbamate is reacted with the spirocyclic piperidine to afford the intermediate urea-ester. Treatment of the ester with 2N HCl provided the desired target.

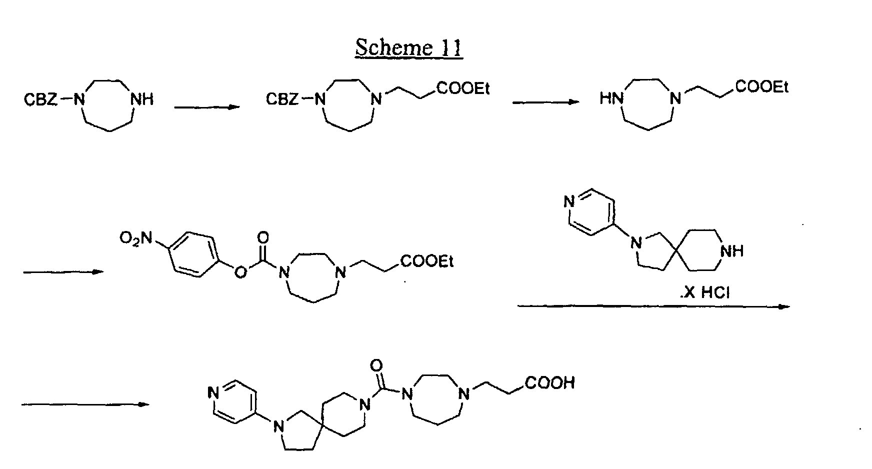

Scheme 11 describes the synthesis of 3-oxo-3-(4-{[2-(4-pyridyl)-2,8-diazaspiro [4.5]dec-8-yl]carbonyl} 1,4-diazepan-1-yl)propanoic acid. N-Cbz-homopiperazine is alkylated with bromoethyl propionate. The Cbz-group was removed under standard hydrogenolysis conditions. This amine is then reacted with 4-nitrophenyl chloroformate to afford the 4-nitrophenyl carbamate, which is subsequently coupled with the spirocyclic piperidine to give rise to the desired compound as its ethyl ester. Hydrolysis of the ester with 2N HCl yielded the target compound.

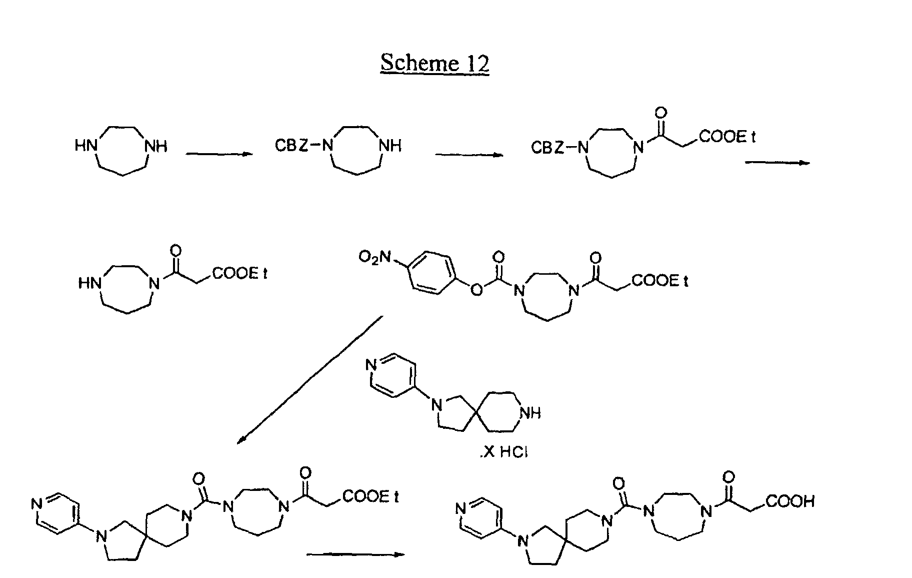

Scheme 12 describes the synthesis of 3-oxo-3-(4-{[2-(4-pyridyl)-2,8-diazaspiro[4.5]dec-8-yl]carbonyl}-1,4-diazepan-1-yl)propanoic acid. Homopiperazine is converted to the mono Cbz-derivative by reaction with Cbz-Cl and then isolation by chromatography. N-Cbz-homo piperazine is acylated with ethyl malonyl chloride, followed by removal of the Cbz-group under standard hydrogenolysis conditions. This material is then reacted with 4-nitrophenyl chloroformate to afford the 4-nitrophenyl carbamate, which is further reacted with the spirocyclic piperidine to give rise to intermediate urea-ester. Hydrolysis of the ester with 2N HCl yielded the target compound.

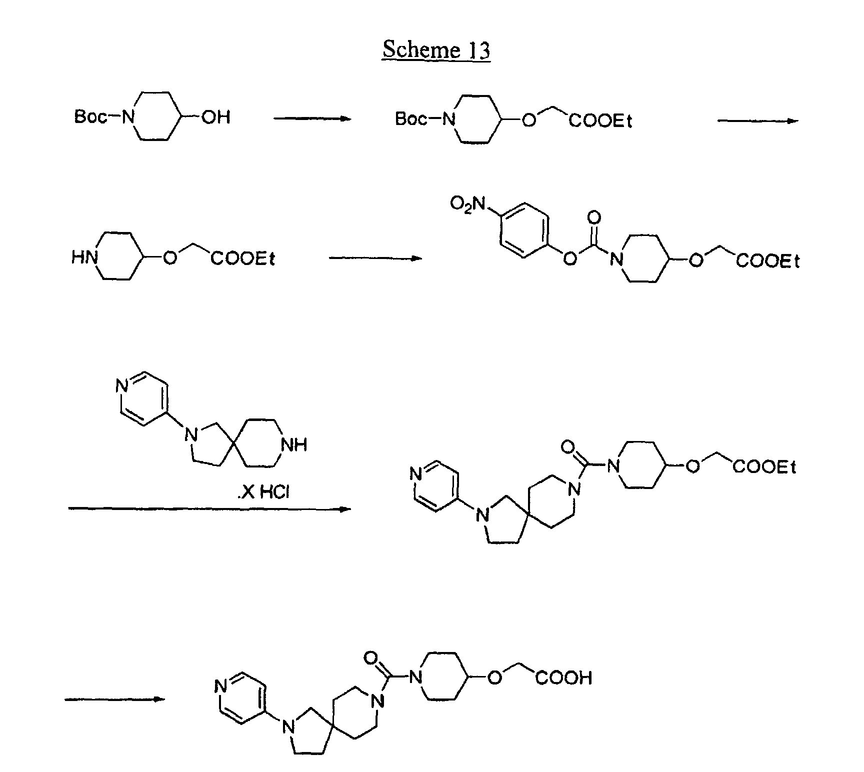

Scheme 13 describes the synthesis of 2-[(1-{2-(4-pyridyl)-2,8-diazaspiro[4.5)dec-8-yl]carbonyl}-4-piperidyl)oxy]acetic acid via the coupling of a p-nitrophenyl carbamate derivative of an O-substituted 4-hydroxypiperidine with 2-(4-pyridyl)-2,8-diazaspiro[4.5]decane. N-Boc-4-hydroxy piperidine is reacted with ethyl diazoacetate and rhodium diacetate dimer to give rise to the oxy-ethyl acetate. This intermediate is treated with TFA, and the resultant piperidine is reacted with 4-nitrophenyl chloroformate. The 4-nitro phenyl carbamate is then reacted with the spirocyclic piperidine to afford the coupled urea-ester. Hydrolysis of the ester with 2N HCl yielded the desired target.

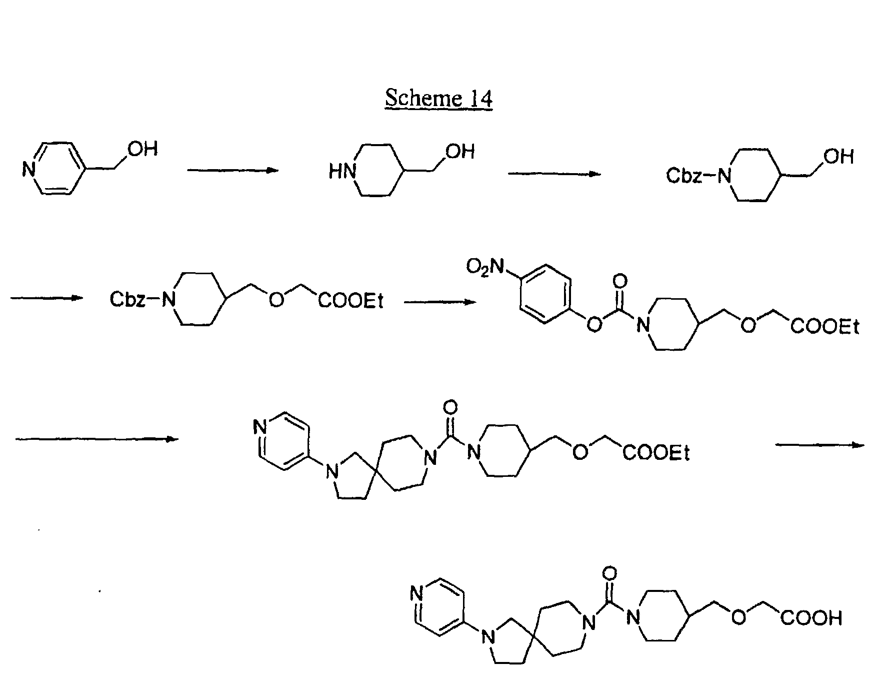

Scheme 14 describes the synthesis of 2-[(1-{[2-(4-pyridyl)-2,8-diazaspiro[4.5]dec-8-yl]carbonyl}-4-piperidyl)-methoxy]acetic acid. 4-Pyridylcarbinol is hydrogenated over PtO₂ in acetic acid. The resulting piperidine is then converted to its N-Cbz-derivative using Cbz-Cl. This compound is then reacted with ethyl diazoacetate and rhodium diacetate dimer to give rise to the oxy-ethyl acetate intermediate. This is then hydrogenolyzed and reacted with 4-nitrophenyl chloroformate to afford its 4-nitrophenyl carbamate. This carbamate is then reacted with the spirocyclic piperidine to yield the urea-ester. Hydrolysis of the ester with 2N HCI afforded the desired compound.

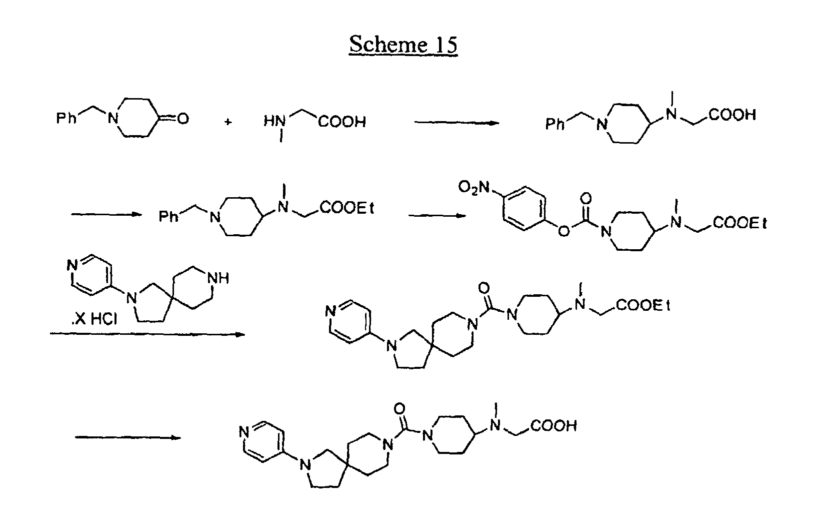

Scheme 15 describes the synthesis of 2-[methyl(1-{[2-(4-pyridyl)-2,8-diazaspiro[4.5]dec-8-yl]carbonyl}-4-piperidyl)amino]acetic acid. N-Benzyl 4-piperidone is reductively alkylated with N-methyl glycine using sodium triacetoxy borohydride and acetic acid. This intermediate is then converted to its ethyl ester, N-debenzylated, followed by reaction with p-nitrophenyl chloroformate. The resultant p-nitrophenyl carbamate is then reacted with the spirocyclic piperidine to furnish the urea-ester intermediate. Hydrolysis of the ester with 2N HCI provided the desired target.

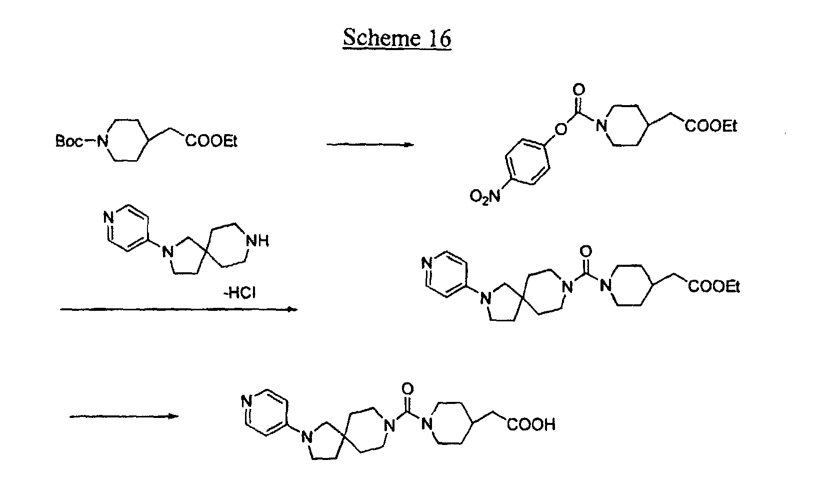

Scheme 16 describes the synthesis of 2-(1-{[2-(4-pyridyl)-2,8-diazaspiro[4.5]dec-8-yl]carbonyl]-4-piperidyl)acetic acid. Ethyl-4-piperidine acetate is reacted with 4-nitrophenyl chloroformate to give rise to the 4-nitrophenyl carbamate analog. This is then reacted with the spirocyclic piperidine to afford the coupled urea-ester. Treatment of the resulting ester with 2N HCl provided the desired target.

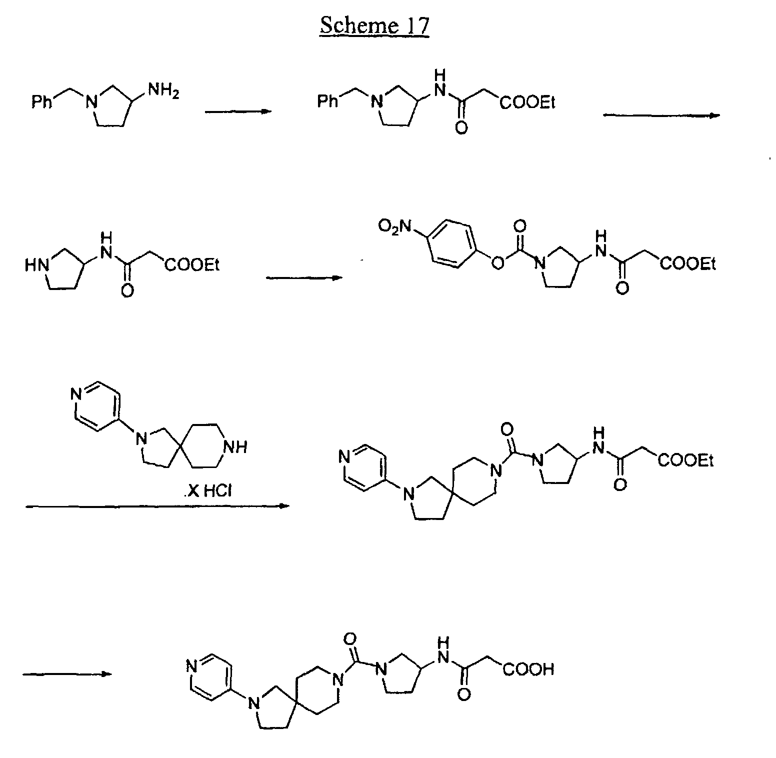

Scheme 17 describes the synthesis of 3-oxo-3-[(1-{[2-(4-pyridyl)-2,8-diazaspiro[4.5]dec-8-yl]carbonyl)-tetrahydro- 1 H-3-pyrrolyl)amino]propanoic acid. N-benzyl 3-amino-pyrrolidine is acylated with ethyl malonyl chloride, and the product is debenzylated. The free amine is then reacted with 4-nitrophenyl chloroformate to afford the 4-nitrophenyl carbamate. This carbamate is coupled with the spirocyclic piperidine to afford the urea-ester. Hydrolysis of the ester with 2N HCI afforded the desired compound.

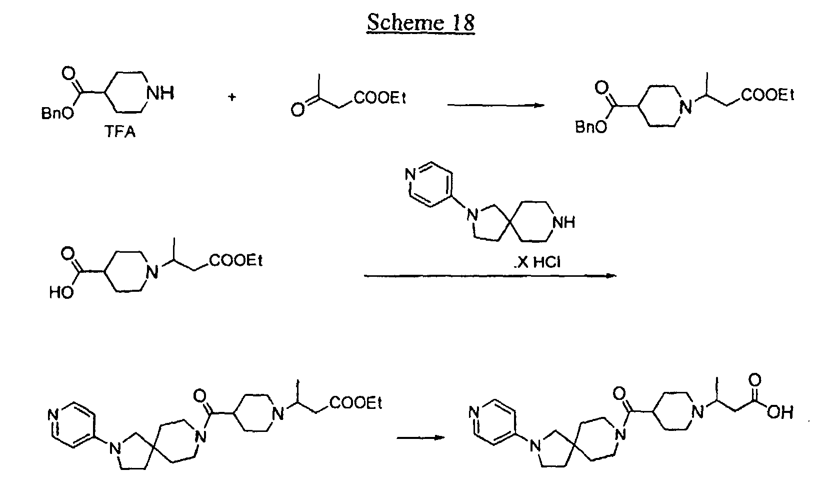

Scheme 18 describes the synthesis of 3-4-{[2-(4-pyridyl)-2,8-diazaspiro[4.5]dec-8-yl]carbonyl}-piperidino)butanoic acid. The 4-Carbobenzyloxy piperidine is reductively alkylated with ethyl acetoacetate using sodium tri-acetoxyborohydride in acetic acid. The intermediate was then debenzylated using standard hydrogenolysis conditions followed by coupling with the spirocyclic piperidine to afford the amide. Hydrolysis of the ester with 2N HCl provided the desired target.

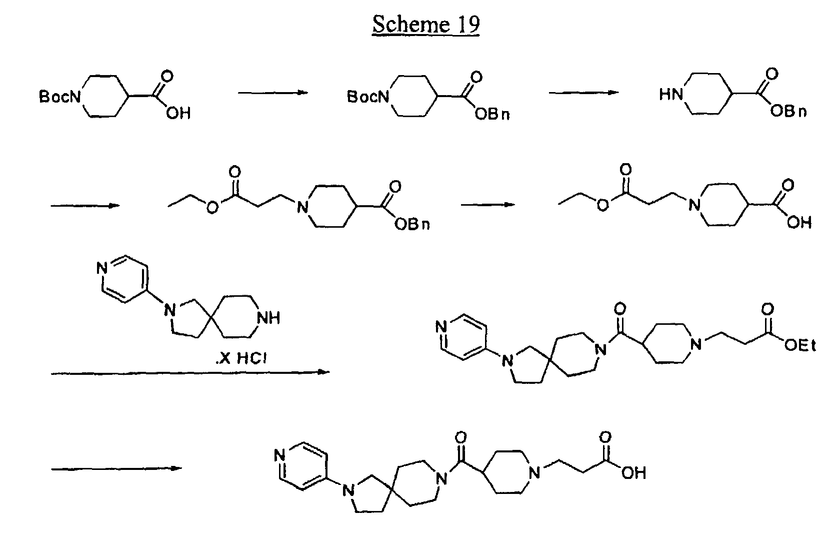

Scheme 19 describes the synthesis of 3-(4-{[2-(4-pyridyl)-2,8-diazaspiro[4.5]dec-8-yl]carbonyl}piperidino)propanoic acid from 2-(4-pyridyl)-2,8-diazaspiro[4.5]decane and N-alkyl isonipecotic acid derivatives. N-Boc-piperidine 4-carboxylic acid is converted to its benzyl ester using DCC and benzyl alcohol. The removal of the Boc-group with TFA provides the piperidine. After alkylation with bromoethyl propionate, the benzyl group is hydrogenolyzed. This intermediate is then coupled with the spirocyclic piperidine to afford the amide. Hydrolysis of the ester with 2N HCl provided the desired target.

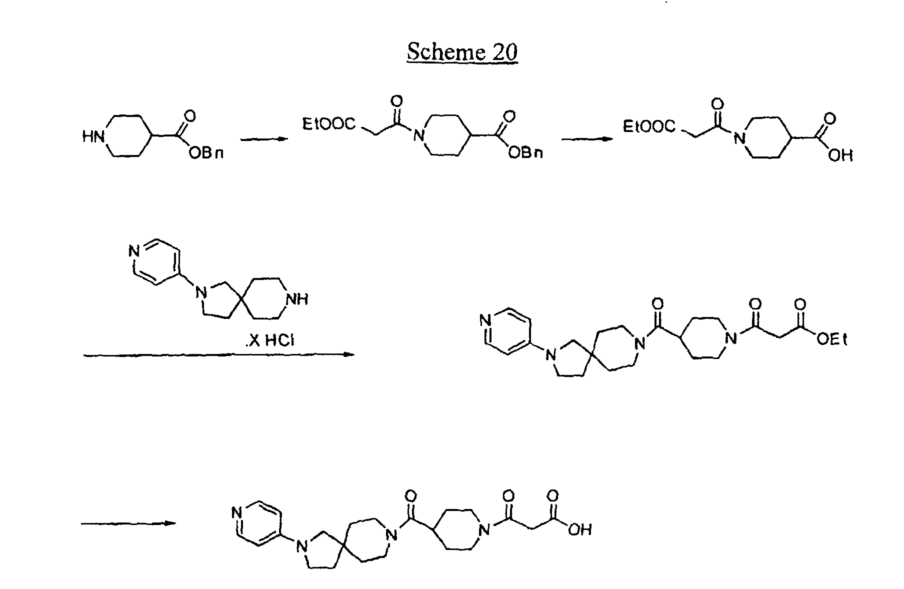

Scheme 20 describes the synthesis of 3-(4-{[2-(4-pyridyl)-2,8-diazaspiro[4.5]dec-8-yl]carbonyl}piperidino)propanoic acid. 4-Carbobenzyloxy piperidine is acylated with ethyl glutaryl chloride and then the benzyl group was hydrogenolyzed. This intermediate is then coupled with the spirocyclic piperidine under standard peptide coupling conditions to afford the amide. Treatment with 2N HCI hydrolyzed the ester and provided the desired target.

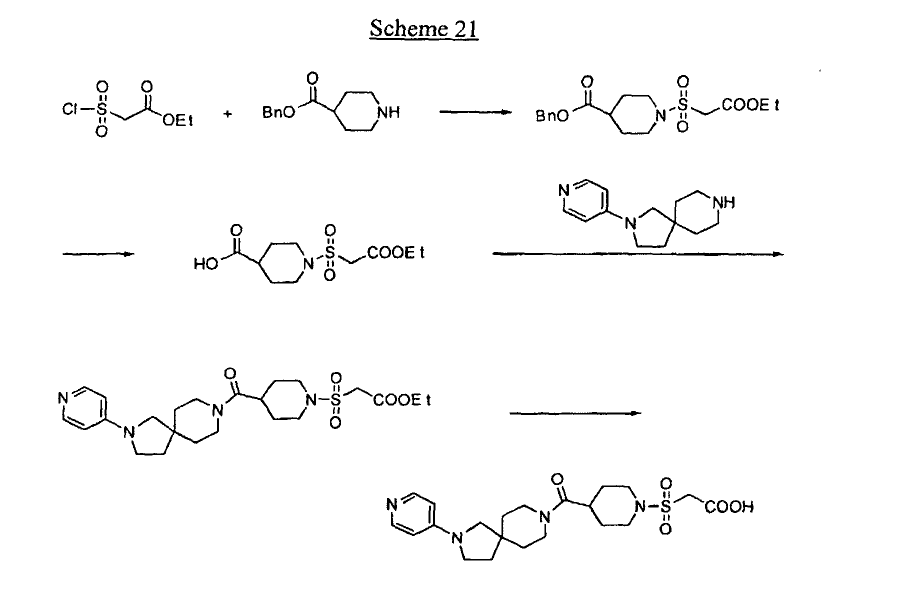

Scheme 21 describes the synthesis of ethyl 2-[(4-{[2-(4-pyridyl)-2,8-diazaspiro[4.5]dec-8-yl]carbonyl}-piperidino)sulfonyl]acetic acid. Ethyl 2-chlorosulfonylacetate (Oliver, J. E., and Demilo, A. B., *Synthesis,* 321 **1975**) is reacted with 4-Carbobenzyloxy piperidine and the resultant sulfonamide-containing compound is de-benzylated under standard hydrogenolysis conditions. This intermediate is then coupled with the spirocyclic piperidine to afford the key ester. Treatment of this ester with 2N HCI provided the desired target.

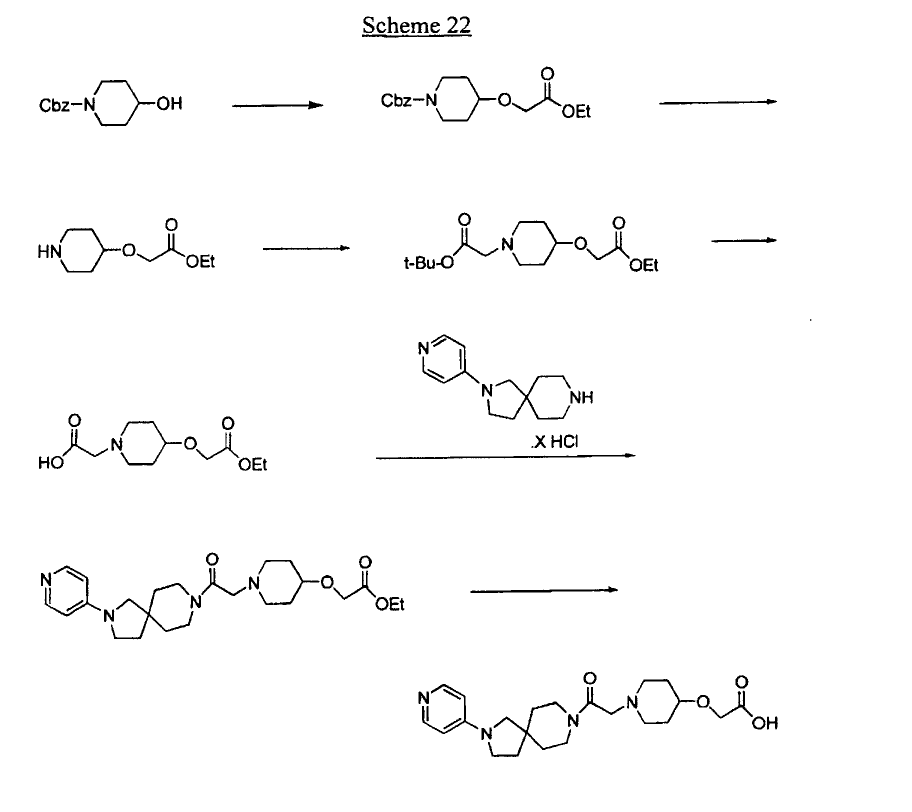

Scheme 22 describes the synthesis of 2-[(1-{2-oxo-2-[2-(4-pyridyl)-2,8-diazaspiro[4.5]dec-8-yl]ethyl}-4-piperidyl)oxy]acetic acid. N-Cbz-4-hydroxy-piperidine is reacted with ethyl diazoacetate and rhodium diacetate dimer to give rise to the oxy-ethyl acetate intermediate. It is then hydrogenolyzed and alkylated with tert-butyl bromoacetate. Treatment with TFA provided the acetic acid derivative which was then coupled with the spirocyclic piperidine to yield the amide. Hydrolysis of the ester with 2N HCl provided the desired target.

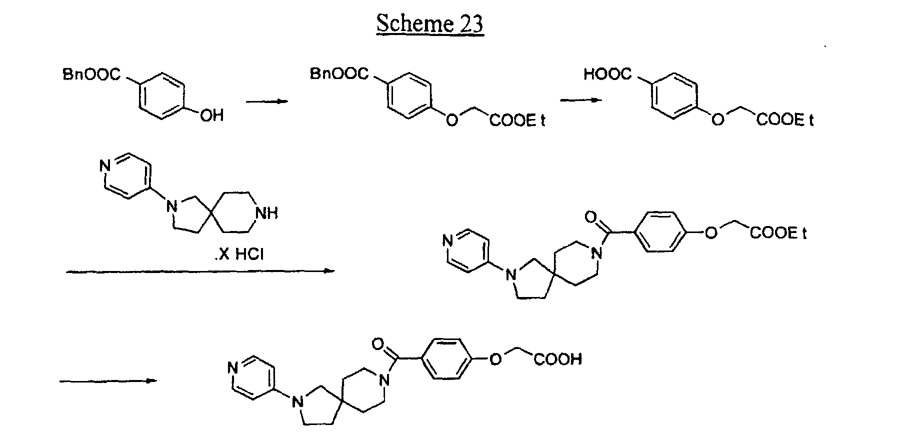

Scheme 23 describes the synthesis of 2-(4-{[2-(4-pyridyl)-2,8-diazaspiro[4.5)dec-8-yl]carbonyl}phenoxy)acetic acid. Benzyl 4-hydroxy benzoate is reacted with ethyl diazoacetate and rhodium diacetate dimer to give rise to the intermediate oxy-ethyl acetate. This compound is then de-benzylated and coupled with the spirocyclic piperidine to afford the benzamide. Hydrolysis of the ester with 2N HCl provided the desired target.

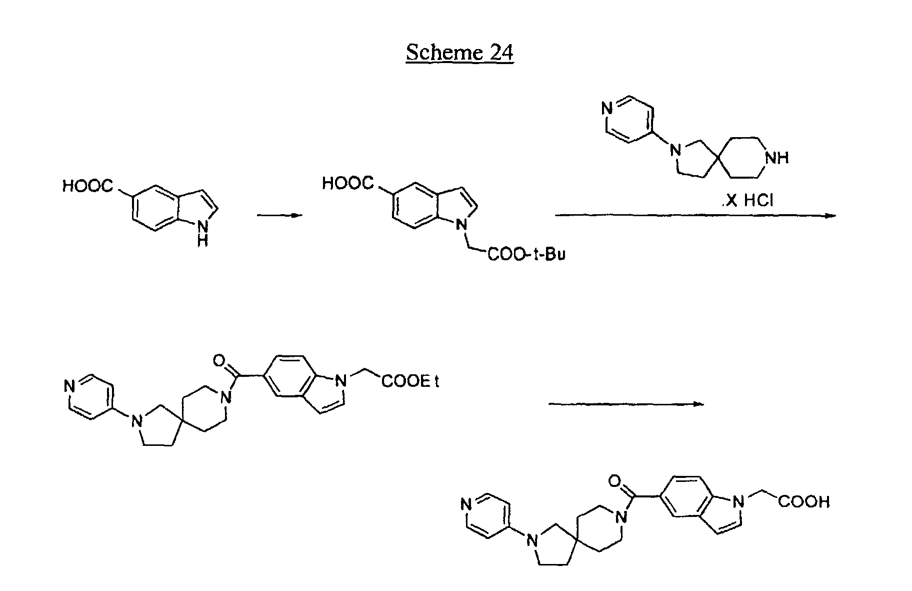

Scheme 24 describes the synthesis of 2-(5-{[2(4-pyridyl)-2,8-diazaspiro[4.5]dec-8-yl]carbonyl}-1H-1-indolyl)acetic acid. Indole-4-carboxylic acid was treated with excess sodium hydride and *tert*-butyl bromoacetate to afford 1-(2-tert-butoxy-2-oxoethyl)-1H-5-indole carboxylic acid. This was then coupled with the spirocyclic piperidine to afford the amide. Hydrolysis of the ester with 2N HCl yielded the desired product.

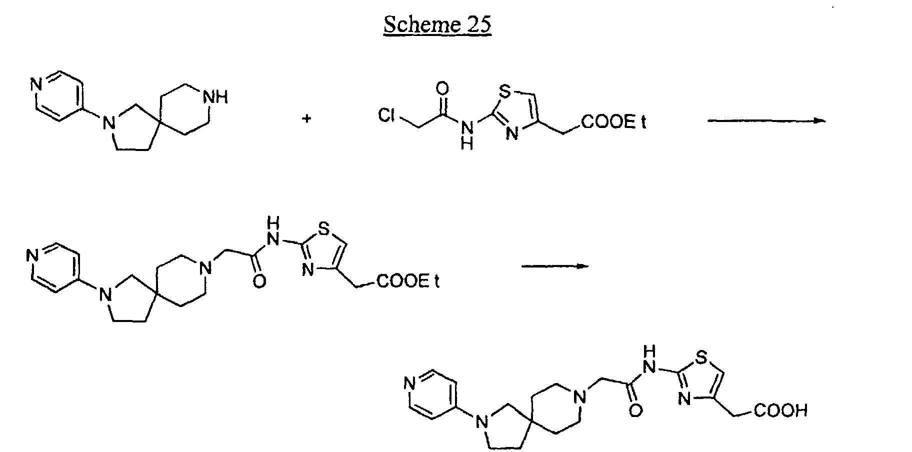

Scheme 25 describes the facile synthesis of 2-[2-({2-[2-(4-pyridyl)-2,8-diazaspiro[4.5]dec-8-yl]acetyl}amino)-1,3-thiazol-4-yl]acetic acid from the alkylation of 2-(4-pyridyl)-2,8-diazaspiro[4.5]decane with ethyl 2-(2-chloroacetamido)-4-thiazoleacetate to afford the desired compound as its ethyl ester. Hydrolysis of the ester with 2N HCl yielded the target compound.

The following Examples illustrate the practice of the invention.

### Example 1

### Preparation of 5-oxo-5-(9-(4-pyridyl)-3,9-diazaspiro[5.5]undec-3-yl)pentanoic acid.

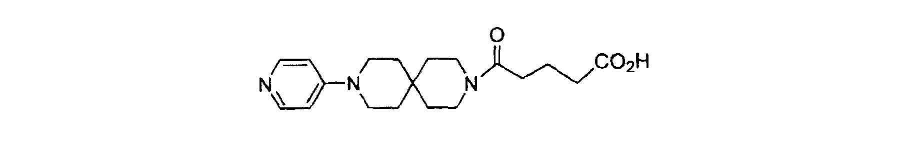

### Step A: Preparation of tert-butyl 9-(4-pyridyl)-3,9-diazaspiro[5.5]undecane-3-carboxylate.

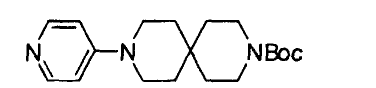

The synthesis of the starting material, tert-butyl 3,9-diazaspiro[5.5]undecane-3-carboxylate was accomplished as described in US patent #5,451,578. This solid (1.0 gm) is combined with 4-bromopyridine-HCl (766 mg), tris(dibenzylideneacetone) dipalladium(0)-chloroform adduct (124 mg), S-BINAP (174 mg), and sodium *tert*-butoxide (1.51 gm) under argon in a dry round bottom flask. To this mixture of solids is added 20 ml of anhydrous toluene. The mixture is then warmed to 90-95°C and stirred until done based on analytical RP-HPLC analysis. The solution is cooled to 0 °C, 70 ml of EtOAc is added, and the solution is stirred for 15 minutes. After filtering through celite, the solvent is evaporated under reduced pressure. The semi-solid residue is then purified using RP-HPLC to afford pure title compound (1.08 gm, 83%).
MS (ES) 332 (M+H)⁺

### Step B: Preparation of methyl 5-oxo-5-(9-(4-pyridyl)-3,9-diazaspiro[5.5]undec-3-yl)pentanoate.

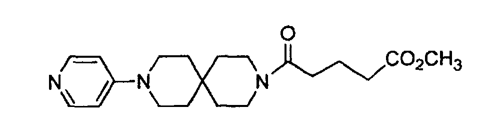

To the material obtained in step A (110 mg) was added 40% TFA in CH₂Cl₂ (2 ml) at room temperature. After stirring for ½ hr, the solvent was evaporated. The resultant residue was dissolved in anhydrous CH₂Cl₂ (3 ml) under argon at room temperature. After adding DIEA (0.54 ml) slowly, methyl glutaryl chloride (0.1 ml) was added dropwise via syringe and stirring was maintained overnight. After evaporation of the solvent under reduced pressure, EtOAc (5 ml) was added. This solution was then washed with H₂O (5 ml), 1 M HCl (5 ml), and then the combined aqueous washes were extracted with EtOAc (2 x 5 ml). The aqueous washes were then lyophilized and purified via RP-HPLC to afford the desired ester (18 mg).
MS (APCI) 360 (M+H)⁺

### Step C: Preparation of the title compound.

To the material obtained in step B (11 mg) in MeOH (2 ml) was added 1M LiOH (0.12 ml) and H₂O (0.2 ml). After stirring for 2 days at room temperature, the MeOH was removed under reduced pressure and the resultant material was purified via RP-HPLC to afford the title compound (8.9 mg, 83%).
MS (APCI) 346 (M+H)⁺
ELISA: IC₅₀=0.238 µM PRP: IC₅₀=5.1 µM

### Example 2

### Preparation of 6-oxo-6-(9-(4-pyridyl)-3,9-diazaspiro[5.5]undec-3-yl)hexanoic acid.

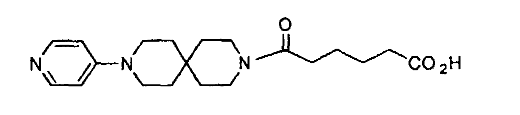

This compound was prepared by substantially following the procedure in Example 1 except that methyl adipyl chloride was used in place of methyl glutaryl chloride in step B.
MS (APCI) 360 (M+H)⁺
ELISA: IC₅₀=0.258 µM PRP: IC₅₀=4.5 µM

### Example 3

### Preparation of 7-oxo-7-(9-(4-pyridyl)-3,9-diazaspiro[5.5]undec-3-yl)heptanoic acid.

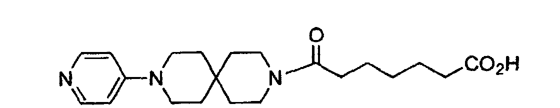

This compound was prepared by substantially following the procedure in Example 1 except that ethyl 6-(chloroformyl)hexanoate was used in place of methyl glutaryl chloride in step B.
MS (APCl) 374 (M+H)⁺
ELISA: IC₅₀=0.265 µM PRP: IC₅₀=4.5 µM

### Example 4

### Preparation of 8-oxo-8-(9-(4-pyridyl)-3,9-diazaspiro[5.5]undec-3-yl)octanoic acid.

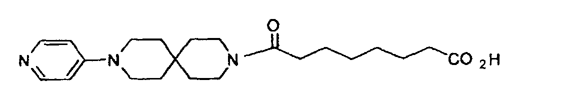

This compound was prepared by substantially following the procedure in Example 1 except that methyl 8-chloro-8-oxo-octanoate was used in place of methyl glutaryl chloride in step B.
MS (APCI) 388 (M+H)⁺
ELISA: IC₅₀=1.16µM PRP: IC₅₀=18 µM

### Example 5

### Preparation of 2(S)-2- { [(benzyloxy)carbonyl]amino}-5-oxo-5-[9-(4-pyridyl)-3,9-diazaspiro[5.5]undec-3-yl]pentanoic acid.

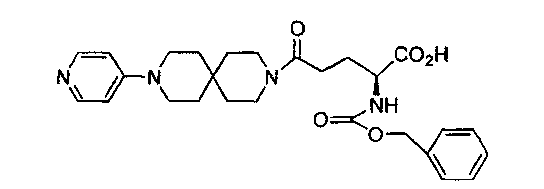

### Step A: Preparation of ethyl 2(S)-2-{[(benzyloxy)carbonyl]amino}-5-oxo-5-[9-(4-pyridyl)-3,9-diazaspiro[5.5]undec-3-yl]pentanoate.

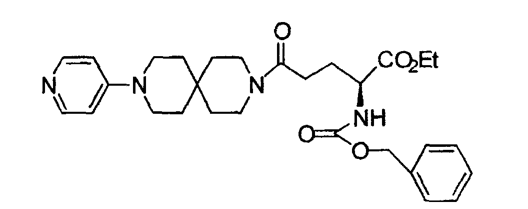

To *tert*-hutyl 9-(4-pyridyl-3,9-diazaspiro[5.5]undecane-3-carboxylate (300 mg) under argon at room temperature was added 40% TFA in CH₂Cl₂ (5 ml). After stirring for 1 hr, the solvent was evaporated. The resultant residue was dissolved in anhydrous DMF (7.5 ml) under argon at room temperature. After adding HBTU (750 mg), (4S)-4-{[(benzyloxy)carbonyl]amino}-5-ethoxy-5-oxopentanoic acid (306 mg) (obtained from Example 40), and then DIEA (1.4 ml) slowly, the solution was stirred at room temperature overnight. The mixture was poured into 50 ml of brine and then 5 ml of H₂O was added. This solution was washed with EtOAc (2 x 30 ml). The EtOAc was washed with saturated NaHCO₃ (30 ml), dried with MgSO₄, and then evaporated under vacuum to give the crude product. This was then purified on RP-HPLC to afford the desired ester.
MS (ES) 523 (M+H)⁺

### Step B: Preparation of the title compound.

The ester (4.6 mg) from Step A in this example was treated with 1 ml of 2M HCl at room temperature and stirred overnight. The reaction was followed by analytical RP-HPLC until the starting material had been consumed. The resultant solution was then submitted to purification via RP-HPLC thus affording the title acid (2.3 mg, 53%).
MS (ES) 495 (M+H)⁺
ELISA: IC₅₀=0.007 µM PRP: IC₅₀=4.6 µM

### Example 6

### Preparation of 2(S)-2-[(butylsulfonyl)amino]-5-oxo-5-[9-(4-pyridyl)-3,9-diazaspiro [5.5]undec-3-yl]pentanoic acid.

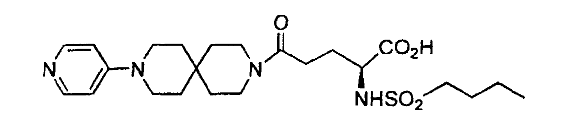

The title compound was prepared by substantially the same procedure as for Example 5 except that (4S )-4-[(butylsulfonyl)amino]-5-ethoxy-5-oxopentanoic acid was used in place of (4S)-4-{[(benzyloxy)carbonyl] amino}-5-ethoxy-5-oxopentanoic acid.
MS (ES) 481 (M+H)⁺
ELISA: IC₅₀=0.023 µM PRP: IC₅₀=0.79 µM

### Example 7

### Preparation of 2(S)-2-{[(4-methylphenyl)sulfonyl]amino}-5-oxo-5-[9-(4-pyridyl)-3,9-diazaspiro[5.5]undec-3-yl]pentanoic acid.

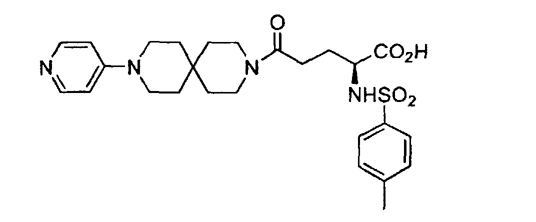

The title compound was prepared by substantially the same procedure as for Example 5 except that (4S)-4-{ [(4-methylphenyl)sulfonyl]amino}-5-ethoxy-5-oxopentanoic acid was used in place of (45)-4- { [(benzyloxy)carbonyl]amino} -5-ethoxy-5-oxopentanoic acid.
MS (ES) 515 (M+H)⁺
ELISA: IC₅₀=0.002 µM PRP: IC₅₀=0.077 µM

### Example 8

### Preparation of 2(S)-2-{[(3,5-dimethyl-4-isoxazolyl)sulfonyl]amino}-5-oxo-5-[9-(4-pyridyl)-3,9-diazaspiro[5.5]undec-3-yl]pentanoic acid.

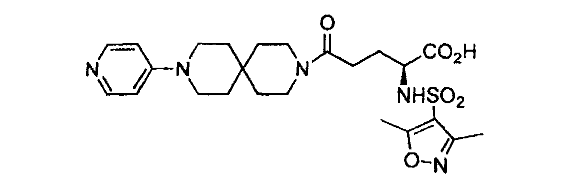

The title compound was prepared by substantially the same procedure as for Example 5 except that (4S)-4-{[(3,5-dimethyl-4-isoxazolyl)sulfonyl]amino}-5-ethoxy-5-oxopentanoic acid was used in place of (4S)-4-{[(benzyloxy)carbonyl]amino}-5-ethoxy-5-oxopentanoic acid.
MS (ES) 520 (M+H)⁺
ELISA: IC₅₀=0.002 µM PRP: IC₅₀=0.021 µM

### Example 9

### Preparation of 2(S)-2-[(butoxycarbonyl)amino]-5-oxo-5-[9-(4-pyridyl)-3,9-diazaspiro [5.5]undec-3-yl]pentanoic acid.

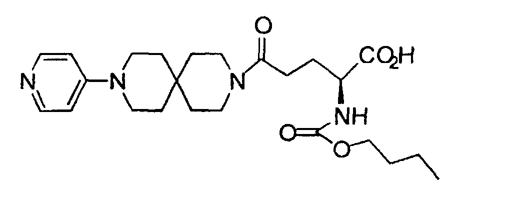

The title compound was prepared by substantially the same procedure as for Example 5 except that (4S)-4-[(butoxycarbonyl)amino]-5-ethoxy-5-oxopentanoic acid was used in place of (4S)-4-{[(benzyloxy)carbonyl]amino) -5-ethoxy-5-oxopentanoic acid.
MS (ES) 461 (M+H)⁺
ELISA: IC₅₀ 0.002 µM PRP: IC₅₀= 0.722 µM

### Example 10

### Preparation of (2S)-2- { [(3 ,5-dimethyl-4-isoxazolyl)sulfonyl]amino } -3-({ [9-(4-pyridyl)-3,9-diazaspiro[5.5]undec-3-yl]carbonyl}amino)propanoic acid.

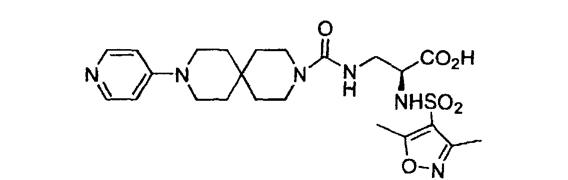

### Step A: Preparation of 3-(4-pyridyl)-3,9-diazaspiro[5.5]undecane.

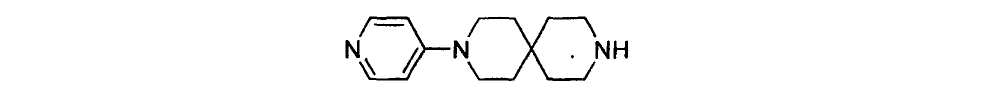

To *tert-butyl* 9-(4-pyridyl)-3,9-diazaspiro[5.5]undecane-3-carboxylate (100 mg) (obtained in Example 1, step A) is added 40% TFA in CH₂Cl₂ (3 ml) at room temperature under argon. After stirring for 1 hr, the solvent is evaporated under reduced pressure. The resultant syrup is used without further purification in Step C of this Example.

### Step B: Preparation of ethyl (4S)-3-[(3,5-dimethyl-4-isoxazolyl)sulfonyl]-2-oxotetrahydro-1H-4-imidazolecarboxylate.

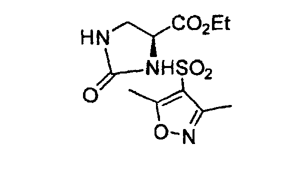

To ethyl (2S)-3-amino-2-{[(3,5-dimethyl-4-isoxazolyl)sulfonyl]amino}propanoate (291 mg) (as prepared in US Patent No. 5648368) in CH₂Cl₂ (5 ml) at 0°C under argon was added 4-nitrophenyl chloroformate (222 mg) followed by dropwise addition of DIEA (1.0 ml). The resultant yellow solution was stirred overnight as it warmed to room temperature. Additional CH₂Cl₂ (10 ml) was then added followed by washes of 50% saturated NaHCO₃ (5 x 10 ml), 1M HCI (10 ml)_{,} and brine (10 ml). The colorless solution was then dried with MgSO₄, filtered, and evaporated *in vacuo* to afford the desired compound (311 mg, 98%).
MS (ES) 318 (M+H)⁺

### Step C: Preparation of ethyl (2S)-2-([(3,5-dimethyl-4-isoxazolyl)sulfonyl]amino}-3-({ [9-(4-pyridyl)-3,9-diazaspiro[5.5]undec-3-yl)carbonyl}amino)propanoate.

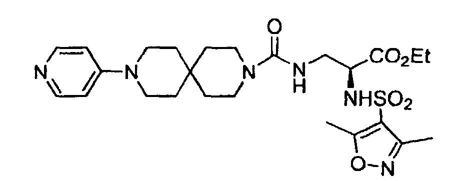

A mixture of the materials obtained in Step A and in Step B (95 mg) of this example in DMF (3 ml) under argon was warmed to 65°C and stirred overnight. The reaction mixture was then cooled to room temperature, diluted with 20 ml H₂O, and acidified to pH=2 with TFA. This solution was then purified utilizing RP-HPLC to afford the desired ester (126 mg, 76%)
MS (ES) 549 (M+H)⁺

### Step D: Preparation of the title compound.

The ester from Step C in this example was hydrolyzed by substantially following the procedure from Example 5, Step B to afford the title compound as a white fluffy solid in quantitative yield.
MS (ES) 521 (M+H)⁺
ELISA: IC₅₀=0.001 µM PRP: IC₅₀=0.031 µM

### Example 11

### Preparation of 2-[(1-{[9-(4-pyridyl)-3,9-diazaspiro[5.5]undec-3-yl]carbonyl}-4-piperidyl)oxy]acetic acid.

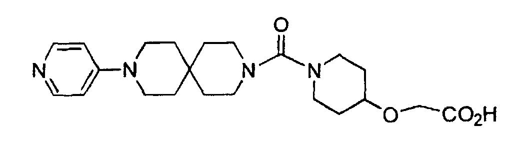

### Step A: Preparation of tert-butyl 4-hydroxy-1 -piperidinecarboxylate.

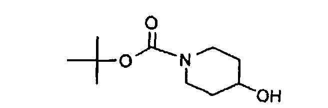

To a suspension of 4-piperidinol (5.06 gm) in anhydrous acetonitrile (50 ml) at room temperature under argon was added di-*tert*-butyl dicarbonate (11.4 gm) in 2 portions. Then DMAP (610 mg) was added and stirring maintained for 3.5 hrs. After evaporation of the solvent under reduced pressure, the residue was partitioned between CH₂Cl₂ (50 ml) and 1M HCl (50 ml). The HCI was then washed with more CH₂Cl₂ (2 x 50 ml). The combined CH₂Cl₂ washes were then extracted with brine (50 ml), dried with MgSO₄, and then evaporated *in vacuo* to afford the desired product (9.95 gm, 99%) in sufficient purity to carry into the next step.
MS (IS) 202 (M+H)⁺

### Step B: Preparation of tert-butyl 4-(2-ethoxy-2-oxoethoxy)-1-piperidinecarboxylate.

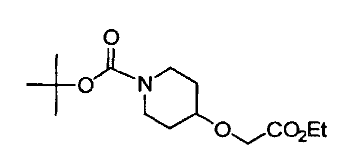

To the material from Step A of this example (5.0 gm) in anhydrous CH₂Cl₂ (100 ml) at room temperature under argon was added rhodium(II) acetate dimer (300 mg) followed by very slow dropwise addition of a solution of ethyl diazoacetate (3.9 ml) in CH₂Cl₂ (21 ml). After stirring for 0.5 hrs, the solution was evaporated to dryness and the residue purified utilizing flash chromatography (hexane/EtOAc, 4:1) to afford the desired compound (5.56 gm, 78%).
MS (DCI) 288 (M+H)⁺

### Step C: Preparation of 4-nitrophenyl 4-(2-ethoxy-2-oxoethoxy)-1-piperidinecarboxylate.

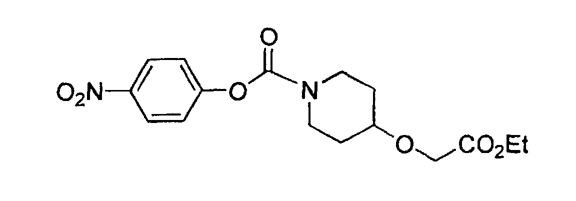

To the material obtained in Step B of this example (1.5 gm) was added 40% TFA in CH₂Cl₂ (10 ml) at room temperature and stirring was maintained for 3 hrs. The solvent was then removed under reduced pressure. To this residue was added CH₂Cl₂ (15 ml) and the solution was cooled to 0°C. To this was added 4-nitrophenyl chloroformate (1.16 gm) and then DIEA (2.7 ml) was added dropwise. Stirring was continued for 3.5 hrs as the reaction warmed to room temperature. The reaction mixture was the partitioned between 0.5M HCl (30 ml) and more CH₂Cl₂ (20 ml). The HCl was then washed with CH₂Cl₂ (20 ml). The combined CH₂Cl₂ extracts were then washed with brine (20 ml), dried over MgSO₄, and evaporated *in vacuo.* This crude material was purified using flash chromatography (hexane/EtOAc, 3:1) thus affording the desired product (1.36 gm, 74%).
MS (DCI) 353 (M+H)⁺

### Step D: Preparation of ethyl 2-[(1-{[9-(4-pyridyl)-3,9-diazaspiro[5.5]undec-3-yl]carbonyl } -4-piperidyl)oxy]acetate.

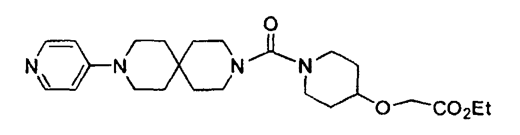

To the material obtained in Example 1, Step A (100 mg) at room temperature under argon was added 40% TFA in CH₂Cl₂ (3 ml) and stirring was maintained for 0.5 hrs. The solvent was then removed under reduced pressure. To this residue was added DMF (2 ml) at room temperature under argon followed by the material from Step C of this example (116 mg) and then DIEA (0.31 ml). This solution was then warmed to 100°C and stirred for 2 days. After cooling to room temperature, brine (10 ml) was added followed by H₂O (2 ml). This solution was washed with EtOAc (2 x 15 ml), and the EtOAc was then dried with Na₂SO₄ and evaporated. The crude product was purified with
RP-HPLC.
MS (ES) 445 (M+H)⁺

### Step E: Preparation of the title compound.

The ester obtained above was readily hydrolyzed while lyophilizing due to a defrosting of the sample. It was purified utilizing RP-HPLC.
MS (ES) 417 (M+H)⁺
ELISA: IC₅₀=0.129 µM PRP: IC₅₀=2.65 µM

### Example 12

### Preparation of 3-oxo-3-(4-{[9-(4-pyridyl)-3,9-diazaspiro[5.5]undec-3-yl]carbonyl} piperidino)propanoic acid.

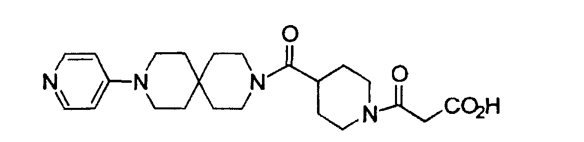

### Step A: Preparation of 4-benzyl 1-(tert-butyl) 1,4-piperidinedicarboxylate.

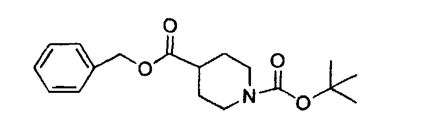

To 1-(*tert*-butoxycarbonyl)-4-piperidinecarboxylic acid (5 gm) in CH₂Cl₂ (40 ml) at room temperature under argon was added DCC (5.4 gm), DMAP (0.8 gm), and then benzyl alcohol (22.6 ml). The mixture was then stirred overnight. After evaporation of the solvent under reduced pressure, the residue was purified using dry column chromatography (2-3 column volumes of Hexane/EtOAc, 9:1) to afford the desired product (4.92 gm, 70%).
MS (CI) 320 (M+H)⁺

### Step B: Preparation of benzyl 1-(3-ethoxy-3-oxopropanoyl)-4-piperidine carboxylate.

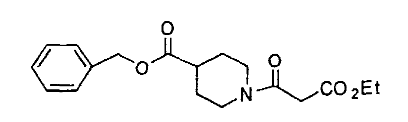

Treatment of the material from Step A in this example (4.9 gm) with neat TFA (25 ml) at 0°C for ½ hr followed by evaporation under vacuum gave 7.4 gm of the deprotected amine. To this material (1.4 gm) was added CH₂Cl₂ (6 ml) under argon followed by cooling to 0°C. Then DIEA (5 ml) was added slowly followed by slow dropwise addition of ethyl malonyl chloride (1.1 gm). After stirring overnight as it warmed to room temperature, the reaction mixture was mixed with saturated NaHCO₃ (15 ml). This mixture was washed with CH₂Cl₂ (3 x 30 ml) and the CH₂Cl₂ was then dried with MgSO₄ and evaporated under vacuum. The crude product was purified using flash chromatography (gradient elution from 10-30% EtOAc in hexane) to afford the desired material (0.56 gm). MS (CI) 334 (M+H)⁺

### Step C: Preparation of 1-(3-ethoxy-3-oxopropanoyl)-4-piperidinecarboxylic acid.

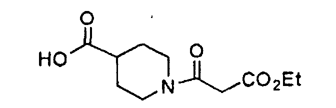

The benzyl ester (0.56 gm) from Step B of this example was dissolved in EtOH (30 ml) at room temperature. Then Pd(OH)₂/C (Pearlman's Catalyst, 100 mg) was added and the solution was shaken on a Parr hydrogenation apparatus at 50 psi of H₂ for 2.5 hrs. Filtration of the catalyst through Celite and evaporation of the solvent under vacuum gave the desired acid (0.392 gm, 96%).
MS (ES) 244 (M+H)⁺

### Step D: Preparation of ethyl 3-oxo-3-(4-{[9-(4-pyridyl)-3,9-diazaspiro[5.5]undec-3-yl]carbonyl}piperidino)propanoate.

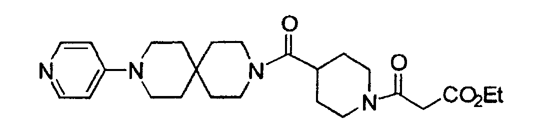

To the material obtained in Example 1, Step A (100 mg) at room temperature under argon was added 40% TFA in CH₂Cl₂ (3 ml) and stirring was maintained for 2 hrs. The solvent was then removed under reduced pressure. To this residue was added DMF (3 ml) at room temperature under argon followed by HBTU (834 mg), the acid from Step C of this example (80 mg), and then DIEA (0.31 ml). After stirring overnight at room temperature, the solution was mixed with brine (10 ml) and H₂O (3 ml). This mixture was then washed with EtOAc (2 x 20 ml). Analysis of the organic and aqueous layers revealed the desired product to be only in the H₂O, so it was lyophilized and purified via RP-HPLC to afford the target ester (27 mg, 20%).
MS (IS) 457 (M+H)⁺

### Step E: Preparation of the title compound.

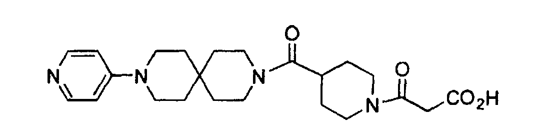

The ester from Step D in this example was hydrolyzed by substantially following the procedure from Example 5, Step B to afford the title compound as a white fluffy solid in quantitative yield.
MS (ES) 429 (M+H)⁺
ELISA: IC₅₀=0.039µM PRP: IC₅₀=1.77 µM

### Example 13

### Preparation of (2S)-2- {[(3,5-dimethyl-4-isoxazolyl)sulfonyl]amino}-3-({[3-(4-pyridyl)-3-azaspiro[5.5]undec-9-yl]carbonyl}amino)propanoic acid

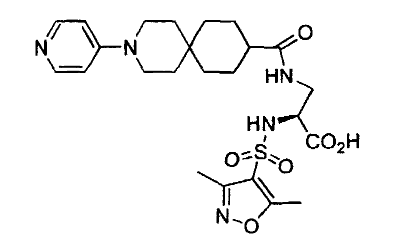

### StepA: Preparation of 3-{2[(3-Azaspiro[5.5]undecane-9-carbonyl)formic acid

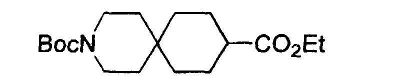

The synthesis of the starting material was accomplished as described in US patent #5,451,578 from 1-benzyl-4-piperidone. The acid is then converted to ethyl ester and deprotection of Boc in one step with EtOH/HCl(g).

### Step B: Preparation of Ethyl-3-{[3-(4-pyridyl)-3-azaspiro[5.5]undec-9-yl]carbonyl}formate

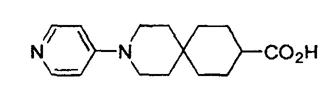

To the piperidine-ester (130mg, 0.5mmol), 4-bromopyridine (104mg, 0.535mmol), Pd₂(dba)₃ (15mg, 0.016mmol), S-BINAP (25mg, 0.04mmol), NaO-*t*-Bu (196mg, 2.04mmol), and toluene (6mL) were mixed together under argon. The reaction mixture was then heated to 90°C for 12h. The reaction mixture is then cooled to room temperature, taken up in EtOAc washed with brine. The organic layer is then dried, filtered and evaporated to give crude product. The RP-HPLC purification afforded 115mg(75%) of the desired spirocycle with pyridine. MS (ES) 275 (M+H)⁺

### Step C: Preparation of Ethyl (2S)-2-{[(3,5-dimethyl-4-isoxazolyl)sulfonyl]amino}-3-( { [3-(4-pyridyl)-3-azaspiro[5.5]undec-9-yl]carbonyl}amino)propanoate

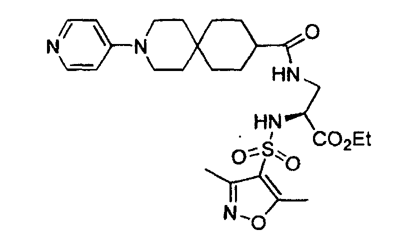

To the DMF (3mL) solution of the acid from Step B (102mg, 0.372mmol) added EDC (78mg, 0.409mmol, HOBt (568mg, 0.409mmol), and the resulting suspension was stirred for 30 min at room temperature. To this mixture was added DMF solution (2mL) of ethyl(2S)-3-amino-2- { [(3,5-dimethyl-4-isoxazolyl)sulfonyl]amino}propanoate (162mg, 0.558mmo1, synthesized as reported in US Patent No. 5648368) and DIEA (260uL, 1.48mmol). The reaction mixture was stirred at room temperature overnight. EtOAc was added and the solution was washed with 10% citric acid, brine, 10% NaHCO₃, dried, and concentrated. Purification on RP-HPLC gave 145mg of the product. MS (ES) 548 (M+H)⁺. ¹H NMR (400MHz, CD₃OD) δ 8.03 (t, 1H), 7.98 (d, 2H), 7.03 (d, 2 H), 3.97 (t, 1H), 3.92 (q, 2H), 3.60 (m, 6H), 2.49 (s, 3H), 2.29 (s, 3H), 2.10 (m,1H), 1.78-1.46 (m, 11H), 1.22 (m, 3H), 1.05 (t, 3H)

### Step D: Preparation of (2S)-2-{[(3,5-dimethyl-4-isoxazolyl)sulfonyl]amino}-3-({[3-(4-pyridyl)-3-azaspiro[5.5]undec-9-yl]carbonyl}amino)propanoic acid

The hydrolysis of ester (73mg, 0.13mmol) from Step C was carried out with 3N HCl at 60°C for 3h. The solvent was evaporated and the residue purified by RP-HPLC to afford 65mg (80%) of the product. MS (ES) 520 (M+H)⁺. ¹H NMR (400MHz, CD₃OD) δ 8.02 (t, 1H), 7.97 (d, 2H), 7.03 (d, 2 H), 3.97 (t, 1H), 3.96 (dd, 1H), 3.60 (m, 6H), 2.48 (s, 3H), 2.29 (s, 3H), 2.10 (m,1H), 1.78-1.46 (m, 11H), 1.22 (m, 3H), 1.05 (t, 3H)
ELISA: IC₅₀= 0.001 µM PRP (ADP-Citrate) IC₅₀ = 0.044 µM
PRP (ADP-PPACK) IC₅₀ = 0.068 µM

### Example 14

### Preparation of (2S)-2-{[(4-methylphenyl)sulfonyl]amino}-3-({[3-(4-pyridyl)-3-azaspiro[5.5]undec-9-yl]carbonyl}amino)propanoic acid

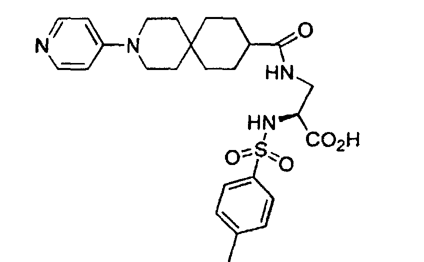

### StepA: Preparation of Ethyl (2S)-2-{[(benzyloxy)carbonyl]amino}-3-({[3-(4-pyridyl)-3-azaspiro[5.5]undec-9-yl]carbonyl}amino)propanoate

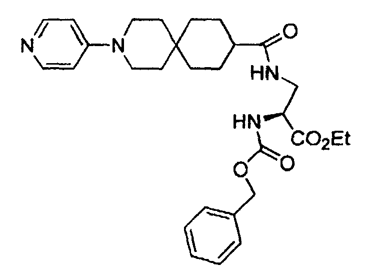

To the DMF (3mL) solution of the acid (70mg, 0.256mmol, Step B, Example 13) added DCC(74mg, 0.358mmol, HOBt (46mg, 0.332mmol), and the resulting suspension was stirred for 30 min at room temperature. To this mixture was added DMF solution (1mL) of ethyl(2S)-3-amino-2-{[(benzyloxy)carbonyl]amino}propanoate (102mg, 0.384mmol, for synthesis see US Patent 5648368). The reaction mixture was stirred at room temperature overnight, EtOAc was added and the solution was washed with 10% citric acid, brine, 10% NaHCO₃, dried, and concentrated. Purification on RP-HPLC gave 80mg(60%) of the product.
MS(ES) 523 (M+H)⁺

### Step B: Preparation of Ethyl (2S)-2-amino-3-({[3-(4-pyridyl)-3-azaspiro[5.5] undec-9-yl] carbonyl}amino)propanoate

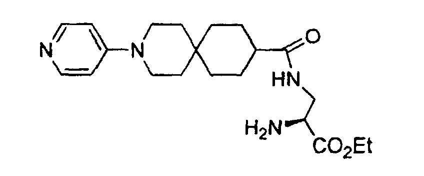

The carbobenzyloxy-protected product (56mg) from Step A was dissolved in 5 mL of EtOH, and to this added 10% Pd/C (20mg). The reaction was stirred under latm of H₂ for 4h. The catalyst was filtered through celite, and filtrate concentrated to give the product (34mg, 99%) as an oil. MS (ES) 389 (M+H)⁺.

### Step C: Preparation of Ethyl (2S)-2-{[(4-methylphenyl)sulfonyl]amino}-3-({[3-(4-pyridyl)-3-azaspiro[5.5]undec-9-yl]carbonyl} amino)propanoate

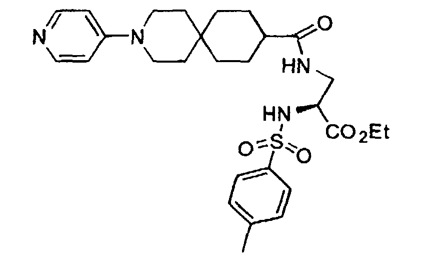

To the CH₂Cl₂ solution (2mL) of the spirocycle-aminoester (33.2mg, 0.086mmol) from Step B added DIEA (36uL, 0.256mmol) and p-toluenesulfonyl chloride (19.6mg, 0.103mmol). The resulting solution was stirred at room temperature overnight. The solution was extracted with EtOAc, washed with 10%HCI, dried, filtered, and concentrated to afford crude sulfonamide. The residue was purified by RP-HPLC to afford 30mg (64%) of the pure product.
MS (ES) 543 (M+H)⁺

### Step D: Preparation of (2S)-2-{[(4-methylphenyl)sulfonyl]amino}-3-({[3-(4-pyridyl)-3-azaspiro[5.5]undec-9-yl]carbonyl}amino)propanoic acid

The hydrolysis of the ester (30mg, 0.055mmol) from step C was carried out with 3N HCl at 60°C for 6h. The solvent was evaporated and the residue purified by RP-HPLC to afford 25mg (88%) of the product. MS (ES) 515 (M+H)⁺
ELISA: IC₅₀= 0.001 µM PRP (ADP-Citrate) IC₅₀ = 0.098 µM
PRP (ADP-PPACK) IC₅₀ = 0.169 µM

### Example 15

### Preparation of (2S)-2-{[(butylsulfonyl)amino}-3-({[3-(4-pyridyl)-3-azaspiro[5.5]undec-9-yl]carbonyl}amino)propanoic acid

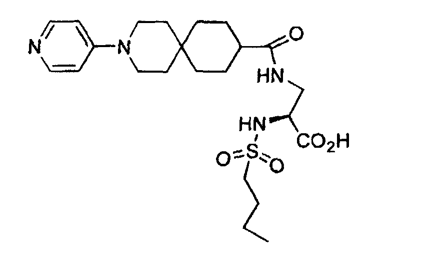

### Step A: Preparation of Ethyl (2S)-2-{[{butylsulfonyl)amino}-3-({[3-(4-pyridyl)-3-azaspiro[5.5]undec-9-yl]carbonyl} amino)propanoate

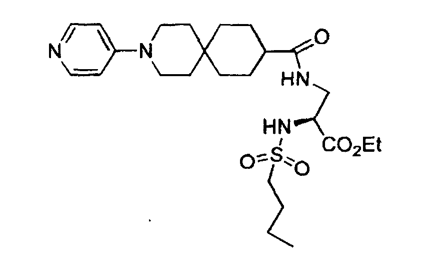

To the CH₂Cl₂ solution (5mL) of Ethyl (2S)-2-amino-3-({[3-(4-pyridyl)-3-azaspiro[5-5]undec-9-yl]carbonyl}amino)propanoate (69mg, 0.178mmol) from Step B of Example 14, added DIEA (78uL, 0.444mmol) and n-butylsulfonyl chloride (51uL, 0.391mmol). The reaction was stirred at room temperature overnight. The solution was extracted with EtOAc, washed with 10%HCl, dried, filtered, and concentrated to afford crude butylsulfonamide. The residue was purified by RP-HPLC to afford 35mg (40%) of the pure product. MS (ES) 509 (M+H)⁺. ¹H NMR (400MHz, CD₃OD) δ 7.98(d, 2H), 7.03 (d, 2H), 4.1 (q, 2H), 3.59 (m,3H), 3.53-3.48 (m, 1H), 3.33-3.30 (m, 1H), 2.97 (t, 2H), 2.10 (m, 1H), 1.77-1.33 (m, 10H), 1.23 (t, 4H), 0.89 (t, 3H)

### Step B: Preparation of (2S)-2-{[(butylsulfonyl)amino}-3-({[3-(4-pyridyl)-3-azaspiro[5.5]undec-9-yl]carbonyl}amino)propanoic acid

The hydrolysis of ester (30mg, 0.055mmol) from step C was carried out with 3N HCl at 60°C for 6h. The solvent was evaporated and the residue purified by RP-HPLC to afford 25mg (88%) of the product. MS (ES) 481 (M+H)⁺. ¹H NMR (400MHz, CD₃OD) δ 7.95(d, 2H), 7.01 (d, 2H), 4.06 (m, 1H), 3.56 (m,5H), 3.24-3.19 (m, 3H), 2.95 (t, 2H), 2.10 (m, 1H), 1.77-1.58 (m, 16H), 0.85 (t, 3H)
ELISA: IC₅₀= 0.010 µM PRP (ADP) IC₅₀ = 0.168 µM
PRP (PPACK) IC₅₀ = 0.686 µM

### Example 16

### Preparation of (2S)-2-{[(butyloxyl)carbonyl]amino}-3-({[3-(4-pyridyl)-3-azaspiro[5.5] undec-9-yl]carbonyl}amino)propanoic acid

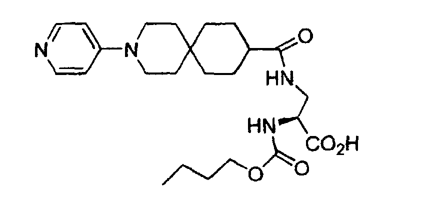

### Step A: Preparation of Ethyl-(2S)-2{[(butyloxyl)carbonyl]amino}-3-({[3-(4-pyridyl)-3-azaspiro[5.5]undec-9-yl]carbonyl}amino)propanoate

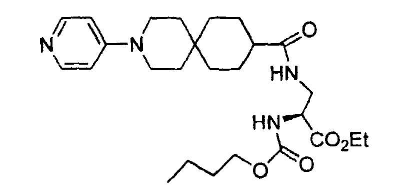

To the DMF solution (3mL) of 3-{[3-(4-pyridyl)-3-azaspiro[5.5]undec-9-yl]carbonyl}formic acid (104mg, 0.380mmol) added EDC (80mg, 0.418mmol), HOBt (56mg, 0.418mmol), DIEA (330uL, 1.90mmol), and the resulting suspension was stirred for 30 min at room temperature. To this added DMF solution (2mL) of Ethyl(2S)-3-amino-2{[(butyloxy)carbonyl]amino}propanoate (132mg, 0.570mmol) and stirred the reaction mixture at room temperature for 24h. Following dilution with EtOAc it was washed with 10%HCl, 10%NaHCO₃, sat'd brine, dried, filtered and evaporated to give the crude product. This was purified by RP-HPLC to give 70mg (38%) of the desired product. MS (ES) 489 (M+H)⁺. ¹H NMR (400MHz, CD₃OD) δ 7.98 (d, 2H), 7.03 (d, 2H), 4.21 (t, 1H), 4.10 (bt, 2H), 3,97 (t, 2H), 3.59-3.40 (m, 6H), 2.10 (m, 1H), 1.77-1.46 (m, 9H), 1.32 (m, 1H), 1.20 (t, 4H), 0.87 (t, 3H)

### Step B: Preparation of (2S)-2-{[(butyloxyl)carbonyl]amino}-3-({[3-(4-pyridyl)-3-azaspiro[5.5]undec-9-yl]carbonyl }amino)propanoic acid

The hydrolysis of the ester (30mg, 0.06mmol) from Step A was carried out with 3N HCl at 60°C for 3h. The solvent was evaporated and the residue purified by RP-HPLC to afford 20mg (72%) of the product.
MS (ES) 461 (M+H)⁺
PRP (ADP) IC₅₀ = 1.88 µM

### Example 17

### Preparation of 3-({[3-(4-pyridyl)-3-azaspiro[5.5]undec-9-yl]carbonyl}amino)butanoic acid

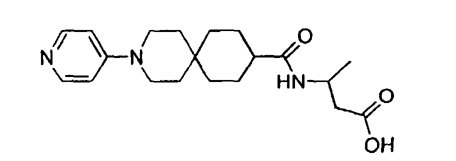

### Step A: Preparation of DL-Ethyl-3-aminobutyrate

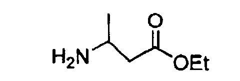

To the commercially available Boc-DL-3-aminobutyric acid (684mg, 3.37mmol) in EtOH (15mL) added thionyl chloride (614uL, 8.42mmol) and the mixture was refluxed for 3h. After cooling the solvent was evaporated to afford the desired ester as a white solid.
MS (ES) 132 (M+H)⁺

### Step B: Preparation of Ethyl-3-([3-(4-pyridyl)-3-azaspiro[5.5]undec-9-yl] carbonylamino)butanoate

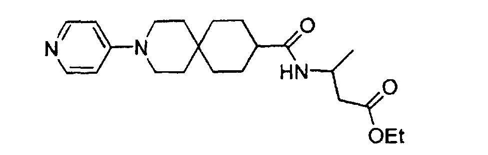

To the DMF solution (2mL) of 3-{[3-(4-pyridyl)-3-azaspiro[5.5]undec-9-yl)carbonyl}formic acid (51mg, 0.186mmol) added EDC (40mg, 0.205mmol), HOBt (28mg, 0.205mmol), DIEA (162uL, 0.93mmol), and the resulting suspension was stirred for 30 min at room temperature. To this added DMF solution (1mL) of Ethyl(DL)-3-aminobutyrate (36.6mg, 0.279mmol) and stirred the reaction mixture at room temperature for 18h. Following dilution with EtOAc it was washed with 10%HCl, 10%NaHCO₃, sat'd brine, dried, filtered and evaporated to give the crude product. This was purified by RP-HPLC to give 40mg (55%) of the desired product. MS (ES) 388 (M+H)⁺. ¹H NMR (400MHz, CD₃OD) δ 7.98 (d, 2H), 7.02 (d, 2H), 4.19 (m, 1H), 4.04 (q, 2H), 3.59 -3.56 (m, 4H), 2.39 (t, 2H), 2.06 (m, 1H), 1.76-1.43 (m, 11H), 1.16 (t, 3H), 1.09 (d, 6H)

### Step C: Preparation of 3-([3-(4-pyridyl)-3-azaspiro[5.5]undec-9-yl] carbonylamino)butanoic acid

The hydrolysis of the ester (20mg, 0.05mmol) from Step B was carried out with 3N HCl at 60°C for 3h. The solvent was evaporated and the residue purified by RP-HPLC to afford 15mg (82%) of the product. MS (ES) 360 (M+H)⁺. ¹H NMR (400MHz, CD₃OD) δ 7.73 (d, 2H), 6.78 (d, 2H), 3.92 (m, 111), 3.36-3.25 (m, 4H), 2.15-2.09 (m, 2H), 1.87 (m, 1H), 1.56-1.22 (m, 11H), 0.85 (d, 6H)
PRP (ADP) ICₛₒ = 14.8 µM

### Example 18

### Preparation of 3-methyl-4-( { [3-(4-pyridyl)-3-azaspiro[5.5]undec-9-yl]carbonyl}amino) butanoic acid

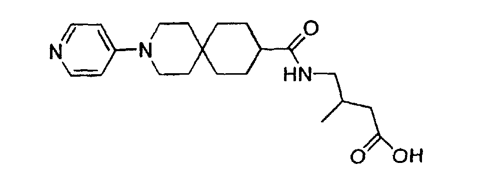

### Step A: Preparation of ethyl 4-amino-3-methyl butanoate

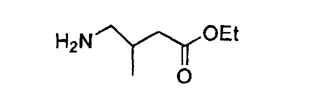

To ethyl trans crotonate (1g, 124.35mmol) added nitromethane (34mL,621.7mmol), tetramethyl guanidine (3.1mL, 25mmol), and stirred the reaction at room temperature for 18h. To this added EtOAc, washed with 10% HCI, brine, dried, filtered and evaporated to give crude residue. The crude material was chromatographed on silica gel (20% EtOAc/Hexane) to afford desired product as colorless oil (60%). MS(ES) 176 (M+H)⁺ The colorless oil (456mg, 2.60mmol) was suspended in glacial acetic acid (8mL), to this added 10%Pd/C (100mg) and was stirred overnight under latm of H₂. The reaction mixture was filtered through celite and evaporated out the acetic acid to afford the desired amino-ester (350mg, 92%). MS (ES) 146 (M+H)⁺

### Step B: Preparation of Ethyl 3-methyl-4-({[3-(4-pyridyl)-3-azaspiro[5.5]undec-9-yl]carbonyl } amino)butanoate

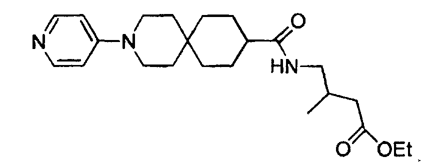

To the DMF solution (2mL) of 3-{[3-(4-pyridyl)-3-azaspiro[5.5]undec-9-yl]carbonyl}formic acid (41mg, 0.150mmol) added EDC (32mg, 0.165mmol), HOBt (22mg, 0.165mmol), DIEA (131uL, 0.75mmol), and the resulting suspension was stirred for 30 min at room temperature. To this added DMF solution (1mL) of ethyl 4-amino-3-methyl butanoate (33mg, 0.225mmol) and stirred the reaction mixture at room temperature for 18h. The solvent was evaporated and the residue was purified by RP-HPLC to give 40mg (66%) of the desired product. MS (ES) 402 (M+H)⁺

### Step C: Preparation of 3-methyl-4-({[3-(4-pyridyl)-3-azaspiro[5.5]undec-9-yl]carbonyl}amino)butanoic acid

The hydrolysis of the ester (20mg, 0.05mmol) from Step B was carried out with 3N HCl at 60°C for 3h. The solvent was evaporated and the residue purified by RP-HPLC to afford 15mg (82%) of the product. MS (ES) 374 (M+H)⁺. ¹H NMR (400MHz, CD₃OD) δ 7.97 (d, 2H), 7.02 (d, 2H), 3.58-3.54 (m, 4H), 3.01 (d, 3H), 2.30-1.97 (m, 6H), 1.87 (m, 111), 1.76-1.42 (m, 12H), 1.18 (m, 4H), 0.87 (d, 3H)
PRP (ADP) IC₅₀ = 3 µM

### Example 19

Preparation of 3-oxo-3-{4-{[8-(4-pyridyl)-2,8-diazaspiro[4.5]dec-2-yl]carbonyl} piperidino)propanoic acid:

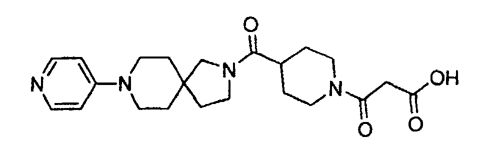

### Step A: Preparation of 8-benzyl-2-oxa-8-azaspiro[4.5]decane- 1,3 -dione

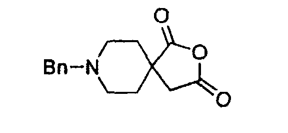

### The synthesis of this material was accomplished as described in WO 97/11940

### Step B: Preparation of 1-benzyl-4-{2-[(4-methoxybenzyl)amino]-2-oxoethyl}-4-piperidinecarboxylic acid

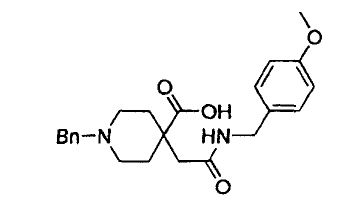

The anhydride from Step A (18.8 gm. 72.3 mmol) was dissolved in 250 ml of anhydrous acetonitrile. To this solution was added (4-methoxyphenyl)methylamine (10.4 ml, 79.5 mmol, commercially available) and the reaction was stirred overnight at room temperature. The solvent was evaporated yielding 32.6 gm (>100%) of the product.
MS (ES) 397 (M+H)⁺

### Step C: Preparation of 8-benzyl-2-(4-methoxybenzyl)-2,8-diazaspiro[4.5]decane-1,3-dione

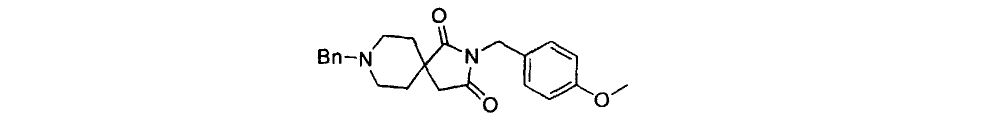

The material from Step B (32.6 gm, 82.2 mmol) was dissolved in 300 ml of acetic anhydride. To this solution was added sodium acetate (33.7 gm, 411 mmol). After refluxing overnight, the reaction was filtered to remove solids and the solvent evaporated. The residue was purified by flash chromatography yielding 18.3 gm (61%) of the product.
MS (ES) 379 (M+H)⁺

### Step D: Preparation of 8-benzyl-2-(4-methoxybenzyl)-2,8-diazaspiro[4.5)decane

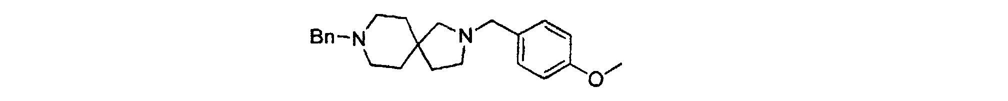

The material from Step C was dissolved in 100 ml of anhydrous THF. BH₃-THF complex (686 ml, 686 mmol) was then slowly added, and the mixture was refluxed overnight under argon. The reaction was cooled to 0°C and quenched with water. The organic solvent was evaporated and the pH raised to 10 with conc. NaOH. The aqueous phase was saturated with NaCl and extracted 3x with EtOAc. The organic phases were combined and the solvent evaporated to afford 6.4 gm (66%) of product.
MS (ES) 351 (M+H)⁺

### Step E: Preparation of 8-benzyl-2,8-diazaspiro[4.5]decane

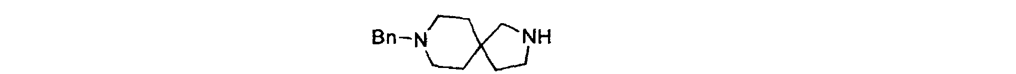

The product from Step D was dissolved in 91.5 ml of 5% H₂O/acetonitrile and cooled to 0°C. Ammonium cerium (IV) nitrate (30 gm, 55mmol) was slowly added and the reaction was allowed to warm to room temperature overnight. The solvent was evaporated and the residue purified using RP-HPLC yielding 2.4 gm (57%) of product.
MS (ES) 231 (M+H)⁺

### Step F: Preparation of tert-butyl 8-benzyl-2,8-diazaspiro[4.5]decane-2-carboxylate

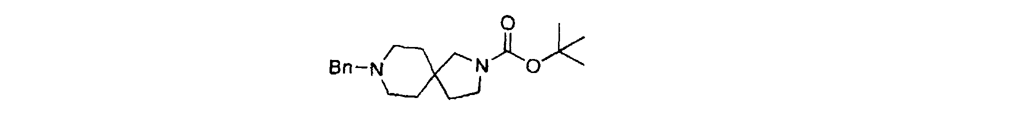

The product from Step E was dissolved in 30 ml of CH₂Cl₂. To this solution was added DIEA (5.4 ml, 31.4 mmol), DMAP (12.1 mg, 0.1 mmol), and di-*tert*-butyl dicarbonate (2.5 gm, 11.5 mmol). The reaction mixture was stirred overnight at room temperature. The solvent was evaporated and the residue purified by RP-HPLC yielding 1.4 gm (29%) of the product.
MS (ES) 331 (M+H)⁺

### Step G: Preparation of tert-butyl 2,8-diazaspiro[4.5]decane-2-carboxylate

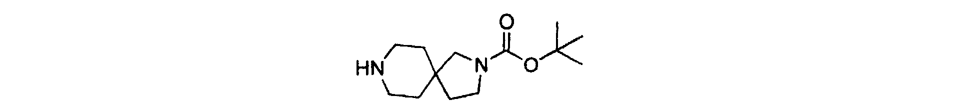

The product from Step F was dissolved in 20 ml of methanol. To this solution was added Pd(OH)₂/C (0.14gm). The reaction mixture was then hydrogenated overnight at 500 psi, filtered through celite, and the solvent evaporated to afford 1.0 gm (100%) of the product.
MS (ES) 241 (M+H)⁺

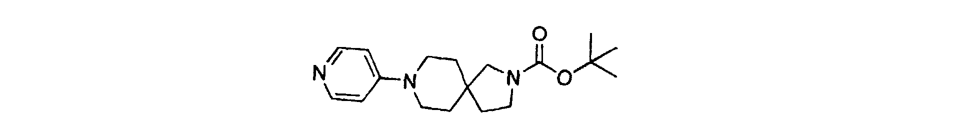

### Step II: Preparation of tert-butyl 8-(4-pyridyl)-2,8-diazaspiro[4.5]decane-2-carboxylate

To the product from Step G was added 4-bromopyridine hydrochloride (877 mg, 4.5 mmol), s-BINAP (0.105 mg, 0.17 mmol), sodium *tert* butoxide (788 mg, 8.2 mmol), and tris(dibenzylideneacetone)-dipalladium(0)-chloroform adduct (70 mg, 0.068 mmol). Then 25 ml of anhydrous toluene was added under argon. The reaction mixture was heated to 100°C and stirred overnight. After cooling to room temperature, EtOAc (50 ml) was added to the reaction mixture. The solution was filtered through celite and the solvent evaporated. The residue was purified by RP-HPLC yielding 291 mg (22%) of the desired product.
MS (ES) 318 (M+H)⁺

### Step 1: Preparation of 8-(4-pyridyl)-2,8-diazaspiro(4.5]decane

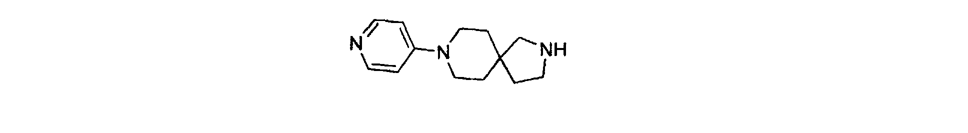

The product from Step H (35 mg, 0.16 mmol) was dissolved in 50 % TFA/CH₂Cl₂ (10 ml) and stirred at room temperature for 30 minutes. The solvent was evaporated to yield 37 mg (100%) of product.
MS (ES) 231 (M+H)⁺

### Step J: Preparation of ethyl 3-oxo-3-(4-{[8-(4-pyridyl)-2,8-diazaspiro[4.5]dec-2-yl]carbonyl}piperidino)propanoate

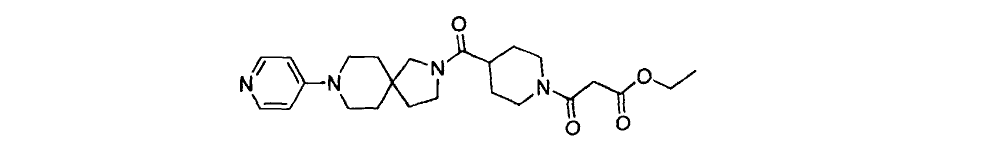

The product from Step I (37 mg, 0.16 mmol) was dissolved in DMF (4.0 ml). To this solution was added DIEA (0.24ml, 1.4 mmol), HBTU (95 mg, 0.253 mmol), and 1-(3-ethoxy-3-oxopropanoyl)-4-piperidinecarboxylic acid (62 mg, 0.253 mmol) (see Step C Example 12). The reaction mixture was stirred at room temperature overnight. The solvent was evaporated and the residue was purified by RP-HPLC to afford 25 mg (36%) of the product.
MS (ES) 443 (M+H)⁺

### Step K: Preparation of 3-oxo-3-(4- { [8-(4-pyridyl)-2,8-diazaspiro[4.5]dec-2-yl]carbonyl } piperidino)propanoic acid

The ester from Step J (12.1 mg, 0.027 mmol) was dissolved in 4.0 ml of dioxane. To this solution was added 1M LiOH (0.164mL, 0.164 mmol), and the resulting reaction mixture was stirred overnight at room temperature. The solvent was evaporated and the residue purified by RP-HPLC to afford 3.9 mg (34%) of the title compound.
MS (ES) 415 (M+H)⁺
ELISA IC₅₀=0.028 µM PRP (ADP-Citrate) IC₅₀=1.6 µM

### Example 20

### Preparation of (25)-2-{[(benzyloxy)carbonyl]amino}-5-oxo-5-[8-(4-pyridyl)-2,8-diazaspiro[4.5]dec-2-yl]pentanoic acid:

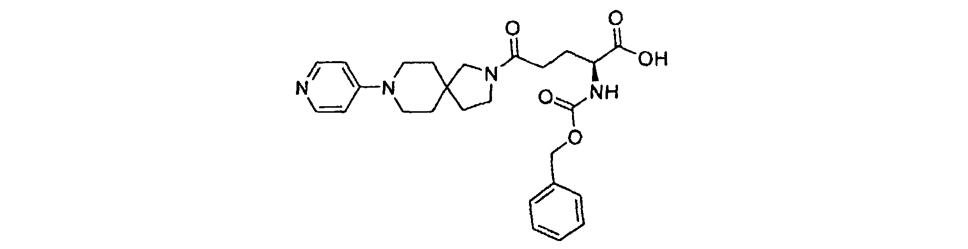

### Step A: Preparation of ethyl (2S)-2-{[(benzyloxy)carbonyl]amino}-5-oxo-5-[8-(4-pyridyl)-2,8-diazaspiro[4.5]dec-2-yl]pentanoate

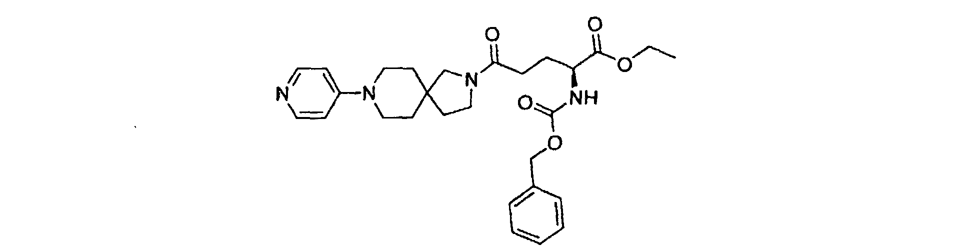

The product from Step I, Example 19 (137 mg, 0.63 mmol) was dissolved in DMF (4.0 ml). To this solution was added DIEA (0.658 ml, 3.8 mmol), HBTU (263 mg, 0.693 mmol), and (4S)-4-{[(benzyloxy)carbonyl]amino}-5-ethoxy-5-oxopentanoic acid (214 mg, 0.693 mmol) (see Example 40). The reaction mixture was stirred at room temperature overnight. The solvent was evaporated and the residue was purified by RP-HPLC to afford 70 mg (23%) of the product.
MS (ES) 508 (M+H)⁺

### Step B: Preparation of (2S)-2-{[(benzyloxy)carbonyl]amino}-5-oxo-5-[8-(4-pyridyl)-2,8-diazaspiro[4.5]dec-2-yl]pentanoic acid

The ester from Step A (70 mg, 0.138 mmol) was dissolved in 10.0 ml of dioxane. To this solution was added 1M LiOH (0.827 ml, 0.827 mmol), and the resulting reaction mixture was stirred overnight at room temperature. The solvent was evaporated and the residue purified by RP-HPLC to afford 25 mg (38%) of the title compound.
MS (ES) 415 (M+H)⁺
ELISA IC₅₀=0.012 µM PRP (ADP-Citrate) IC₅₀=9.1 µM

### Example 21

### Preparation of (2S)-2-[(butoxycarbonyl)amino]-5-oxo-5-[8-(4-pyridyl)-2,8-diazaspiro [4.5]dec-2-yl]pentanoic acid:

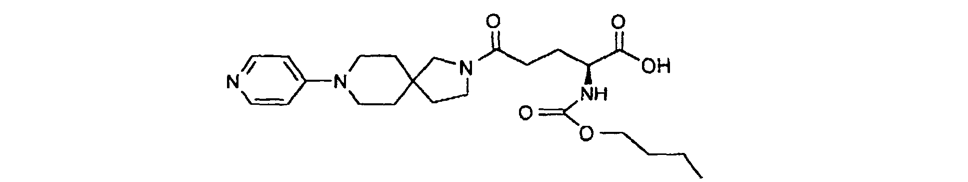

### Step A: Preparation of ethyl (2S)-2-[(butoxycarbonyl)amino]-5-oxo-5-[8-(4-pyridyl)-2,8-diazaspiro[4.5]dec-2-yl]pentanoate

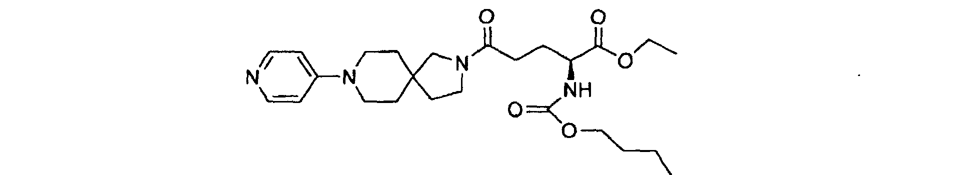

The product from Step I, Example 19 (35 mg, 0.160 mmol) was dissolved in DMF (4.0 ml). To this solution was added DIEA (0.240mL, 0.14 mmol), HBTU (95 mg, 0.23 mmol), and (4S)-5-ethoxy-4-[(butoxycarbonyl)amino]-5-oxopentanoic acid (78 mg, 0.253 mmol) (see Step D Example 36). The reaction mixture was stirred at room temperature overnight. The solvent was evaporated and the residue was purified by RP-HPLC to afford 47 mg (43%) of the product.
MS (ES) 475 (M+H)⁺

### Step B: Preparation of (2S)-2-[(butoxycarbonyl)amino]-5-oxo-5-[8-(4-pyridyl)-2,8-diazaspiro[4.5]dec-2-yl]pentanoic acid

The ester from Step A in this example (47 mg, 0.099 mmol) was dissolved in 10.0 ml of dioxane. To this solution was added 1M LiOH (0.595 ml, 0.595 mmol) and the resulting reaction mixture stirred overnight at room temperature. The solvent was evaporated and the residue purified by RP-HPLC to afford 18 mg (41%) of the title compound.
MS (ES) 447 (M+H)⁺
ELISA IC₅₀=0.162 µM PRP (ADP-Citrate) IC₅₀=5.7 µM

### Example 22

### Preparation of 6-oxo-6-(8-(4-pyridyl)-2,8-diazaspiro[4.5]dec-2-yl)hexanoic acid:

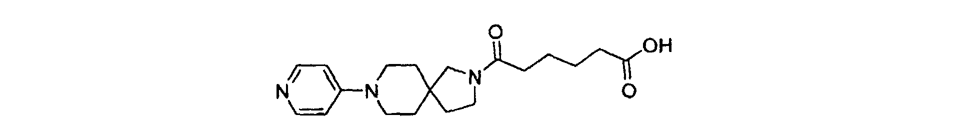

### Step A: Preparation of methyl 6-oxo-6-(8-(4-pyridyl)-2,8-diazaspiro[4.5]dec-2-yl)hexanoate

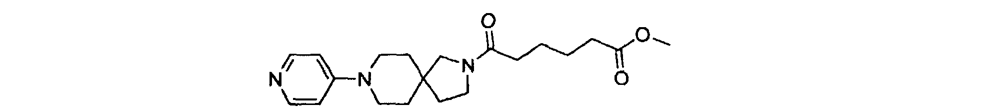

The product from Step I, Example 19 (50 mg, 0.230mmol) was dissolved in anhydrous CH₂Cl₂ (2.0 ml). To this solution was added DIEA (0.200 ml, 1.1 mmol) and methyl adipyl chloride (37.5 mg, 0.208 mmol). The reaction mixture was stirred at room temperature overnight under argon. The solvent was evaporated, and the residue was purified by RP-HPLC to afford 8 mg (36%) of the product.
MS (ES) 360 (M+H).

### Step B: Preparation of 6-oxo-6-(8-(4-pyridyl)-2,8-diazaspiro[4.5]dec-2-yl)hexanoic acid

The ester from Step A of this example (8 mg, 0.022 mmol) was dissolved in 2.0 ml of 2N HCl and stirred for 2 hours at room temperature. The solvent was evaporated and the residue purified by RP-HPLC to afford 6.6 mg (87%) of the title compound.
MS (ES) 346 (M+H)⁺
PRP (ADP-Citrate) IC₅₀= >80 µM

### Example 23

### Preparation of 8-oxo-8-(8-(4-pyridyl)-2,8-diazaspiro[4.5]dec-2-yl)octanoic acid:

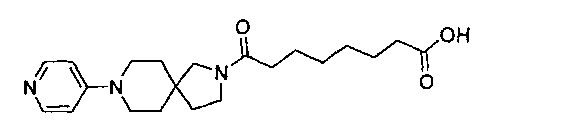

### Step A: Preparation of methyl 8-oxo-8-(8-(4-pyridyl)-2,8-diazaspiro[4.5)dec-2-yl)octanoate

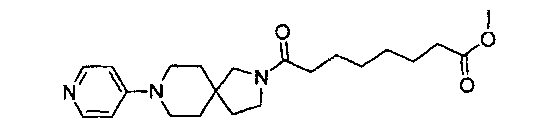

The product from Step I Example 19 (50 mg, 0.230mmol) was dissolved in anhydrous CH₂Cl₂ (2.0 ml). To this solution was added DIEA (0.200 ml, 1.1 mmol) and methyl 7-(chlorocarbonyl)heptanoate (42 mg, 0.208 mmol). The reaction mixture was stirred at room temperature overnight under argon. The solvent was evaporated and the residue was purified by RP-HPLC to afford 49.5 mg (81%) of the product.
MS (ES) 360 (M+H)⁺

### Step B: Preparation of 8-oxo-8-(8-(4-pyridyl)-2,8-diazaspiro[4.5]dec-2-yl)octanoic acid

The ester from Step A (25 mg, 0.064 mmol) was dissolved in 2.0 ml of MeOH. 1M LiOH (0.260 ml, 0.26 mmol) was added and the reaction stirred for 2 hours at room temperature. The solvent was evaporated and the residue purified by RP-HPLC to afford 16.3 mg (67%) of the title compound.
MS (ES) 374 (M+H)⁺
ELISA IC₅₀=0.196 µM PRP (ADP-Citrate) IC₅₀=18.7 µM

### Example 24

### Preparation of 7-oxo-7-(8-(4-pyridyl)-2,8-diazaspiro[4.5]dec-2-yl)heptanoic acid:

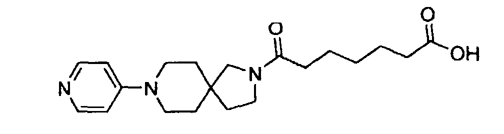

### Step A: Preparation of ethyl 7-oxo-7-(8-(4-pyridyl)-2,8-diazaspiro[4.5]dec-2-yl)heptanoate

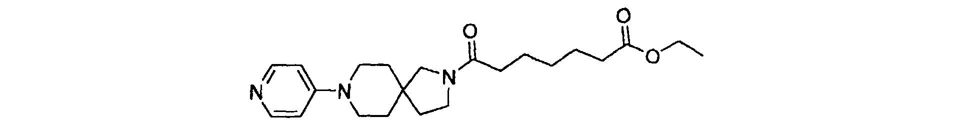

The product from Step I Example 19 (50 mg, 0.230mmol) was dissolved in anhydrous CH₂Cl₂ (2.0 ml). To this solution was added DIEA (0.200 ml, 1.1 mmol) and ethyl 6-(chlorocarbonyl)hexanoate (42 mg, 0.208 mmol). The reaction mixture was stirred at room temperature overnight under argon. The solvent was evaporated and the residue was purified by RP-HPLC to afford 66 mg (91%) of the product.
MS (ES) 388 (M+H)⁺

### Step B: Preparation of 7-oxo-7-(8-(4-pyridyl)-2,8-diazaspiro[4.5]dec-2-yl)heptanoic acid

The ester from Step A (33 mg, 0.086 mmol) was dissolved in 2.0 ml of EtOH. 1M LiOH (0.342 ml, 0.342 mmol) was added and the reaction stirred for 2 hours at room temperature. The solvent was evaporated and the residue purified by RP-HPLC to afford 27 mg (89%) of the title compound. MS (ES) 360 (M+H)⁺
ELISA IC₅₀=0.016 µM PRP (ADP-Citrate) IC₅₀=1.3 µM

### Example 25

### Preparation of 3-(2-oxo-4-((8-(4-pyridyl)-2,8-diazaspiro[4.5]dec-2-yl)carbonyl) piperazino)propanoic acid:

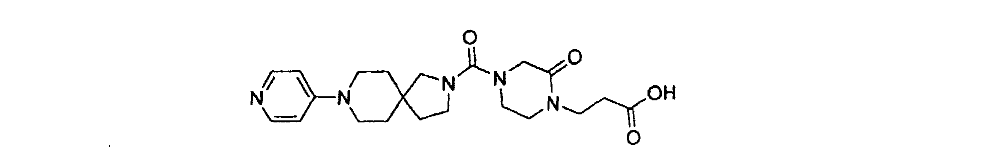

### Step A: Preparation of ethyl 3-(2-oxo-4-((8-(4-pyridyl)-2,8-diazaspiro[4.5]dec-2-yl)carbonyl)piperazino)propanoate

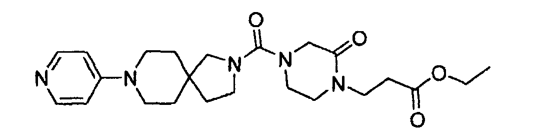

The product from Step I Example 19 (19 mg, 0.091 mmol) was dissolved in anhydrous DMF (2.0 ml). To this solution was added DIEA (0.064 ml, 0.365 mmol) and ethyl 3-{4-[(4-nitrophenyl)oxycarbonyl]-2-oxopiperazinyl}propanoate (37 mg, 0.1 mmol) (see Step C, Example 30). The reaction mixture was stirred overnight at 100°C under argon. The solvent was evaporated and the residue was purified by RP-HPLC to afford 27 mg (67%) of the product.
MS (ES) 444 (M+H)⁺

### Step B: Preparation of 3-(2-oxo-4-((8-(4-pyridyl)-2,8-diazaspiro[4.5]dec-2-yl)carbonyl)piperazino)propanoic acid

The ester from Step A (13.5 mg, 0.03 mmol) was dissolved in 2.0 ml of dioxane. 1M NaOH (2.0 ml) was added and the reaction stirred for 2 hours at room temperature. The solvent was evaporated and the residue purified by RP-HPLC to afford 6.5 mg (72%) of the title compound. MS (ES) 416 (M+H)⁺
ELISA IC₅₀=0.112 µM PRP (ADP-Citrate) IC₅₀=8.3 µM

### Example 26

### Preparation of 2-((1-((8-(4-pyridyl)-2,8-diazaspiro[4.5]dec-2-yl)carbonyl)-4-piperidyl)oxy)acetic acid:

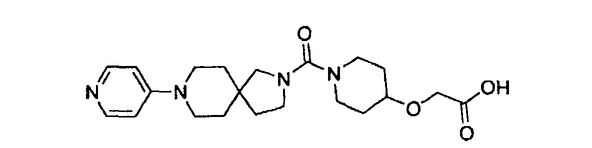

### Step A: Preparation of ethyl 2-((1-((8-(4-pyridyl)-2,8-diazaspiro[4.5]dec-2-yl)carbonyl)-4-piperidyl)oxy)acetate

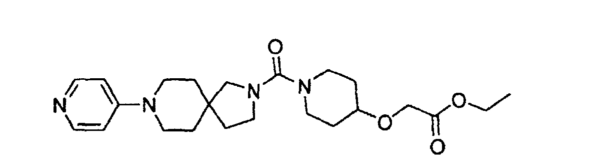

The product from Step I Example 19 (37 mg, 0.16 mmol) was dissolved in anhydrous DMF (2.0 ml). To this solution was added DIEA (0.24 ml, 1.4 mmol) and ethyl 2-{ 1 -[(4-nitrophenyl)oxycarbonyl]-3-oxo-4-piperidyloxy}acetate (89 mg, 0.253 mmol) (see Step C Example 11) The reaction mixture was stirred overnight at 100°C under argon. The solvent was evaporated and the residue was purified by RP-HPLC to afford 10 mg (9.6%) of the product.
MS (ES) 445 (M+H)⁺

### Step B: Preparation of 2-((1-((8-(4-pyridyl)-2,8-diazaspiro[4.5]dec-2-yl)carbonyl)-4-piperidyl)oxy)acetic acid

The ester from Step A (10 mg, 0.023 mmol) was dissolved in 1.0 ml of dioxane. 1M LiOH (0.137 ml, 0.137 mmol) was added and the reaction stirred for 2 hours at room temperature. The solvent was evaporated and the residue purified by RP-HPLC to afford 6.0 mg (65%) of the title compound.
MS (ES) 416 (M+H)⁺
ELISA IC₅₀=0.457 µM PRP (ADP-Citrate) IC₅₀=1.0 µM

### Example 27

### Preparation of 2-(2-oxo-4-((8-(4-pyridyl)-2,8-diazaspiro[4.5]dec-2-yl)carbonyl)-4-piperazino)acetic acid:

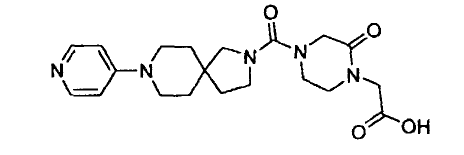

### Step A: Preparation of ethyl 2-(2-oxo-4-((8-(4-pyridyl)-2,8-diazaspiro[4.5]dec-2-yl)carbonyl)-4-piperazino)acetate

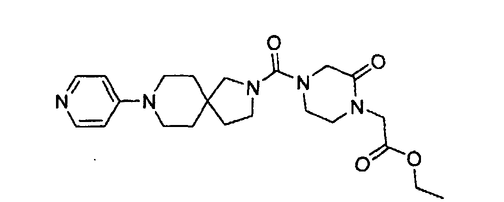

The product from Step I Example 19 (37 mg, 0.16 mmol) was dissolved in anhydrous DMF (2.0 ml). To this solution was added DIEA (0.182 ml, 0.105 mmol) and ethyl 2-{4-[(4-nitrophenyl)oxycarbonyl]-2-oxopiperazinyl}acetate (61 mg, 0.174 mmol) (see Step A Example 29). The reaction mixture was stirred overnight at 100°C under argon. The solvent was evaporated and the residue was purified by RP-HPLC to afford 44 mg (65%) of the product.
MS (ES) 429 (M+H)⁺

### Step B: Preparation of 2-(2-oxo-4-((8-(4-pyridyl)-2,8-diazaspiro[4.5]dec-2-yl)carbonyl)-4-piperazino)acetic acid

The ester from Step A (22 mg, 0.052 mmol) was dissolved in 2.0 ml of 2N HCl and stirred for 2 hours at room temperature. The solvent was evaporated and the residue purified by RP-HPLC to afford 9.9 mg (47%) of the title compound.
MS (ES) 402 (M+H)⁺
ELISA IC₅₀=0.035 µM PRP (ADP-Citrate) IC₅₀=0.568 µM

### Example 28

### Preparation of 2-(4-pyridyl)-2,8-diazaspiro[4.5]decane

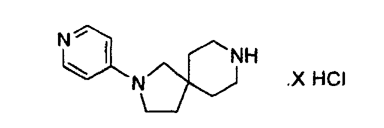

### Step A: Preparation of 8-Benzyl-2-(4-pyridyl)-2,8-diazaspiro[4.5]decane-1,3-dione

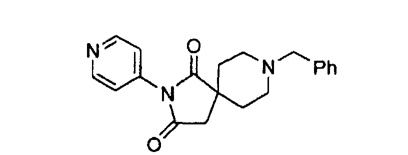

1-Benzyl-4-(carboxymethyl)-4-piperidinecarboxylic acid hydrochloride (WO 97/11940) (23.0 mMol) in DMF (60.0 mL) was treated with DCC (25.0 mMol), and the soon-formed colorless suspension was stirred at room temperature for 2.0 h.

This anhydride, 8-benzyl-2-oxa-8-azaspiro[4.5]decane-1,3-dione, was then treated with 4-amino pyridine (30.0 mMol), and DIEA (70.0 mMol). The suspension was first stirred at room temperature for 15 h, and then heated to 100 °C for 6 h. Dicyclohexyl urea was filtered off and the clear filtrate was concentrated to yield a brown glue (half-cyclized amide acid).

This was then mixed with NaOAc (230.0 mMol) and acetic anhydride (100.0 mL), and heated to reflux for 2.0 h. The mixture was filtered and the filtrate was concentrated to yield the crude imide. Trituration with ether yielded the title compound as a colorless solid (63% overall).

### Step B: Preparation of 8-Benzyl-2-(4-pyridyl)-2,8-diazaspiro[4.5]decane

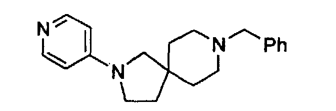

The imide (12.0 mMol) from step A was treated with BH₃.THF (1.0 M in THF) (300.0 mMol) and the mixture was refluxed for 30 h. It was cooled to 0 °C and quenched with ice (EXOTHERMIC!), and then treated with 1N NaOH to a pH of ca. 10-11. The mixture was then extracted with EtOAc. Concentration of the EtOAc extract yielded the title compound.
MS: (M+H)⁺ = 308.

### Step C: Preparation of 2-(4-pyridyl)-2,8-diazaspiro[4.5]decane hydrochloride

The crude product from Step B (25.0 mMol) was suspended in EtOH (50.0 mL) and diluted with 1N HCl (50.0 mL). 10% Pd/C (1.50 g) was added and the mixture was shaken on a Parr hydrogenator at 50 psi for 24 h. It was then filtered and the clear, colorless filtrate was concentrated to yield a colorless hygroscopic solid (61%).
MS: (M+H)⁺ = 218.

### Example 29

### Preparation of 2-(2-oxo-4-((2-(4-pyridyl)-2,8-diazaspiro[4.5]dec-8-yl)carbonyl) piperazino)acetic acid:

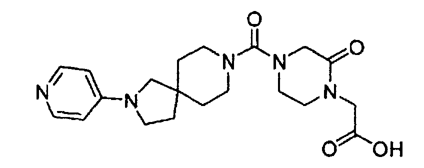

### Step A: Preparation of 4-nitrophenyl 4-(2-ethoxy-2-oxoethyl)-3-oxo-1-piperazinecarboxylate

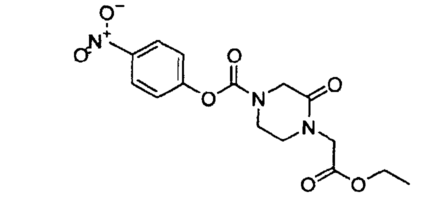

Ethyl 2-(2-oxopiperazinyl)acetate (500 mg, 2.7 mmol) was dissolved in anhydrous CH₂Cl₂ (10mL and cooled to 0°C. To this solution was added DIEA (1.0 ml, 5.7 mmol) and 4-nitrophenyl chloroformate (600 mg, 3.0 mmol). The reaction mixture was stirred overnight and allowed to warm to room temperature. The reaction was poured into a brine solution and extracted 3x with EtOAc. The organic layers were combined and the solvent evaporated. The resulting residue was purified by flash chromatography yielding 500 mg (55.0%) of the desired product.
MS (ES) 338 (M+H)⁺

### Step B: Preparation of ethyl 2-(2-oxo-4-{[2-(4-pyridyl)-2,8-diazaspiro[4.5]dec-8-yl)]carbonyl}piperazino)acetate

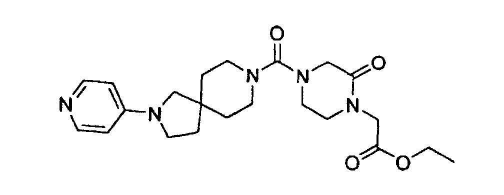

The product from Step C Example 28 (21 mg, 0.0.90 mmol) was dissolved in anhydrous DMF (2.0 ml). To this solution was added DIEA (0.182 ml, 0.105 mmol) and ethyl 2-{4-[(4-nitrophenyl)oxycarbonyl]-2-oxopiperazinyl} acetate (61 mg, 0.174 mmol). The reaction mixture was stirred overnight at 100°C under argon. The solvent was evaporated and the residue was purified by RP-HPLC to afford 44.8 mg (70%) of the product.
MS (ES) 430 (M+H)⁺

### Step C: Preparation of 2-(2-oxo-4-((2-(4-pyridyl)-2,8-diazaspiro[4.5]dec-8-yl)carbonyl)piperazino)acetic acid:

The ester from Step B (44.8 mg, 0.104 mmol) was dissolved in 2.0 ml of 2N HCl and stirred for 2 hours at room temperature. The solvent was evaporated and the residue purified by RP-HPLC to afford 18.8 mg (45%) of the title compound.
MS (ES) 402 (M+H)⁺
ELISA IC₅₀=0.122 µM PRP (ADP-Citrate) IC₅₀=2.65 µM

### Example 30

### Preparation of 3-(2-oxo-4-((2-(4-pyridyl)-2,8-diazaspiro[4.5]dec-8-yl)carbonyl) piperazino)propanoic acid:

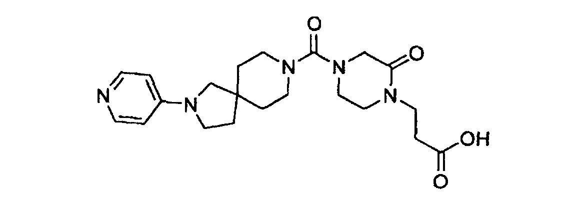

### Step A: Preparation of benzyl 4-(3-ethoxy-3-oxopropyl)-3-oxo-l-piperazine carboxylate

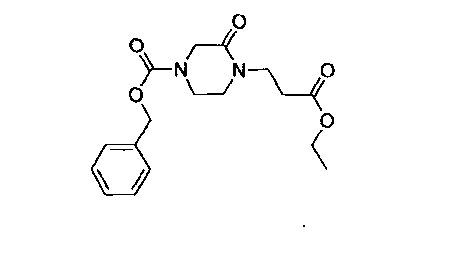

Phenylmethyl 3-oxopiperazinecarboxylate (234 mg, 1.0 mmol) was dissolved in anhydrous DMF (5mL) and cooled to 0°C. To this solution was added NaH (44 mg, 1.1 mmol). The reaction mixture was stirred for 1 hour and allowed to warm to room temperature. The reaction was cooled to 0°C and bromo ethyl propionate (0.144 ml, 1.1 mmol) was added. The reaction was stirred overnight and allowed to warm to room temperature. The reaction was poured into brine and extracted 3x with EtOAc. The organic layers were combined and the solvent evaporated. The resulting residue was purified by flash chromatography yielding 250 mg (75.0%) of the desired product.
MS (ES) 335 (M+H)⁺

### Step B: Preparation of ethyl 3-(2-oxopiperazino)propanoate

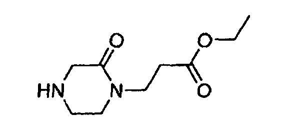

The product from Step A was dissolved in ethanol (20 ml). To this solution was added Pd(OH)₂/C (25 mg). The reaction mixture was then hydrogenated overnight at 50 psi, filtered through celite and the solvent evaporated to afford 150 mg (100%) of the product.
MS (ES) 201 (M+H)⁺

### Step C: Preparation of 4-nitrophenyl 4-(3-ethoxy-3-oxopropyl)-3-oxo-]-piperazinecarboxylate

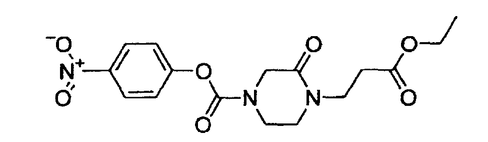

The product from Step B was dissolved in anhydrous CH₂Cl₂ (10 ml) and cooled to 0°C. To this solution was added DIEA (0.323 ml, 1.9 mmol) and 4-nitrophenyl chloroformate (200 mg, 0.974 mmol). The reaction mixture was stirred overnight and allowed to warm to room temperature. The reaction was poured into brine and extracted 3x with EtOAc. The organic layers were combined and the solvent evaporated. The resulting residue was purified by flash chromatography yielding 218 mg (60%) of the desired product.
MS (ES) 366 (M+H)⁺

### Step D: Preparation of ethyl 3-(2-oxo4-{ [2-(4-pyridyl)-2,8-diazaspiro[4.5]dec-8-yl]carbonyl } piperazino)propanoate

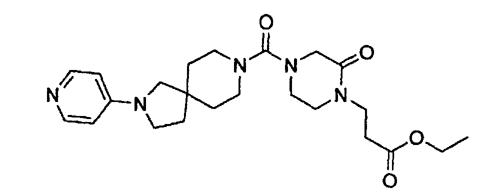

The product from Step C Example 28 (21 mg, 0.090 mmol) was dissolved in anhydrous DMF (2.0 ml). To this solution was added DIEA (0.182 ml, 0.105 mmol) and the product from Step C (61 mg, 0.174 mmol). The reaction mixture was stirred overnight at 100°C under argon. The solvent was evaporated and the residue was purified by RP-HPLC to afford 27 mg (67%) of the product.
MS (ES) 444 (M+H)⁺

### Step E: Preparation of 3-(2-oxo-4-((2-(4-pyridyl)-2,8-diazaspiro[4.5]dec-8-yl)carbonyl)piperazino)propanoic acid

The ester from Step D (44.8 mg, 0.104 mmol) was dissolved in 2.0 ml of 2N HCI and stirred for 2 hours at room temperature. The solvent was evaporated and the residue purified RP-HPLC to afford 18.8 mg (45%) of the title compound.
MS (ES) 416 (M+H)⁺
PRP (ADP-Citrate) IC₅₀ 1.29 µM

### Example 31

### Preparation of 3-oxo-3-(4-{[-(4-pyridyl)-2,8-diazaspiro[4.5]dec-8-yl]carbonyl} piperazino) propanoic acid

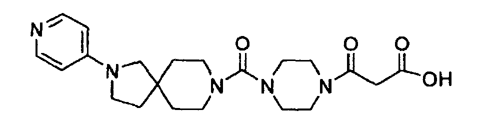

### Step A:

To the DMF (2mL) solution of N'-CBz-piperazine (660mg, 3mmol) added DIEA (2.1mL, 12mmol) this was cooled to 0°C and then to this slowly added ethyl malonyl chloride (677mg, 4.5mmol). The reaction mixture was stirred at room temperature overnight, then solvent was evaporated. The residue suspended in water and extracted with EtOAc. The organic layer was washed with sat'd brine, dried, filtered and evaporated to give golden yellow oil. The residue was purified on silica gel chromatography (20%EtOAc/Hexane) to afford 750mg (74%) of the desired product.

### Step B:

To the EtOH solution (10mL of N'-CBz-N⁴-acylated piperazine (408mg) from Step A of this example, added 10%Pd/C (100mg) and this was stirred under latm of H₂ for 3h. The solution was filtered through celite and evaporated to give the desired product (228mg, 95%) as a colorless oil.

### Step C: Preparation of 4-nitrophenyl-4-(3-oxo-3-propoxypropanoyl)-1-piperazine carboxylate

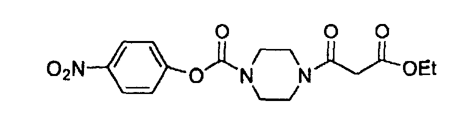

To the CH₂Cl₂ solution (5mL) of piperazine (348mg, 1.7mmol) from above Step B, added DIEA (740uL, 4.25mmol) and the reaction was cooled to 0°C. To this cold solution added p-nitrophenylchloroformate (342uL, 1.7mmol) and then reaction slowly warmed up to room temperature. After stirring for 4h, the reaction mixture was diluted with CH₂Cl₂ washed with sat'd NaHCO₃, dried, filtered and evaporated to afford the crude residue. The residue was purified on silica gel chromatography (5%MeOH/CH₂Cl₂) to afford the desired product as a yellow oil (270mg).

### Step D: Preparation of Ethyl 3-oxo-3-(4-{[-(4-pyridyl)-2,8-diazaspiro[4.5]dec-8-yl]carbonyl}piperazino) propanoate

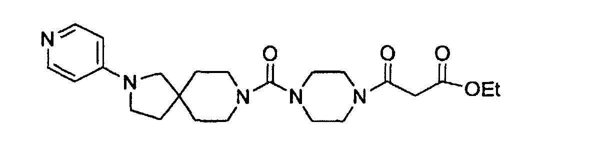

To the cold (0°C) DMF (1mL) solution of p-nitrophenyl carbamate (119mg, 0.32mmol) from Step C added DIEA (325uL, 1.85mmol) followed by DMF (1mL) solution of 2-(4-pyridyl)2,8-diazaspiro[4.5]undecane as an HCl salt ( 100mg, 0.31mmol, from Step C, Example 28). The clear solution was heated to 75°C for 18h, then cooled and the solvent was evaporated. The residue was suspended in CH₂Cl₂, washed with 10% NaHCO₃, dried, filtered and evaporated give the crude residue. The residue was purified by RP-HPLC to give the desired product as white solid (90mg, 70%) MS (DCI) 443 (M+H)⁺. ¹H NMR (400MHz, CD₃OD) δ 8.10 (d, 2H), 6.60 (d, 2H), 4.18 (q, 2H), 3.6-3.54 (m, 4H), 3.46 (s, 2H), 3.35-3.25 (m, 12H), 2.06 (t, 2H), 1.64 (m, 4H), 1.27 (t, 3H)

### Step E: Preparation of 3-oxo-3-(4-{[-(4-pyridyl)-2,8-diazaspiro[4.5]dec-8-yl]carbonyl}piperazino) propanoic acid

To the THF (1mL) solution of ester (40mg, from Step D) added 1N LiOH (100uL) and the reaction mixture stirred at room temperature for 2h. After that the solvent was evaporated and the residue purified by RP-HPLC to afford the desired acid (20mg) as a hygroscopic solid. MS (DCI) 415 (M+H)⁺. ¹H NMR (400MHz, CD₃OD) δ 8.06 (d, 2H), 6.82 (d, 1H), 6.85 (d, 1H), 3.6-3.54 (m, 16H), 3.37 (s, 2H), 2.04 (t, 2H), 1.64 (m, 4H)
PRP (ADP-Citrate) IC₅₀ = 0.660 µM ELISA: IC₅₀= 0.136 µM
PRP (ADP-PPACK) IC₅₀ = 3.6 µM

### Example 32

### Preparation of 2- {4-(2-(4-pyridyl)-2,8-diazaspiro[4.5]dec-8-yl]piperidino}acetic acid

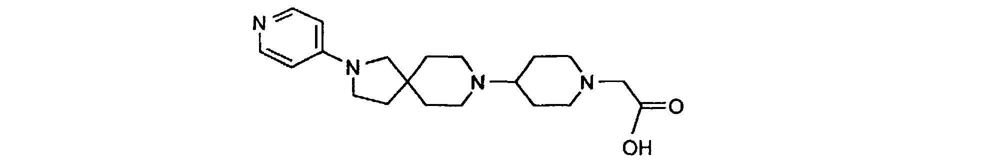

### Step A: Preparation of ethyl-4-piperidinoacetate

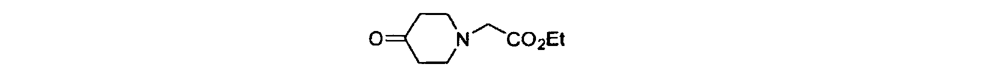

To the CH₃CN solution (10mL) of 4-piperidone.hydrate (307mg, 2mmol), added K₂CO₃ (608mg, 4.4mmol), followed by ethyl iodoacetate (300uL, 2.52mmol) and the reaction mixture was refluxed for 24h. After that filtered the salts, added EtOAc to the filtrate and washed with sat'd brine. The organic layer was dried, filtered and evaporated to give the desired product as a white solid (290mg, 78%).

### Step B: Preparation of ethyl 2-{4-(2-(4-pyridyl)-2,8-diazaspiro[4.5]dec-8-yl]piperidino}acetate

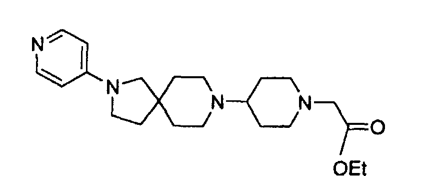

To the CH₃OH solution (1mL) of ketone (57mg, 0.305mmol, from Step A) added KOH(12mg, 0.2mmol), followed by CH₃OH (1mL) solution of 2-(4-pyridyl)2,8-diazaspiro[4.5]undecane (130mg, 0.4mmol, Step C, Example 28) and sodium cyanoborohydride (50mg, 0.8mmol). The reaction mixture was stirred overnight at room temperature, to that then added 10%HCl and evaporated the solvent. The residue was purified by RP-HPLC to afford the desired product as a white solid (60mg, 51%). MS (DCI) 387 (M+H)⁺. ¹H NMR (400MHz, CD₃OD) δ 8.10 (d, 2H), 6.84 (d, 2H), 4.31 (q, 2H), 4.12 (s, 2H), 3.81-3.58 (m, 8H), 3.28-3.02 (m, 8H), 2.49-1.99 (m, 8H), 1.29 (t, 3H)

### Step C: Preparation of 2-{4-(2-(4-pyridyl)-2,8-diazaspiro[4.5]dec-8-yl]piperidino}acetic acid

To the ester from Step B (45mg) was added 2N HCl (1mL) and the reaction was heated at 60°C for 4h. After that evaporated the solvent and the residue was purified by RP-HPLC to afford 28mg of the desired acid.
MS (ES) 360 (M+H)⁺
PRP (ADP-Citrate) IC₅₀ = 9.5 µM

### Example 33

### Preparation of 2-(4-{2-oxo-2-[2-(4-pyridyl)-2,8-diazaspiro[4.5]dec-8-yl)ethyl} piperidino)acetic acid

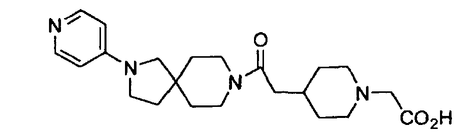

### Step A: Preparation of ethyl 2-(4-{2-oxo-2-[2-(4-pyridyl)-2,8-diazaspiro[4.5]dec-8-yl]ethyl }piperidino)acetate

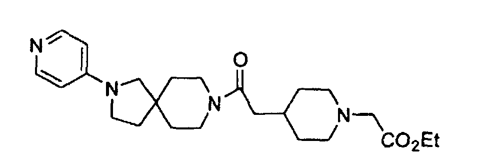

To the DMF (2mL) solution of Fmoc-piperidine-4-acetic acid (93mg, 0.255mmol) added HATU (123mg, 0.325mmol), followed by DMF (2mL) solution of (4-pyridyl)2,8-diazaspiro[4.5]undecane (81mg, 0.25mmol). The reaction mixture was stirred at room temperature overnight, after that solvent was evaporated and the residue was suspended in EtOAc. The EtOAc layer was washed with 10%HCl, then the aqueous layer was basified with 10% NaOH and re-extracted with ethyl acetate. The EtOAc was dried, filtered, evaporated to give crude residue. The residue was purified by silica gel chromatography (10%MeOH/CH₂Cl₂) to give the desired product. MS (ES) 564 (M+H)⁺.

The Fmoc material was stirred in 20% piperidine in DMF for 1h, after that solvent was evaporated. The residue was purified by RP-HPLC to give the desired de-Fmoc piperidine product. MS(ES) 343 (M+H)⁺

To the DMF (1mL) solution of deprotected-piperidine (25mg, 0.07mmol) added DIEA (49uL, 0.28mmol) followed by ethyl chloroacetate (11mg, 0.084mmol). The reaction was heated to 65°C for 4h, after that the solvent was evaporated and the residue purified on RP-HPLC. The purification afforded the desired product ethyl 2-(4-{2-oxo-2-[2-(4-pyridyl)-2,8-diazaspiro[4.5]dec-8-yl]ethyl}piperidino)acetate as a white solid (20mg).
MS(ES) 429(M+H)⁺

### Step B: Preparation of 2-(4-{2-oxo-2-[2-(4-pyridyl)-2,8-diazaspiro[4.5]dec-8-yl]ethyl } piperidino)acetic

To the ester (10mg) added 2N HCl (500uL) and the reaction was heated at 60°C for 4h. After that evaporated the solvent and the residue was purified by RP-HPLC to afford 5mg of the desired acid. MS (ES) 401 (M+H)⁺
PRP (ADP-Citrate) IC₅₀ = 80 µM

### Example 34

### Preparation of (2S)-2-{[(3,5-dimethyl-4-isoxazolyl)sulfonyl]amino}-5-oxo-5-[2-(4-pyridyl)-2,8-diazaspiro[4.5]dec-8-yl]pentanoic acid

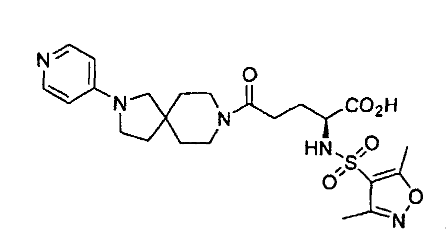

### Step A: Preparation of ethyl (2S)-2-{[(benzyloxy)carbonyl]amino }-5-oxo-5 [2-(4-pyridyl)-2,8-diazaspiro[4.5]dec-8-yl]pentanoate

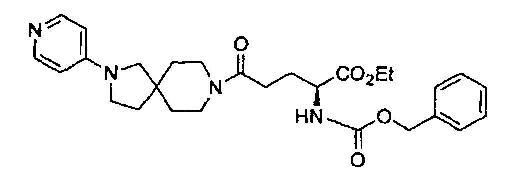

To the DMF (3mL) solution of (4S)-4-{[(benzyloxy)carbonyl]amino}-5-ethoxy-5-oxopentanoic acid (prepared according to Liebigs Ann. Chem. **1961**, 646, 127) (170mg, 0.55mmol) added HATU (380mg, 1mmol) followed by DMF solution (1mL) of (4-pyridyl)2,8-diazaspiro[4.5]undecane from Step C, Example 28, (163mg, 0.5mmol) and DIEA (350uL, 2mmol). The reaction mixture was stirred at room temperature overnight, then poured into water and extracted with EtOAc. The EtOAc layer was washed with 10%HCl, 10% NaHCO₃, brine, dried, filtered and evaporated to give brown viscous oil. The residue was purified by RP-HPLC to afford the desired product as a yellow solid (150mg, 59%). MS (ES) 509 (M+H)⁺

### Step B: Preparation of ethyl (2S)-2-amino-5-oxo-5-[2-(4-pyridyl)-2,8-diazaspiro[4.5]dec-8-yl]pentanoate

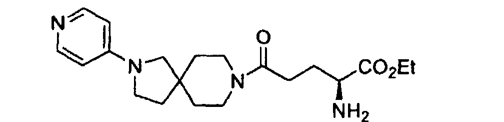

To the EtOH (5mL) solution of Cbz-protected ester from Step A (135mg) was added 10%Pd/C (60mg) and reaction was stirred for 6h at room temperature under an atmosphere of hydrogen. The catalyst was removed by filtration through celite and the filtrate was concentrated under reduced pressure to afford the desired product as a colorless viscous oil (105mg)

### Step C: Preparation of ethyl (2S)-2-{[(3,5-dimethyl-4-isoxazolyl)sulfonyl]amino}-5-oxo-5-[2-(4-pyridyl)-2,8-diazaspiro[4.5]dec-8-yl]pentanoate

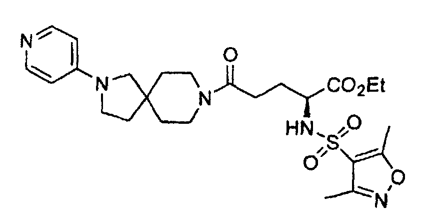

To the CH₂Cl₂ solution (2mL) of ethyl (2S)-2-amino-5-oxo-5-[2-(4-pyridyl)-2,8-diazaspiro[4.5]dec-8-yl]pentanoate (105mg, 0.28mmol) added 3,5-dimethylisoxazole-4-sulfonyl chloride (61mg, 0.308mmol) followed by DIEA (100uL, 0.56mmol). The reaction mixture was stirred at room temperature overnight. After that extracted with EtOAc and washed with water, brine, dried, filtered and evaporated to give the crude residue. This residue was purified by RP-HPLC to afford the desired product as a white solid (60mg, 40%). MS (ES) 534 (M+H)⁺

### Step D: Preparation of (2S)-2-{[(3,5-dimethyl-4-isoxazolyl)sulfonyl]amino}-5-oxo-5-[2-(4-pyridyl)-2,8-diazaspiro[4.5]dec-8-yl]pentanoic acid

To the ester (20mg,0.036mmol) from Step C added EtOH(lmL), 1M LiOH (250uL) and the reaction mixture was stirred at room temperature overnight. After that evaporated the solvent and residue purified by RP-HPLC to give the desired product (10mg). MS (ES) 506 (M+H)⁺
PRP (ADP-Citrate) IC₅₀ = 0.271 µM ELISA: IC₅₀= 0.002 µM

### Example 35

### Preparation of (2S)-2-[(butoxycarbonyl)amino]-5-oxo-5-[2-(4-pyridyl)-2,8-diazaspiro[4.5]dec-8-yl]pentanoic acid

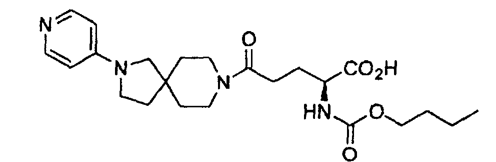

### Step A: Preparation of ethyl (2S)-2-[(butoxycarbonyl)amino)-5-oxo-5-[2-(4-pyridyl)-2,8-diazaspiro[4.5]dec-8-yl]pentanoate

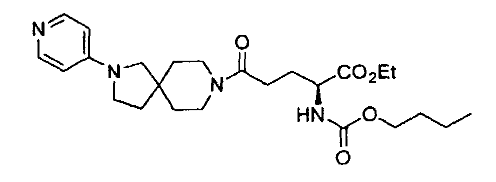

To the DMF (3mL) solution of (4S)-4-{[(butoxy)carbonyl]ammo}-5-ethoxy-5-oxopentanoic acid (prepared according to Step D, Example 36) (96mg,0.352mmol) added HATU (243mg, 0.64mmol) followed by DMF solution (1mL) of (4-pyridyl)2,8-diazaspiro[4.5]undecane from Step C, Example 28, (105mg, 0.32mmol) and DIEA (225uL, 1.28mmol). The reaction mixture was stirred at room temperature overnight, then poured into water and extracted with EtOAc. The EtOAc layer was washed with 10%HCl, 10% NaHCO₃, brine, dried, filtered and evaporated to give brown viscous oil. The residue was purified by RP-HPLC to afford the desired product as a yellow solid (100mg, 63%). MS (ES) 475 (M+H)⁺. ¹H NMR (400MHz, CDCl₃) δ 8.1 (d, 2H), 6.60 (d, 2H), 5.4 (bs, 1H), 4.28 (m,1H), 4.13 (q,2H), 4.00 (m, 2H), 3.56-3.35 (m, 8H), 2.4 (m, 1H), 2.20-2.01 (m, 4H), 1.62-1.51 (m, 6H), 1.35-1.29 (m, 2H), 1.24 (t, 3H), 0.87 (t, 3H)

### Step B: Preparation of (2S)-2-[(butoxycarbonyl)amino]-5-oxo-5-[2-(4-pyridyl)-2,8-diazaspiro[4.5]dec-8-yl]pentanoic acid

To THF (1mL) solution of ester (25mg, 0.05mmol) added 1N LiOH (200uL) and the reaction mixture stirred at room temperature overnight. The solvent was evaporated and the residue purified by RP-HPLC to afford the desired product (18mg, 81%).
MS (ES) 447 (M+H)⁺
PRP (ADP-Citrate) IC₅₀ = 4 µM

### Example 36

### Preparation of (4S)-4-{(butoxycarbonyl)amino}-5-ethoxy-5-oxopentanoic acid

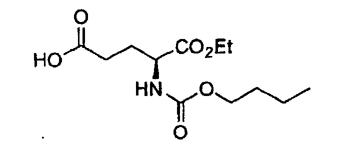

### Step A: Preparation of 5-(tert-butyl)-1-ethyl-(2S)-2-{[(benzyloxy) carbonyl] amino }pentanedioate

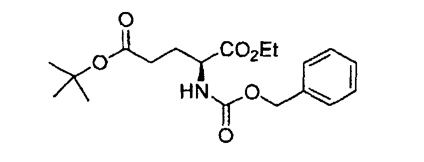

To the DMF (115mL) solution of Z-Glu-(O-tBu) (10g, 29.6mmol) added EtOH (17mL), EDC.HCl (13.06g, 68mmol), HOBt (9.06g, 59mmol) and the reaction mixture was stirred at room temperature overnight. After evaporation of the DMF under reduced pressure, the residue was dissolved in EtOAc (15 mls) and washed with 10% HCI (10 mls), brine (10 mls), and dried with MgSO₄. The solvent was removed *in vacuo,* and the residue was purified using flash chromatography (3% MeOH/CH₂Cl₂) to afford the desired product. MS (ES) 388 (M+Na), 266 (M+H-Boc). ¹H NMR (400MHz, CDCl₃) δ 5.40 (d, 111), 5.10 (s, 2H), 4.35 (m, 1H), 4.22 (q, 2H), 2.15 (m, 1H), 1.95 (m, 111), 1.40 (s, 9H), 1.25 (t, 3H).

### Step B: Preparation of 5-(tert-butyl)-1-ethyl-(2S)-2-aminopentanedioate

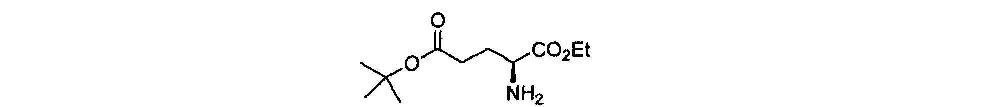

To the CH₃OH (100mL) solution of the Cbz-protected material (9.96g) from Step A added Pd(OH)₂/C (500mg) and then hydrogenated at 50psi on parr hydrogenator for 4h. After that filtered through celite and concentrated to afford the desired product as a viscous oil (5.8g, 92.5%). MS (DCI) 232 (M+H⁺). ¹H NMR (400MHz, CDCl₃) δ 4.22 (q, 2H), 3.40 (dd, 12H), 2.30 (t, 2H), 2.00 (m, 1H), 1.6 (t, 2H), 1.75 (t, 3H), 1.41 (s, 9H), 1.20 (t, 3H).

### Step C: Preparation of 5-(tert-butyl)-1-ethyl-(2S)-2-[(butoxycarbonyl)amino}pentanedioate

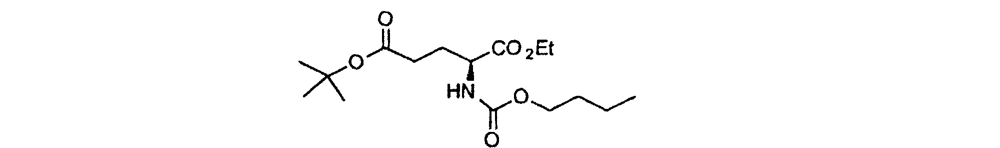

To the CH₂Cl₂; solution (70mL) of the aminoester (1.63g, 7.05mmol) from Step B added DIEA (2.5mL, 14. lmmol) and this was cooled to 0°C. To this cold solution slowly added n-butyl chloroformate (1.17mL, 9.2mmol) and the reaction was slowly warmed to room temperature and stirred for 4h. After stirring the solvent was removed *in vacuo,* and the residue purified by silica gel chromatography (1%MeOH/CH₂Cl₂) to afford the desired product (1.65g, 70.5%). MS (DCI) 332 (M+H⁺). ¹H NMR (400MHz, CDCl₃) δ 5.50 (d, 1H), 4.40 (m, 1H), 4.22 (q, 2H), 4.05 (t, 2H), 2.35 (m, 2H), 2.20 (m, 1H), 1.99 (m, 1H), 1.6 (t, 2H), 1.42 (s, 9H), 1.46 (q, 2H), 1.30 (t, 3H), 0.91 (t, 3H)

### Step D: Preparation of (4S)-4-{(butoxycarbonyl)amino}-5-ethoxy-5-oxopentanoic acid

To the t-butyl ester (2.61g) from Step C added 15ml TFA and the reaction mixture was stirred for 2h at 0°C. After that the solvent was evaporated and the residue triturated with ether to afford the desired product as a white solid. MS (DCI) 276 (M+H⁺). ¹H NMR (400MHz, CDCl₃) δ 11.2 (s, 1H), 5.50 (bs, 1H), 4.40 (m, 1H), 4.22 (q, 2H), 4.05 (t, 2H), 2.50 q, 2H), 2.20 (m, 1H), 1.99 (m, 1H), 1.6 (t, 2H), 1.46 (q, 2H), 1.30 (t, 3H), 0.91 (t, 3H)

### Example 37

### Preparation of (4S)-4-{[(3,5-dimethyl-4-isoxazolyl)sulfonyl]amino}-5-ethoxy-5-oxopentanoic acid

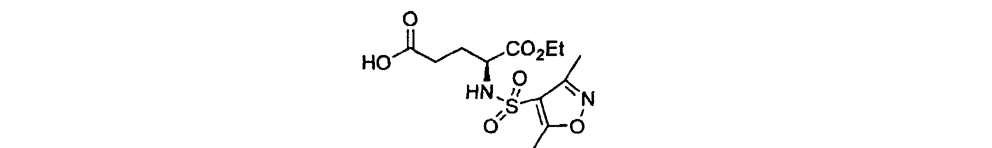

### Step A: Preparation of 5-(tert-butyl)-1-ethyl-(2S)-2-{[(3,5-dimethyl-4-isoxazolyl)sulfonyl]amino }pentanedioate

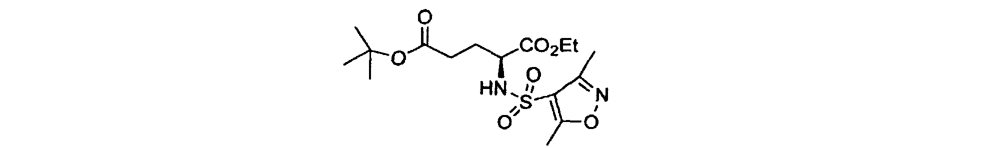

To the CH₂Cl₂ solution (70mL) of the aminoester (3g, 12.98mmol) from Step B Example 36, added DIEA (4.5mL, 25.96mmol) and this was cooled to 0°C. To this cold solution slowly added 3,5-dimethylisoxazole-4-sulfonyl chloride (3.38g, 17.3mmol) and the reaction was slowly warmed to room temperature and stirred overnight. After evaporation of the CH₂Cl₂ under reduced pressure, the residue was dissolved in EtOAc (15 mls) and washed with 10% HCl (10 mls), brine (10 mls), and dried with MgSO₄. The solvent was removed *in vacuo,* and the residue was purified using flash chromatography (0.5/9.5/90, MeOH/EtOAc/Hexane) to afford the desired material (3.6g, 72%) as a colorless oil. MS (ES) 413 (M+Na). ¹H NMR (400MHz, CDCl₃) δ 5.40 (d, 1H), 4.05 (q, 2H), 3.90 (m, 1H), 2.60 (s, 3H), 2.48-2.30 (m,2H), 2.40 (s, 3H),2.05 (m, 1H), 1.80 (m, 1H), 1.43 (s,9H), 1.20 (t, 3H).

### Step B: Preparation of (4S)-4-{[(3,5-dimethyl-4-isoxazolyl)sulfonyl]amino}-5-ethoxy-5-oxopentanoic acid

To the t-butyl ester (2.71g) from Step A added 40% TFA in CH₂Cl₂ (10mL) and the reaction mixture was stirred for 2h at 0°C. After that the solvent was evaporated and the residue triturated with heptane to afford the desired product as a white solid (2.83g, quantitative).

MS (ES) 335 (M+H)⁺. ¹H NMR (400MHz, CDCl₃) δ 10.2 (s, 111), 5.6 (d, 1H), 4.10 (q, 2H), 3.80 (m, 1H), 2.6 (s, 3H), 2.55 (t, 2H), 2.40 (s, 3H), 2.15 (m, 1H), 1.80 (m, 1H), 1.30 (t, 3H).

### Example 38

### Preparation of (4S)-4-[(butylsulfonyl)amino]-5-ethoxy-5-oxopentanoic acid

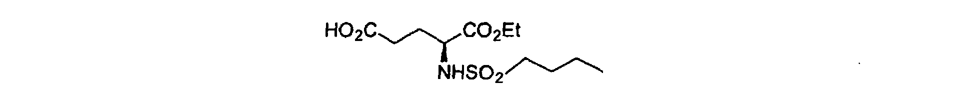

### Step A: Preparation of 5-(tert-butyl) 1-ethyl (2S)-2-[(butylsulfonyl)amino] pentandioate

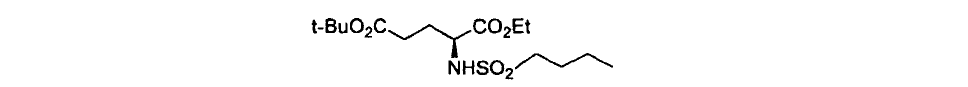

To the material obtained in Step B from Example 36 (230 mgs) in CH₂Cl₂ (10 mls) at 0°C under argon was added pyridine (0.18 mls). Then n-BuSO₂Cl (0.26 mls) was added dropwise via syringe and the resulting solution was stirred overnight as it warmed to room temperature. After evaporation of the CH₂Cl₂ under reduced pressure, the residue was dissolved in EtOAc (15 mls) and washed with 0.3M HCl (10 mls), brine (10 mls), and dried with MgSO₄. The solvent was removed *in vacuo,* and the residue was purified using flash chromatography (Hexane/EtOAc, 4:1) to afford the desired material (199 mgs, 57%).
MS(PES): (2M+Na)⁺=725, (M+Na)⁺=374, (M+H-C₄H₈)⁺=296

### Step B: Preparation of the title compound

To the material from above Step A (190 mgs) was added 40%TFA in CH₂Cl₂ (2 mls) at room temperature and stirring was maintained for 2 hrs. The solvent was removed under reduced pressure thus affording the desired acid as an oil (162 mgs, 100%)
MS(PES): (M+H)⁺=296, (M+Na)⁺=3 8.

### Example 39

### Preparation of (4S)-5-ethoxy-4-{[(4-methylphenyl)sulfonyl]amino}-5-oxopentanoic acid

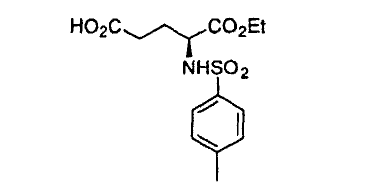

This material was prepared by substantially following the procedure in Example 38 except that p-toluenesulfonyl chloride was used in place of n-butylsulfonyl chloride in Step A.
MS(PES): (M+H)⁺=330, (M+Na)⁺=352.

### Example 40

### Preparation of (4S)-4-{[(benzyloxy)carbonyl]amino)-5-ethoxy-5-oxopentanoic acid

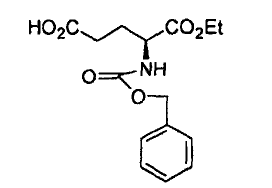

To 5-(*tert*-butyl) 1-ethyl (2S)-2-{[(benzyloxy)carbonyl]amino}pentanedioate (5.0 gms) (obtained from Example 36, Step A) was added 40%TFA in CH₂Cl₂ (15 mls) at room temperature under argon and stirring was continued overnight. The solvent was removed under reduced pressure thus affording the desired acid (4.2 gms, 100%)
MS(PES): (M+H)⁺=310.

### Example 41

### Preparation of 2-(4- { (2-([4-pyridyl)-2,8-diazaspiro[4.5]dec-8-yl)carbonyl}piperazino)-acetic acid

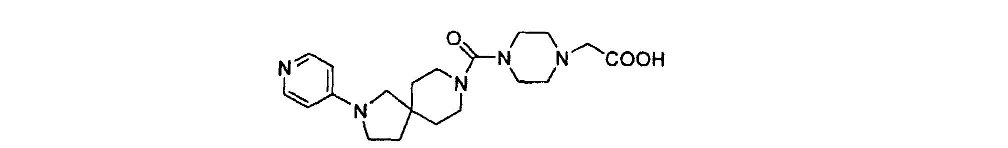

### Step A: Preparation of 4-nitrophenyl 4-(2-ethoxy-2-oxoethyl)-1-piperazine carboxylate

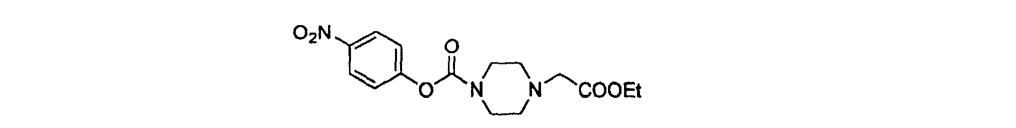

1-(Ethoxy carbonylmethyl)-piperazine (11.61 mmol) in CH₂Cl₂ (10 mL) was added dropwise to a mixture of 4-nitrophenyl chloroformate (15.10 mMol) and DIEA (45 mMol) in the same solvent (15.0 mL). The pale yellow solution was warmed to room temperature and then stirred there overnight. Concentration of the reaction mixture yielded the crude product, which was purified by flash chromatography (82%).

### Step B: Preparation of Ethyl 2-(4-{[(2-(4-pyridyl)-2,8-diazaspiro[4.5]dec-8-yl]-carbonyl } -piperazino)acetate

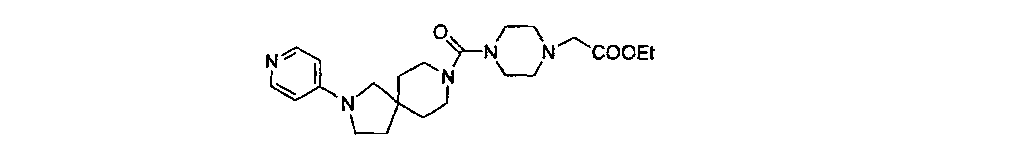

The 4-nitrophenyl carbamate from Step A (0.325 mMol) was mixed with the spirocyclic piperidine (0.32 mmol, Step C, Example 28), and DIEA (2.0 mmol), and the mixture was heated to 75 °C for ca. 20 h. The mixture was then partitioned between CH₂Cl₂ and sodium bicarbonate. Evaporation of the CH₂Cl₂ extract yielded the crude product, which was purified by RP-HPLC (52%).

### Step C: Preparation of 2-(4-{[2-(4-pyridyl)-2,8-diazaspiro[4.5]dec-8-yl]carbonyl}-piperazino)acetic acid

The ethyl ester (0.25 mMol) from Step B was dissolved in THF (10 mL) and treated with 1M LiOH (0.50 mMol), and stirred at room temperature for ca. 4h. Then it was acidified at 0 °C with 1N HCl, and concentrated. The crude product was purified by RP-HPLC (69%).
MS: (M+H)⁺ = 388.
PRP (IC₅₀) = 8.925 µM ELISA (IC₅₀) = 0.252 µM.

### Example 42

### Preparation of 3-oxo-3-(4-{[2-(4-pyridyl)-2,8-diazaspiro[4.5]dec-8-yl]carbonyl}-1,4-diazepan-1-yl)propanoic acid

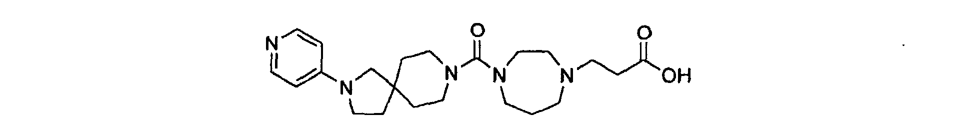

### Step A: Preparation of benzyl 4-(3-ethoxy-3-oxopropanoyl)-1,4-diazepane-1-carboxylate

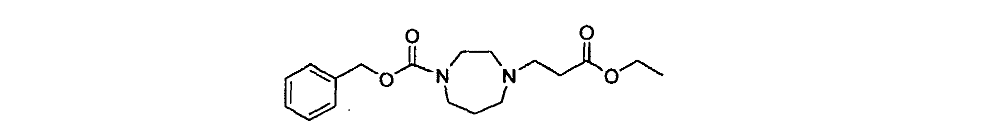

Benzyl 1,4-diazepane-1-carboxylate (769 mg, 3.28 mmol) was suspended in DMF (8 ml) and DIEA (1.14 ml, 6.56 mmol) was added, followed by ethyl 3-bromopropionate (624 mg, 3.45 mmol) and the mixture was stirred at room temperature overnight. After evaporation of the solvent under reduced pressure, the residue was extracted with CH₂Cl₂ versus saturated NaHCO₃ and then the organic layers were combined and dried over MgSO₄, filtered and evaporated. The crude was filtered through a silica plug and concentrated to yield the title compound as a light colored oil (765 mg, 70%).
MS (ES) (M+H)⁺ =335

### Step B: Preparation of ethyl 3-(1,4-diazepan-1-yl)-3-oxopropanoate

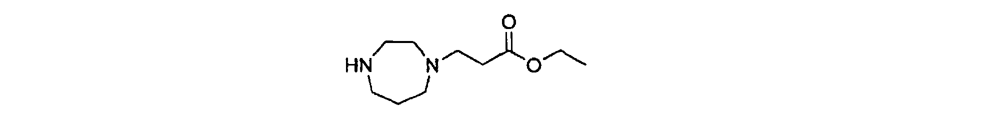

The benzyl ester from Step A (765 mg) was dissolved in EtOH (20 ml), 5% Pd/C (100 mg) was added and the mixture was placed on the Parr hydrogenator at 50 psi H₂ pressure for 1 hr. The reaction was filtered through celite and evaporated under reduced pressure to yield 450 mg (98%) of the title compound.

### Step C: Preparation of 4-nitrophenyl 4-(3-ethoxy-3-oxopropanoyl)-1,4-diazepane-1-carboxylate

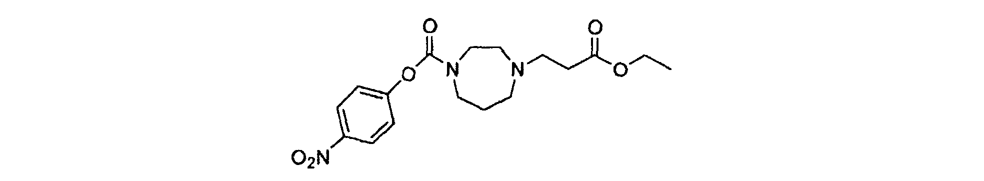

The amine from Step B (400 mg, 2.0 mmol) was dissolved in CH₂Cl₂ (20 ml) with DIEA (0.87 ml, 2.3 mmol) and cooled to 0 °C. Then p-nitrophenyl chloroformate (523 mg, 2.53 mmol) was added and the reaction was stirred 2 hr, then extracted CH₂Cl₂ versus saturated NaHCO₃, dried on MgSO₄, filtered and evaporated. Purification by column chromatography yielded the title compound as a yellowish oil (0.544 mg, 75%).
MS (ES) (M+H)⁺ =366

### Step D: Preparation of ethyl 3-oxo-3-(4-{[2-(4-pyridyl)-2,8-diazaspiro[4.5]dec-8-yl]carbonyl}-1,4-diazepan-1 1 -yl)propanoate

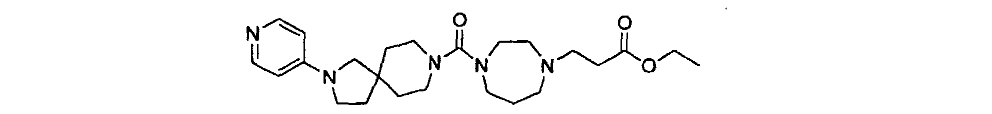

2-(4-pyridyl)-2,8-diazaspiro[4.5]decane (109 mg, 0.334 mmol) was suspended in DMF (3 ml) with DIEA (0.35 ml, 2.0 mmol), heated to 70 °C and the carbamate from Step C (184 mg, 0.50mmol) was added. Heated to 95-100 °C for 24 hr. Evaporated off DMF in vacuum, then extracted with CH₂Cl₂ versus saturated NaHCO₃ and combined the organic layers. Dried them on MgSO₄, filtered and evaporated. Purified by RP-HPLC to yield the title compound (80 mg).
MS (ES) (M+H)⁺ = 444

### Step E: Preparation of the title compound

This compound was hydrolyzed by substantially following the procedure from Example 44, Step E to afford 22.6 mg of the title compound as a white fluffy solid.
MS (ES) (M+H)⁺ = 417
PRP=3.675 µM ELISA=0.176 µM

### Example 43

### Preparation of 3-oxo-3-(4-{[2-(4-pyridyl)-2,8-diazaspiro[4.5]dec-8-yl]carbonyl}-1,4-diazepan-1-yl)propanoic acid

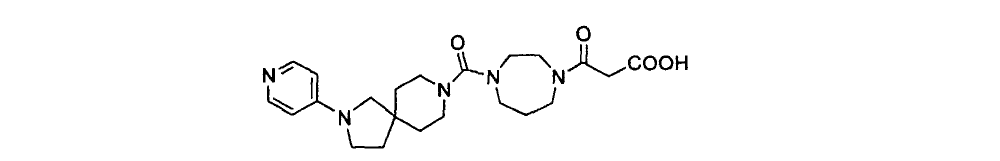

### Step A: Preparation of Benzyl 4-(3-ethoxy-3-oxopropanoyl)-1,4-diazepane-1-carboxylate

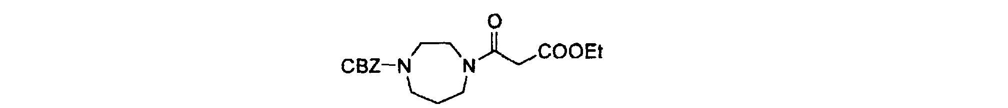

A mixture of N-Cbz-homopiperazine (4.268 mMol) and DIEA (8.60 mMol) in CH₂Cl₂ (15.0 mL) at 0 °C was treated with ethyl malonyl chloride (5.077 mMol). The solution was stirred at room temperature overnight. The reaction mixture was then partioned between CH₂Cl₂ and NaHCO₃. The title compound was obtained by evaporation of the CH₂Cl₂ extract. It was purified by flash chromatography (91%).

### Step B: Preparation of Ethyl 3-(1,4-diazepan-1-yl)-3-oxopropanoate

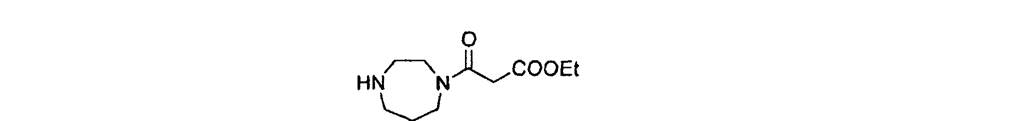

The compound from Step A (3.60 mMol) was dissolved in ethanol (25 mL), Pd(OH)₂ (5% by weight) was added, and the mixture was shaken on a Parr hydrogenator at 30 psi of hydrogen for ca. 4 h. The title compound was obtained by filtration, and concentration (93%).

### Step C: Preparation of 4-nitrophenyl 4-(3-ethoxy-3-oxopropanoyl)-1,4-diazepane-1-carboxylate

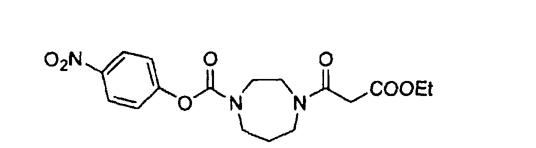

A solution of the acylated homo-piperazine analog from Step B (2.61 mMol) and DIEA (6.30 mMol) in CH₂Cl₂ (20 mL) was treated with 4-nitrophenyl chloroformate (3.175 mMol). The mixture was then stirred at room temperature overnight. The reaction mixture was concentrated and chromatographed to yield the title compound (76%).

### Step D: Preparation of Ethyl 3-oxo-3-(4-{[2-(4-pyridyl)-2,8-diazaspiro[4.5]dec-8-yl]carbonyl) - 1 ,4-diazepan- 1 -yl)propanoate

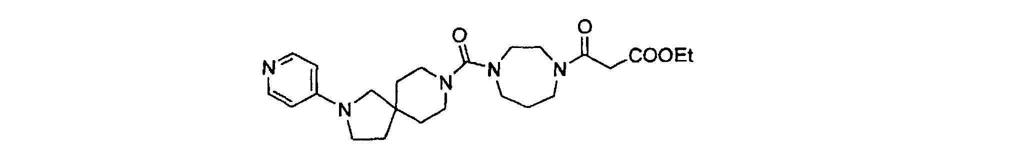

The 4-nitrophenyl carbamate from Step C (0.395 mMol), tricyclic piperidine from Step C, Example 28, (0.325 mMol), and DIEA (1.72 mMol) in DMF (5.0 mL) was heated to 75 °C for 20 h. The mixture was then partitioned between CH₂Cl₂ and NaHCO₃. The title compound was obtained by evaporation of the CH₂Cl₂ extract. It was purified by flash chromatography (52%). MS: ES (M+H)⁺ = 458.

### Step E: Preparation of 3-oxo-3-(4-{[2-(4-pyridyl)-2,8-diazaspiro[4.5]dec-8-yl]carbonyl}-1,4- diazepan-1-yl)propanoic acid

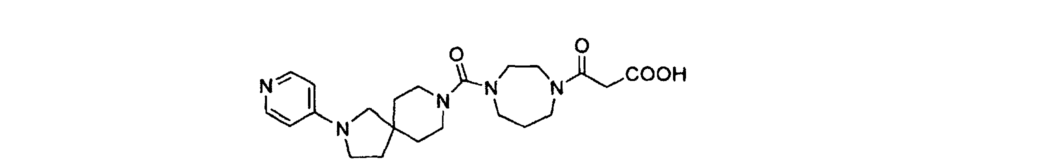

The ethyl ester from Step D (0.20 mMol) was dissolved in THF (7.0 mL) and treated with 1M LiOH (0.45 mL), and stirred at room temperature for ca. 5h. Then it was acidified at 0 °C with 1N HCl, and concentrated. The title compound was obtained by RP-HPLC purification (71%).
MS: ES (M+H-CO₂)⁺ = 386.
PRP (IC₅₀) = 0.697 µM ELISA (IC₅₀) = 0.025 µM.

### Example 44

### Preparation of 2-[(1-{2-(4-pyridyl)-2,8-diazaspiro[4.5]dec-8-yl]carbonyl}-4-piperidyl) oxy]acetic acid

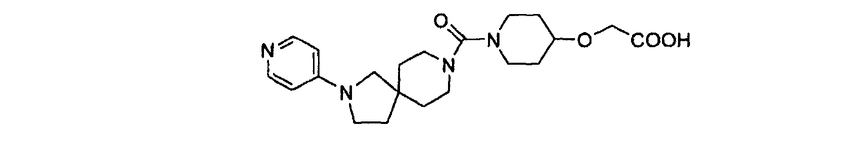

### Step A: Preparation of tert-butyl 4-(2-ethoxy-2-oxoethoxy)-1-piperidine carboxylate

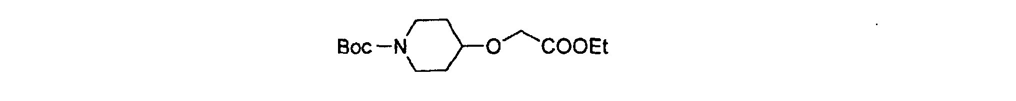

*tert*-Butyl-4-hydroxy-1-piperidine carboxylate (3.0 mMol) in CH₂Cl₂ (5.0 mL) was mixed with rhodium diacetate dimer (0.05 mMol), and treated with ethyl diazoacetate (4.0 mMol) in CH₂Cl₂ (10.0 mL). After the addition was complete the mixture was stirred for another 2 h. It was then filtered and concentrated to yield the title compound (76%).

### Step B: Preparation of Ethyl 2-(4-piperidyloxy)acetate

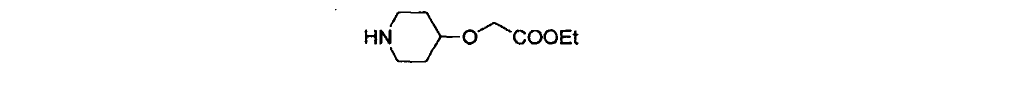

The *tert*-butoxycarbonyl compound from Step A (2.50 mMol) in CH₂Cl₂ (5.0 mL) at 0 °C was treated with TFA (5.0 mL). After 1 h at 0 °C the mixture was concentrated to yield the title compound.

### Step C: Preparation of 4-nitrophenyl 4-(2-ethoxy-2-oxoethoxy)-1-piperidine carboxylate

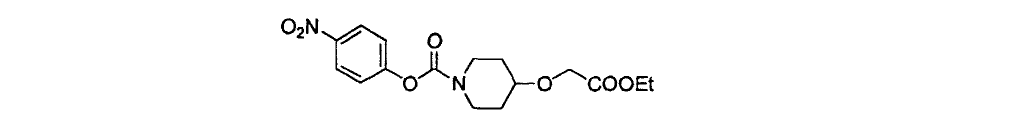

To a solution of piperidine from Step B (2.5 mMol) and DIEA (10 mMol) in CH₂Cl₂ (10 mL) at 0 °C was added 4-nitrophenyl chloroformate (2.8 mMol) in CH₂Cl₂ (10.0 mL). The yellow solution was stirred at room temperature for 19 h and then concentrated. The title compound was obtained by flash chromatography (72%).

### Step D: Preparation of ethyl 2-[(1-([2-(4-pyridyl)-2,8-diazaspiro[4.5]dec-8-yl]carbonyl }-4-piperidyl)oxy]acetate

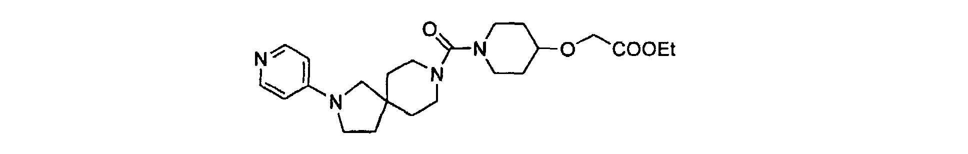

The 4-nitrophenyl carbamate from Step C (0.20 mMol) was mixed with the spirocyclic piperidine (0.20 mMol, from Step C, Example 28), and DIEA (1.0 mMol), and the mixture was heated to 75 °C for 18 h. The mixture was then partitioned between CH₂Cl₂ and satd. NaHCO₃. Evaporation of the CH₂Cl₂ extract yielded the title compound which was purified by RP-HPLC (56%).

### Step E: Preparation of 2-[(1-{2-(4-pyridinyl)-2,8-diazaspiro[4.5]dec-8-yl]carbonyl}-4-piperidyl)oxy]acetic acid

The ethyl ester from Step D (0.15 mMol) was treated with 2N HCl (5.0 mL) at room temperature After 4.0 h, the mixture was concentrated and the crude was purified by RP-HPLC to afford the title compound.
MS: ES (M+H)⁺ = 403
PRP (IC₅₀) = 3.785 µM ELISA (IC₅₀) = 0.138 µM.

### Example 45

### Preparation of 2-[(1-{[2-(4-pyridyl)-2,8-diazaspiro[4.5]dec-8-yl]carbonyl}-4-piperidyl)-methoxy]acetic acid

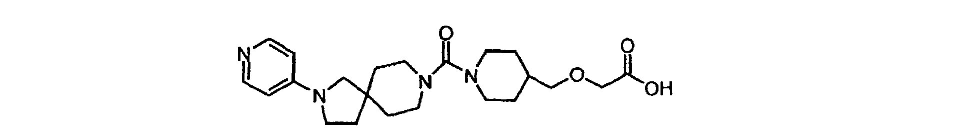

### Step A: Preparation of 4-piperidylmethanol

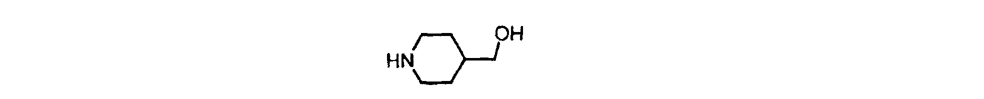

4-pyridylcarbinol (5.05 g, 45.8 mmol) dissolved in EtOH (75 ml) and concentrated HCl (3.81 ml, 45.8 mmol) was added. The starting material precipitated out and H₂O was added until compound dissolved again (~10 ml), then PtO₂ (1 g) was added. Stirred under H₂ pressure (220 psi) overnight.. The catalyst was filtered off and the solvent evaporated under reduced pressure to yield the slightly impure title compound (7.53 g, quantitative). This material was carried on directly to the next step without purification.

### Step B: Preparation of benzyl 4-(hydroxymethyl)-1-piperidinecarboxylate

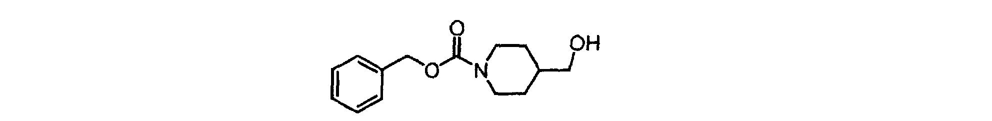

Dissolved the amine (5.0 g, 45.8 mmol) from Step A in CH₂Cl₂ (50 ml) with TEA (22.5 ml, 158.6 mmol) and cooled to 0°C, then added benzyl chloroformate (10.4 ml, 73.3 mmol)and stirred at room temperature ca. 24 hr. Extracted CH₂Cl₂ versus saturated NaHCO₃ and combined the organic layers, dried on MgSO₄, filtered and evaporated. Purified by flash chromatography to yield the pure title compound (3.34 g, 42%).
MS (APCI) (M+H)⁺=250

### Step C: Preparation of benzyl 4-[(2-ethoxy-2-oxoethoxy)methyl]-1-piperidine carboxylate

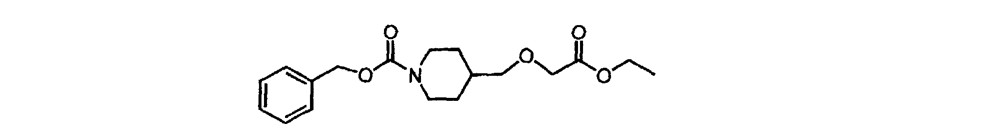

Dissolved the alcohol (3.5 g, 13.35 mmol) from Step B in CH₂Cl₂ (25 ml), then added rhodium diacetate dimer (30 mg, .067 mg) to the solution, followed by the dropwise addition of ethyl diazoacetate (1.5 ml, 14.7 mmol). N₂ gas evolved as the reaction proceeded. Filtered the reaction through celite and removed solvent on the rotary evaporator, then purified by flash chromatography to yield the pure title compound (3.5 g, 78%).

### Step D: Preparation of ethyl 2-(4-piperidylmethoxy)acetate

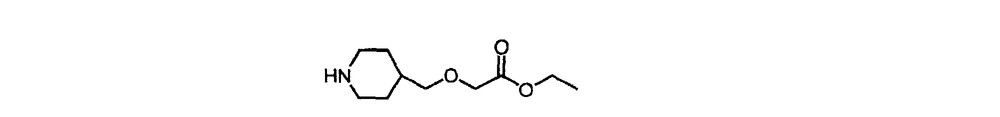

Dissolved the N-benzyl compound from Step C in EtOH (100 ml), added 5% Pd/C Degussa catalyst (400 mg), and stirred overnight under H₂ pressure (200 psi). Filtered through celite and evaporated under reduced pressure to yield a cream colored crystalline compound (2.25 g, quantitative).

### Step E: Preparation of 4-nitrophenyl 4-[(2-ethoxy-2-oxoethoxy)methyl]-1-piperidinecarboxylate

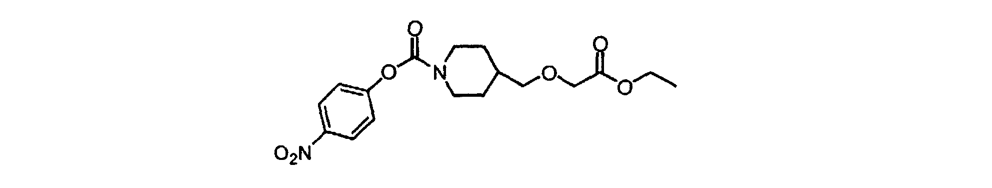

The material from Step D (2.19 g, 10.88 mmol) was converted to the p-nitrophenyl carbamate by substantially following the procedure from Example 44, Step C The compound was flash column purified to afford 2.42 g (61%)of the title compound.
MS (APCI) (M+H)⁺=367

### Step F: Preparation of ethyl 2-[(1-{[2-(4-pyridyl)-2,8-diazaspiro[4.5]dec-8-yl]carbonyl} } 4-piperidyl)methoxy]acetic acid

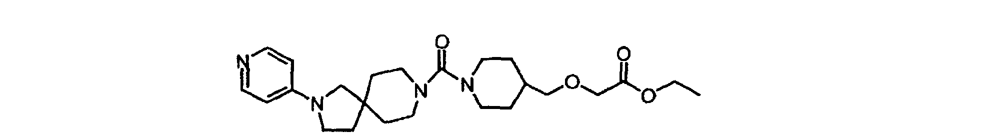

Part of the carbamate material from Step E (200 mg, 0.55 mmol) was reacted with 2-(4-pyridyl)-2,8-diazaspiro[4.5]decane from Step C, Example 28, (116 mg, 0.46 mmol) by substantially following the procedure from Example 44, Step D to afford 80 mg (40%) of the title compound.
MS (APCI) (M+H)⁺= 445

### Step G: Preparation of the title compound

The compound from Step F (80 mg) was hydrolyzed by substantially following the procedure from Example 44, Step E to afford 20 mg (27%) of the title compound.
PRP=2.824 µM ELISA=0.106 µM

### Example 46

### Preparation of 2-[methyl(1-{[2-(4-pyridyl)-2,8-diazaspiro[4.5]dec-8-yl)carbonyl}-4-piperidyl)amino]acetic acid

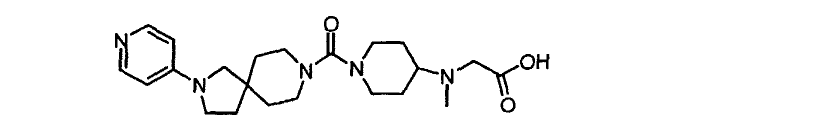

### Step A: Preparation of 2-[(1-benzyl-4-piperidyl)(methyl)amino]acetic acid

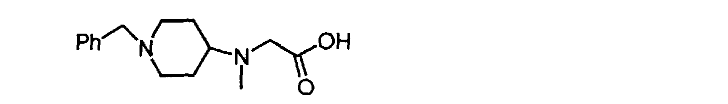

Dissolved N-benzyl 4-piperidone (2.0 g, 10.57 mmol) in 1,2 dichloroethane (10 ml) and added sarcosine (0.97 g, 10.57 mmol), which did not dissolve. Added acetic acid (0.63 ml, 10.57 mmol) and TEA (1.84 ml, 10.57 mmol) and checked pH=4. Stirred room temperature 24 hr, then evaporated the solvent under reduced pressure and suspended the solids with 0.1% TFA to break apart borate salts. RP-HPLC purified and lyophilized to yield the title compound as a fluffy white powder (2.74 g, 99%).
MS (ES) (M+H)⁺=263

### Step B: Preparation of ethyl 2-((1-benzyl-4-piperidyl)(methyl)amino]acetate

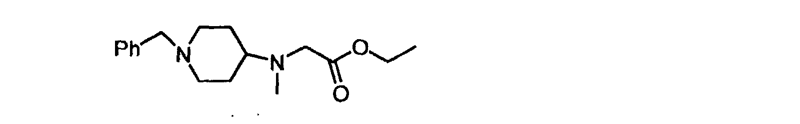

Dissolved the acid from Step A in EtOH (100 ml) and cooled to 0°C, then added thionyl chloride (9.90 ml, 135 mmol) dropwise over 30 minutes. A thick white precipitate formed, which was resuspended by the addition of EtOH (40 ml). Evaporated the solvent under reduced pressure, extracted CH₂Cl₂ versus saturated NaHCO₃, combined the organic layers, dried over MgSO₄, filtered and evaporated. RP-HPLC purified the crude and lyophilized to yield pure title compound as a fluffy white solid (1.58 g, quantitative).

### Step C: Preparation of ethyl 2-[methyl(4-piperidyl)amino]acetate

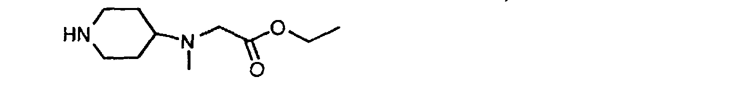

Dissolved the N-benzyl compound from Step B (1.37 g, 3.44mmol) in MeOH (10 mls), then added 10% Pd/C (200 mg) and placed under H₂ pressure (800 psi) o.n.. The catalyst was removed by filtration and MeOH was evaporated to yield 1.13 g the title compound as an oil.
MS (ES) (M+H)⁺=201

### Step D: Preparation of 4-nitrophenyl 4-[(2-ethoxy-2-oxoethyl)(methyl)amino]-1-piperidinecarboxylate

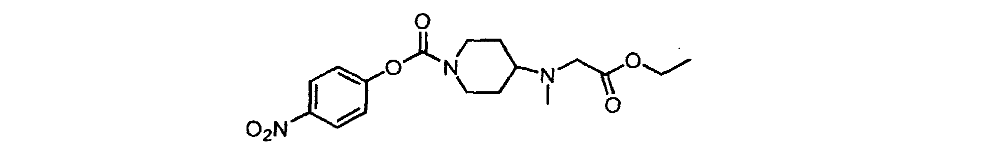

The material from Step C (1.13g, 5.65 mmol) was converted to the p-nitrophenyl carbamate by substantially following the procedure from Example 44, Step C. The compound was RP-HPLC purified to afford 0.56 g of the title compound.
MS (ES) (M+H)⁺=366

### Step E: Preparation of ethyl 2-[methyl(1-{[2-(4-pyridyl)-2,8-diazaspiro[4.5]dec-8-yl]carbonyl}-4-piperidyl)amino]acetate

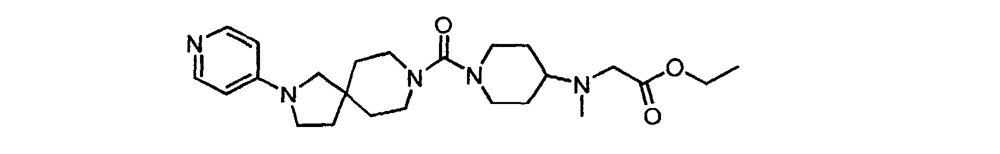

Part of the material from Step D (210 mg, 0.5 mmol) was reacted with 2-(4-pyridyl)-2,8-diazaspiro[4.5]decane from Step C, Example 28, by substantially following the procedure from Example 44, Step D to afford the ester of the title compound (50 mg).
MS (ES) (M+H)⁺=444

### Step F: Preparation of the title compound

This compound was hydrolyzed by substantially following the procedure from Example 44, Step E to afford 16.6 mg of the title compound as a white fluffy solid.
MS (ES) (M+H)⁺=4 17
PRP=9.999 µM

### Example 47

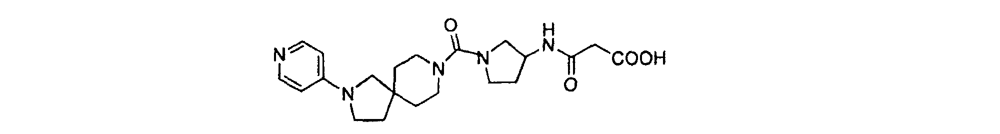

### Preparation of 3-oxo-3-[(1-{[2-(4-pyridyl)-2,8-diazaspiro[4.5]dec-8-yl]carbonyl}-tetrahydro-1 H-3-pyrrolyl)amino]propanoic acid

### Step A: Preparation of Ethyl 3-[(1-benzyltetrahydro-1H-3-pyrrolyl)amino]-3-oxopropanoate

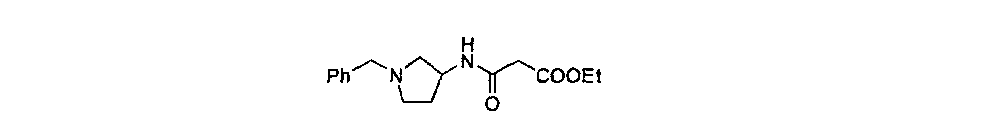

The reaction mixture comprising of 1-Benzyl-3-amino pyrrolidine (17.0 mMol), and DIEA (40.0 mMol) in DMF (20.0 mL) at 0 °C, was treated with ethyl malonyl chloride (21.0 mMol). The clear solution was stirred at room temperature for 17 h. The reaction was quenched with satd. NaHCO₃, and extracted with EtOAc. Concentration of the EtOAc extract yielded the title compound (91%).

### Step B: Preparation of Ethyl 3-oxo-3-(tetrahydro-1H-3-pyrrolylamino)propanoate

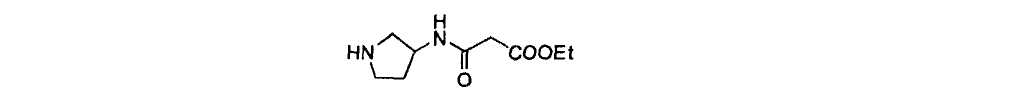

The N-benzyl pyrrolidine derivative from Step A (15 mMol) in EtOH (30.0 mL) was mixed with 20% Pd(OH)₂/C (500 mg) and the mixture was shaken on Parr hydrogenator at 50 psi of hydrogen for 20 h. It was then filtered and the clear filtrate was concentrated to yield the title compound as a colorless glue (93%).

### Step C: Preparation of 4-nitrophenyl 3-[(3-ethoxy-3-oxo-propanoyl)amino]-1-pyrrolidinecarboxylate

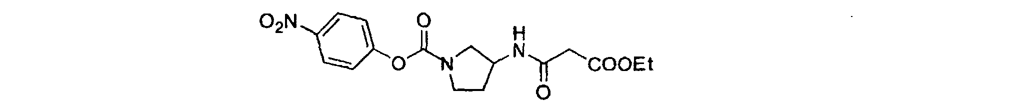

A mixture of pyrrolidine from Step B (5.0 mMol) and DIEA (30.0 mMol) in CH₂Cl₂ (20.0 mL) at 0 °C was treated with 4-nitrophenyl chloroformate (6.0 mMol) in the same solvent (5.0 mL). The clear pale yellow solution was stirred at room temperature for 18 h. Then all the solvents were rotavopped off and the crude product was purified by flash chromatography to afford the title compound (81%).

### Step D: Preparation of Ethyl 3-oxo-3-[(1-{[2-(4-pyridyl)-2,8-diazaspiro[4. 5]dec-8-yl]carbonyl}tetrahydro-1H-3-pyrrolyl)amino]propanoate

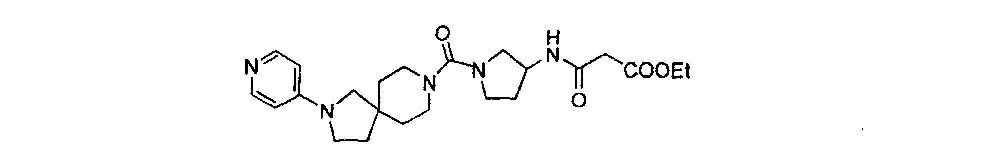

The reaction mixture comprising of 4-nitrophenyl carbamate (0.40 mMol) from Step C, spirocyclic piperidine from Step C, Example 28, (0.40 mMol), and DIEA (2.0 mMol) in DMF (5.0 mL) was heated to 75 °C for 20 h. It was then partitioned between CH₂Cl₂, and NaHCO₃. Concentration of the CH₂Cl₂ extract yielded the title compound. It was purified by RP-HPLC (49%).
MS: ES (M+H)⁺ = 444.

### Step E: Preparation of 3-oxo-3-[(1-{[2-(4-pyridyl)-2,8-diazaspiro[4.5]dec-8-yl]carbonyl}-tetrahydro-1H-3-pyrrolyl)amino]propanoic acid

The ethyl ester from Step D (0.240 mMol) in THF (7.0 mL) was treated with 1M LiOH (0.50 mMol). After 4.0 h at rt, the mixture was acidified with 1N HCl at 0 °C and concentrated. The title compound was obtained by RP-HPLC purification (76%).
MS: ES (M+H)⁺ = 417.
PRP (IC₅₀) = 5.856 µM ELISA (IC₅₀) = 0.447 µM.

### Example 48

### Preparation of 3-(4-{[2-(4-pyridyl)-2,8-diazaspiro[4.5]dec-8-yl]carbonyl}-piperidino)-butanoic acid

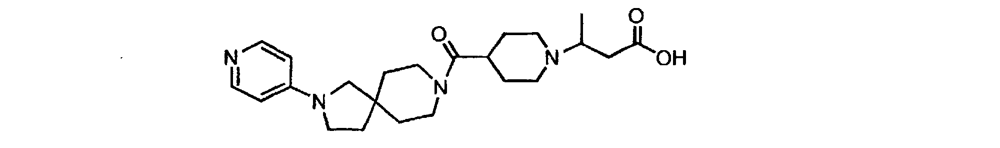

### Step A: Preparation of Benzyl 1-(3-ethoxy-1-methyl-3-oxopropyl)-4-piperidine carboxylate

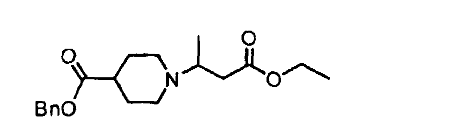

Benzyl 4-piperidinecarboxylate (1.26 g, 3.78 mmol), ethyl acetoacetate (1.44 ml, 11.35 mmol), sodium triacetoxyborohydride (2.4 g, 11.4 mmol), acetic acid (0.216 ml, 5.68 mmol) and TEA (1.98 ml, 11.35 mmol) were dissolved together in 1,2 dichloroethane (5 ml). The solution was adjusted to pH 5 with acetic acid and stirred at room temperature ca. 24 hr, at which time starting material was absent by RP-HPLC. The solution was treated with 0.1% TFA in water to break apart borate salts, extracted CH₂Cl₂, dried on MgSO₄, filtered, evaporated and purified by flash chromatography to yield pure title compound (331 mg, 26%).

### Step B: Preparation of 1-(3-ethoxy-1-methyl-3-oxopropyl)-4-piperidinecarboxylic acid

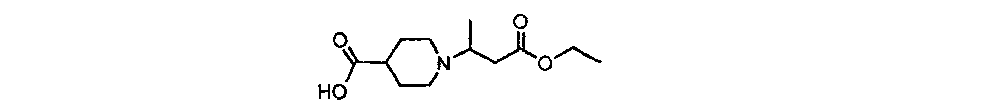

The benzyl ester from Step A (507 mg) was dissolved in Ethanol (30 ml). 10% Pd/C (100 mg) was added and the mixture was placed on the Parr hydrogenator at 50 psi H₂ pressure for 3 hr. Filtered reaction through celite and washed the catalyst with EtOH, then evaporated the solvent under reduced pressure to yield the pure title compound (368 mg, quantitative).
MS (ES) (M+H)⁺ = 244

### Step C: Preparation of ethyl 3-(4-{[2-(4-pyridyl)-2,8-diazaspiro[4.5]dec-8-yl]carbonyl } piperidino)butanoate

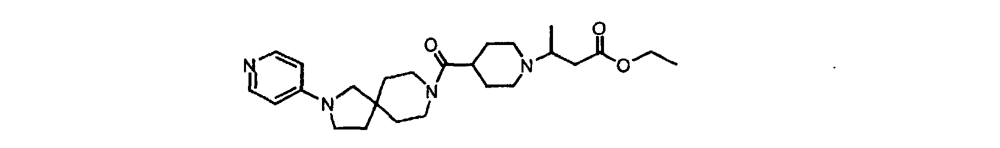

Suspended 2-(4-pyridyl)-2,8-diazaspiro[4.5]decane from Step C, Example 28, (106 mg, 0.324 mmol) in anhyd. DMF (3 ml) with DIEA (0.56 ml, 3.2 mmol) and checked that pH=10. Added acid from Step B (95 mg, 0.39 mmol), followed by HBTU (185 mg, 0.49 mmol), at which time the suspension dissolved and turned dark reddish. Stirred 2 hr at room temperature, until RP-HPLC analysis showed absence of the starting piperidine, then evaporated DMF in vacuo, extracted CH₂Cl₂ versus saturated NaHCO₃, combined the organic layers and dried on MgSO₄, filtered and evaporated. Purified by RP-HPLC to yield the pure title compound (110 mg, 77%).
MS (ES) (M+H)⁺ = 443

### Step E: Preparation of the title compound

This compound was hydrolyzed by substantially following the procedure from Example 44, Step E to afford 6 mg of the title compound as a white fluffy solid.
MS (ES) (M+H)⁺ = 415

### Example 49

### Preparation of 3-(4-{ [2-(4-pyridyl)-2,8-diazaspiro[4.5]dec-8-yl]carbonyl}piperidino)-propanoic acid

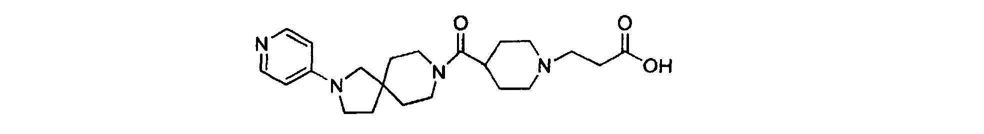

### Step A: Preparation of benzyl 1-(3-ethoxy-3-oxopropyl)-4-piperidinecarboxylate

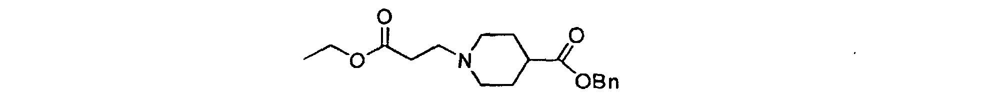

A mixture of 4-Benzyl-1-(*tert*-butyl)1,4-piperidinedicarboxylate from Step A, Example 12 (2.90 mMol), ethyl 3-bromo-propionate (3.20 mMol), and DIEA (30 mMol) in DMF (10.0 mL) was stirred at room temperature for 22 h. It was then washed with 1 N HCl, brine, dried, and concentrated to yield a light brown glue which was purified by flash chromatography to afford the title compound (68%).

### Step B: Preparation of 1-(3-ethoxy-3-oxopropyl)-4-piperidinecarboxylic acid

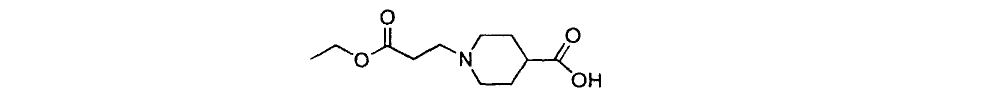

A mixture of the benzyl ester from Step A (2.0 mMol) and 20% Pd(OH)₂/C (200 mg) in ethyl acetate (20 mL) was shaken on a Parr hydrogenator at 50 psi of H₂. It was then filtered and concentrated to yield the title compound as a colorless glue (91%).

### Step C: Preparation of ethyl 3-(4{(2-(4-pyridyl)-2,8-diazaspiro[4.5]dec-8-yl]carbonyl}-piperidino)propanoate

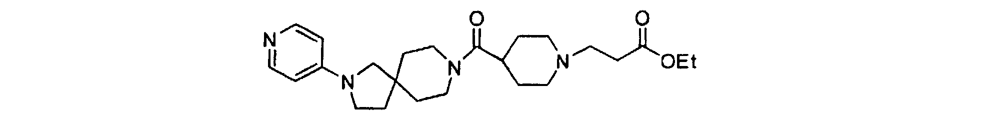

A mixture of the carboxy acid from Step B (0.340 mMol), spirocyclic piperidine from Step C, Example 28, (0.30 mMol), HBTU (0.40 mMol), and DIEA (3.0 mMol) in DMF (5.0 mL) was stirred at room temperature for 19 h. It was then diluted with CH₂Cl₂ (20 mL) and washed with satd. NaHCO₃, brine, dried, and concentrated to yield the crude product. It was purified by RP-HPLC to afford the title compound (66%).

### Step D: Preparation of 3-(4-{[2-(4-pyridyl)-2,8-diazaspiro[4.5]dec-8-yl]carbonyl} piperidino)- propanoic acid

The ethyl ester from Step C (0.150 mMol) was suspended in 2N HCl (10 mL) and stirred at room temperature for 5 h. It was then concentrated and purified by RP-HPLC to afford the title compound.
MS: ES (M+H)⁺= 401
PRP (IC₅₀) = 1.007 µM ELISA (IC₅₀) = 0.017 µM.

### Example 50

### Preparation of 2-[(1-{2-oxo-2-[2-(4-pyridyl)-2,8-diazaspiro[4.5]dec-8-yl]ethyl}-4-piperidyl)oxyl]acetic acid

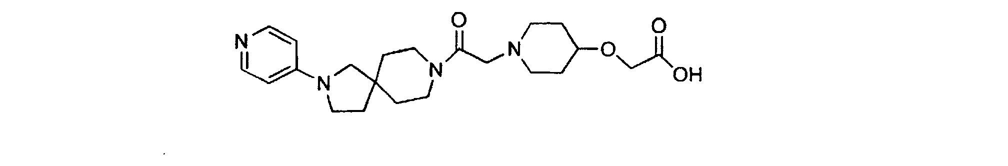

### Step A: Preparation of ethyl 2-({ 1-2[2-tert-butoxy)-2-oxoethyl]-4-piperidyl}oxy)acetate

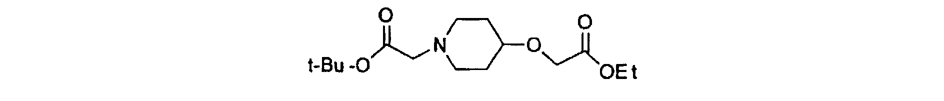

A solution of ethyl 2-(4-piperidyloxy)acetate (7.05 mMol), *tert*-butyl bromoacetate (7.40 mMol), and DIEA (21.0 mMol) in DMF (15 mL) was stirred at room temperature for 18 hours. The mixture was then partitioned between CH₂Cl₂ and saturated NaHCO₃. The CH₂Cl₂ extract was washed with brine, dried, and evaporated to yield the crude product which was purified by flash chromatography (85%).

### Step B: Preparation of 2-[4-(2-ethoxy-2-oxoethoxy)piperidino]acetic acid

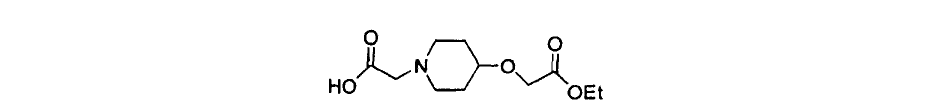

The *tert*-butyl ester (5.5 mMol) from Step A, at 0 °C, was treated with TFA (10 mL). After stirring at room temperature for 2.0 h the mixture was concentrated to yield the crude product.

### Step C: Preparation of ethyl 2-[(1-{2-oxo-2-[2-(4-pyridyl)-2,8-diazaspiro[4.5]dec-8-yl]ethyl }-4-piperidyl)oxy]acetate

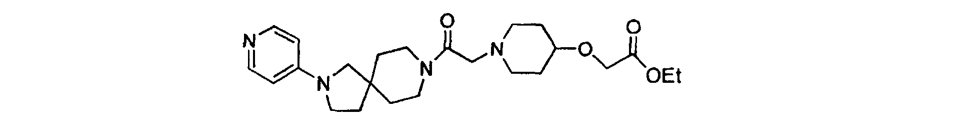

A mixture of the carboxy acid from Step B (0.195 mMol), tricyclic piperidine from Step C, Example 28, (0.189 m Mol), HATU (0.284 mMol), DIEA (0.946 mMol) in DMF (2.0 ml,) was stirred at room temperature for 21 h. The mixture was partitioned between CH₂Cl₂ and satd. NaHCO₃, washed with brine, dried, and concentrated to yield the crude product which was purified by RP-HPLC to afford the title compound (76%).

### Step D: Preparation of 2-[(1-{2-oxo-2-[2-(4-pyridyl)-2,8-diazaspiro[4.5]dec-8-yl]ethyl}-4-piperidyl)oxyl]acetic acid

The ethyl ester from Step C (0.23 mMol) was treated with 2N HCl (10.0 mL) at rt. After 6.0 h. the mixture was concentrated and the crude was purified by RP-HPLC to afford the title compound (74%).
MS: ES (M+H)⁺ = 417
PRP (IC₅₀) = 80.00 µM

### Example 51

### Preparation of ethyl 2-[(4-{[2-(4-pyridyl)-2,8-diazaspiro[4.5]dec-8-yl]carbonyl}-piperidino)sulfonyl]acetic acid

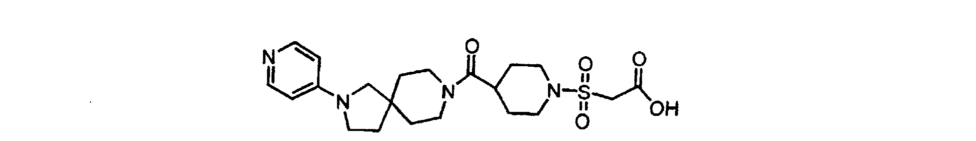

### Step A: Preparation of ethyl 2-chlorosulfonylacetate

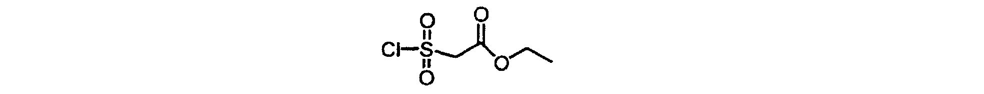

Sodium 2-ethoxy-2-oxo-1-ethanesulfonate (3.52g, 18.53 mmol) and phosphorus pentoxide (4.6 g, 22 mmol) were placed in a round bottom flask with a condenser and stirred magnetically for 1 hr. A liquid resulted which was stirred 1 hr at rt, then 1 hr at 100 °C while a copious white precipitate of NaCl formed. The reaction was concentrated to dryness, dissolved in CH₂Cl₂, filtered through a silica plug, and concentrated to yield the title compound as a light yellow oil.

### Step B: Preparation of benzyl 1-[(2-ethoxy-2-oxoethyl)sulfonyl]-4-piperidine carboxylate

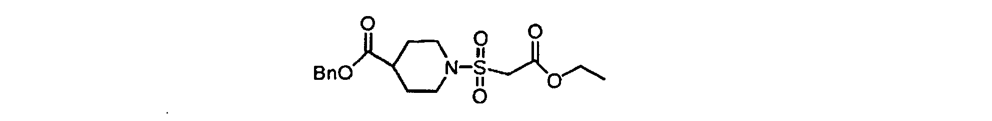

Benzyl 4-piperidinecarboxylate (760 mg, 2.28 mmol) was dissolved in anhydrous CH₂Cl₂ (20 ml) and DIEA (2.38 ml, 13.72 mmol) and cooled to 0 °C. Then ethyl 2-chlorosulfonylacetate (1.02 g, 5.48 mmol) from Step A was added slowly and the solution was stirred 5 hr as it came to rt. Extracted solution with CH₂Cl₂ versus saturated NaHCO₃, dried on MgSO₄, filtered, evaporated and purified by flash chromatography to yield the title compound (150 mg, 18%) as a colorless oil.

### Step C: Preparation of 1-[(2-ethoxy-2-oxoethyl)sulfonyl]-4-piperidinecarboxylic acid

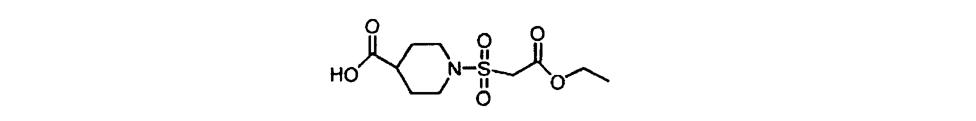

The benzyl ester from Step B (76 mg, 0.205 mmol) was dissolved in MeOH (20 ml) and 25 mg of 10% Pd/C was added and allowed to stir under a balloon of H₂ for 1 hr, then filtered and concentrated to yield pure title compound (67 mg, quantitative).

### Step D: Preparation ethyl 2-[(4-{[2-(4-pyridyl)-2,8-diazaspiro[4.5]dec-8-yl] carbonyl} piperidino)sulfonyl]acetate

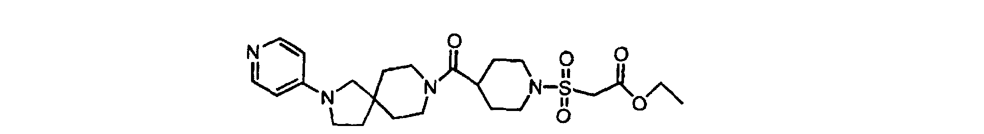

Dissolved the acid from Step C in DMF (63 mg, 0.225 mmol) and added DIEA (0.18 ml, 1.02 mmol) and 2-(4-pyridyl)-2,8-diazaspiro[4.5]decane (67 mg, 0.205 mmol), followed by HBTU (117 mg, 0.308 mmol). The solution turned reddish-orange and it was stirred o.n. at rt, then DMF was evaporated off and the crude was extracted with CH₂Cl₂ versus saturated NaHCO₃. The organic layers were combined and dried on MgSO₄, filtered, evaporated, and purified by RP-HPLC to yield the pure title compound.

### Step E: Preparation of the title compound

The ester from Step D was dissolved in 5 ml of 2 N HCl and stirred for 24 hr room temperature until the reaction was complete by RP-HPLC. Lyophilized to dryness and purified the compound by RP-HPLC to yield pure title compound as a fluffy white solid (22 mg).
MS (ES) (M+H)⁺ = 451
ELISA=0.094 µM PRP (ADP-Citrate) IC₅₀ =0.938 µM

### Example 52

### Preparation of 2-(4-{[2-(4-pyridyl)-2,8-diazaspiro[4.5]dec-8-yl]carbonyl} phenoxy)-acetic acid

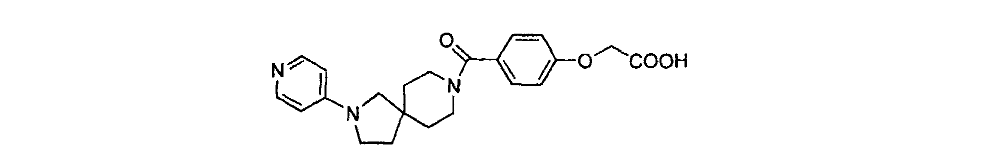

### Step A: Preparation of benzyl 4-(2-ethoxy-2-oxoethoxy)-benzoate

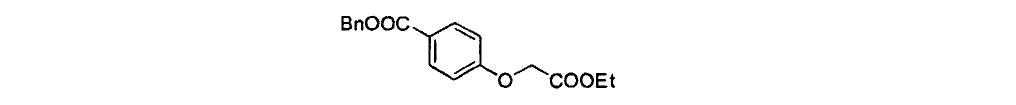

To a mixture of benzyl 4-hydroxybenzoate (10.950 mMol) and rhodium acetate dimer (1.0 mMol) in CH₂Cl₂ (40.0 mL) at room temperature was added a solution of ethyl diazoacetate (12.05 mMol) in CH₂Cl₂ (20.0 mL) dropwise over 1.0 h. The mixture was stirred for another 2.0 h, filtered, and concentrated to yield the crude product which was purified by flash chromatography to afford the title compound. (72%).

### Step B: Preparation of 4-(2-ethoxy-2-oxoethoxy)-benzoic acid

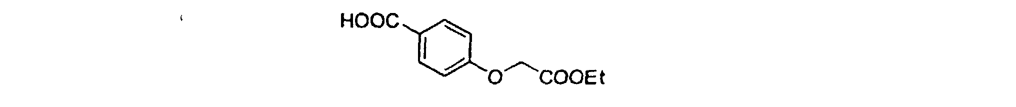

A mixture of the benzyl ester (8.0 mMol) from Step A and 20% Pd(OH)₂/C (250 mg) in EtOH (50 mL) was shaken on Parr hydrogenator at 50 psi of H₂ for 16 h. It was then filtered and concentrated to yield the crude product which was purified by flash chromatography to afford the title compound (91%).

### Step C: Preparation of ethyl 2-{4-{[2-(4-pyidyl)-2,8-diazaspiro[4.5]dec-8-yl]carbonyl} phenoxy)acetate

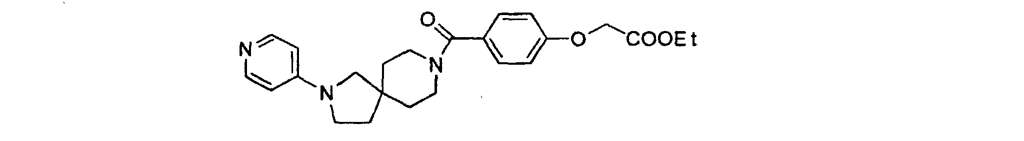

A mixture of the carboxy acid from Step B (0.287 mMol), spirocyclic piperidine from Step C, Example 28, (0.221 mMol), EDC.HCl (0.507 mMol), HOBt (0.442 mMol), and DIEA (0.552 mMol) in DMF (5.0 mL) was stirred at room temperature for 19 h. It was then diluted with CH₂Cl₂ and washed with satd. NaHCO₃, brine, dried, and concentrated to yield the crude product, which was purified by RP-HPLC to afford the title compound (79%).

### Step D: Preparation of 2-(4-{[2-(4-pyridyl)-2,8-diazaspiro[4.5]dec-8-yl]carbonyl} phenoxy)- acetic acid

The ethyl ester from Step C (0.180 mMol) was suspended in 2N HCl (10.0 mL) and stirred at room temperature for 5 h. It was then concentrated and purified by RP-HPLC to afford the title compound (73%).
MS: ES (M+H)⁺ =396.
ELISA (IC₅₀) = 0.688 µM PRP(ADP-Citrate) IC₅₀ = 11.285 µM

### Example 53

### Preparation of 2-(5-{[2(4-pyridyl)-2,8-diazaspiro[4.5]dec-8-yl]carbonyl}-1H-1-indolyl)acetic acid

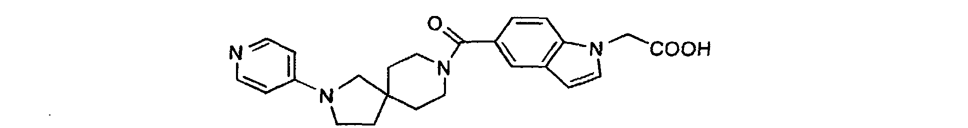

### Step A: Preparation of 1-(2-tert-butoxy-2-oxoethyl)-1H-5-indolecarboxylic acid

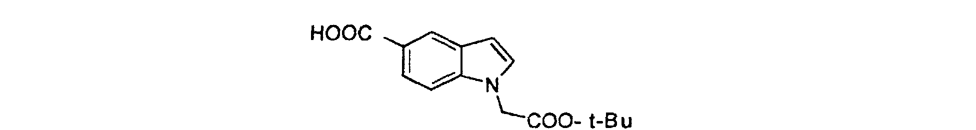

To Sodium hydride (6.87 mMol) in DMF (5.0 mL) at 0 °C was added a solution of Indole-4-carboxylic acid (3.10 mMol) in DMF (4.0 mL). The thick colorless suspension was stirred at 0 °C for 1.0 h and treated with a solution of *tert*-butyl bromoacetate (3.20 mMol) in DMF (1.0 mL). The mixture was warmed to room temperature and then stirred there o.n. The reaction was quenched with 1M citric acid at 0 °C, and extracted with CH₂Cl₂. The title compound was obtained by concentration of the CH₂Cl₂ extract (64%).

### Step B: Preparation of tert-butyl 2-(5-{[2-(4-pyridyl)-2,8-diazaspiro[4.5]dec-8-yl]carbonyl }-1H-1-indolyl)acetate

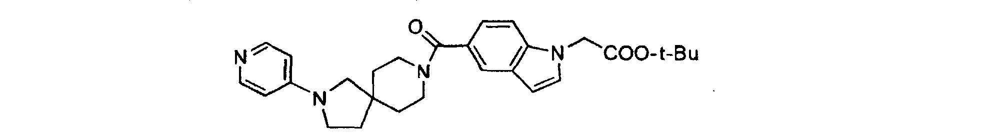

4-Carboxy-indole-N-ethyl acetate from Step A (0.398 mMol) was mixed with the spirocyclic piperidine from Step C, Example 28, (0.39 mMol), HBTU (0.79 mMol), and DIEA (1.72 mMol) in DMF (4.0 mL). The solution was then stirred at room temperature for 18 h. The reaction was quenched with satd. NaHCO₃ and extracted with CH₂Cl₂. The title compound was obtained by evaporation of the CH₂Cl₂ extract, followed by flash chromatography (80%).

### Step C: Preparation of 2-(5-{[2(4-pyridyl)-2,8-diazaspiro[4.5]dec-8-yl]carbonyl}-1 H-1 -indolyl)acetic acid

The *tert*-butyl ester from Step B was treated with TFA (5.0 mL) at rt. After 2.0 h the mixture was concentrated to yield the title compound which was purified by RP-HPLC (81%).
MS: ES (M+H)⁺ = 419.
PRP (ADP-Citrate) IC₅₀= 0.952 µM ELISA (IC₅₀)=0.047 µM.

### Example 54

### Preparation of 2-[2-({2-[2-(4-pyridyl)-2,8-diazaspiro[4.5]dec-8-yl]acetyl}amino)-1,3-thiazol-4-yl]acetic acid

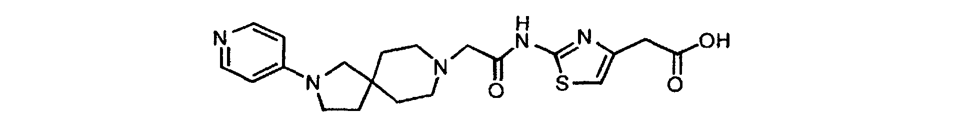

### Step A: Preparation of ethyl 2-[2-({2-[2-(4-pyridyl)-2,8-diazaspiro[4.5]dec-8-yl]acetyl }amino)- 1 ,3-thiazol4-yl]acetate

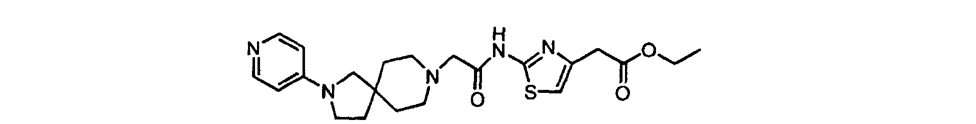

Suspended 2-(4-pyridyl)-2,8-diazaspiro[4.5]decane from Step C, Example 28, (106 mg, 0.324 mmol) in anhydrous 1,2 dichloroethane with DIEA (0.16 ml, 0.92 mmol), added ethyl 2-(2-chloroacetamido)-4-thiazoleacetate (52 mg, 0.199 mmol) and stirred o.n. at rt. Evaporated the solvent, extracted CH₂Cl₂ versus saturated NaHCO₃, combined the organic layers and dried over MgSO₄, filtered and evaporated. Purified by RP-HPLC to yield of pure title compound (6.7 mg, 10%).
MS (ES) (M+H)⁺=444

### Step B: Preparation of the title compound

The compound from Step A (6.7 mg) was hydrolyzed by substantially following the procedure from Example 51, Step E to afford 3.9 mg (62%) of the title compound as a white fluffy solid.
MS (ES) (M+H)⁺=417
PRP (ADP-Citrate) IC₅₀ =32.072 µM ELISA IC₅₀=2.280 µM

### Example 55

### Preparation of 3-({[2-(4-pyridyl)-2,8-diazaspiro[4.5]dec-8-yl]carbonyl}amino)propanoic acid

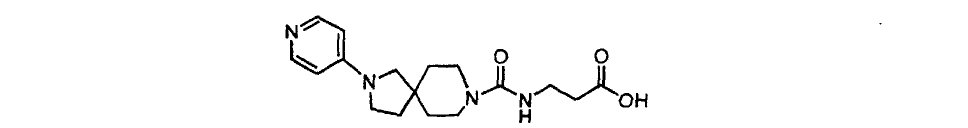

### Step A: Preparation of ethyl 3-({[2-(4-pyridyl)-2,8-diazaspiro[4.5]dec-8-yl]carbonyl } -amino )propanoate

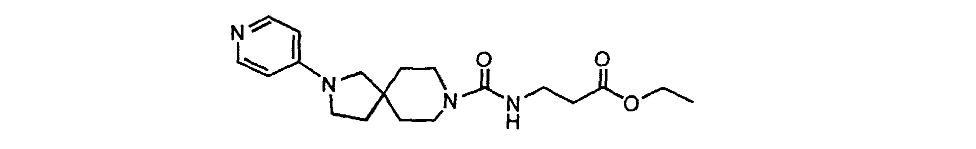

2-(4-pyridyl)-2,8-diazaspiro[4.5]decane from Step C, Example 28, (36 mg, 0.11 mmol) was suspended in CH₂Cl₂ (2 ml) and DIEA (0.077ml, 0.44 mmol), then cooled to 0°C and reacted with ethyl 3-isocyanatopropionate (21 mg, 0.143 mmol) room temperature on.. Evaporated solvent and purified by RP-HPLC to yield pure title compound (42 mg, quantitative).
MS (DCI) (M+H)⁺=361

### Step B: Preparation of the title compound

The compound from Step A (80 mg) was hydrolyzed by substantially following the procedure from Example 51, Step E to afford 20 mg (27%) of the title compound.
MS (DCI) (M+H)⁺=333
PRP IC₅₀ (ADP-Citrate)= 34.63 µM ELISA IC₅₀= 28.9 µM

The following assay methods are suitable for evaluating the compounds of the invention.

### No. 1 - The ELISA IIb-IIIa Assay:

In the following assay, GPIIb-IIIa is prepared in purified form, by a method such as described by Fitzgerald, L.A., et al., Anal Biochem (1985) 151:169-177, (the disclosure of which is incorporated herein by reference).

PIIb-IIIa is coated onto microtiter plates. The coated support is then contacted with fibrinogen and with the test material (e.g., compounds of Formula I) and incubated for a sufficient time to permit maximal binding of fibrinogen to the immobilized GPIIb-IIIa. Fibrinogen is typically provided at a concentration of about 5-50 nM and the test material can, if desired, be added at a series of dilution. Typical incubations are 2 to 4 hours at 25 °C, the time and temperature being interdependent.

After incubation, the solution containing the fibrinogen and test material is removed and the level of binding of fibrinogen measured by quantitating bound fibrinogen to GPIIb-IIIa. Any suitable means of detection may be used, but it is convenient to employ labeled fibrinogen, for example using biotinylated labels. Such methods are well known in the art.

### A. Description of Assays-Plate Assays

Purified platelet GPIIb-IIIa receptor was prepared as described by Fitzgerald, L.A., et al., Anal Biochem (1985) 151:169-177 (1985). Vitronectin receptor was prepared as described by Smith, J.W., J. Biol Chem (1988) 263:18726-18731. After purification, the receptors were stored in 0.1% Triton X-100 at 0.1-1.0 mg/ml.

The receptors were coated to the wells of 96-well flat-bottom ELISA plates (Linbro ElA-Plus microtiter plate, Flow Laboratories) after diluting 1:200 with a solution of 20 mM Tris-HCl, 150 mM NaCl, 1 mM CaCl₂, pH 7.4, to reduce the Triton X-100 concentration to below its critical micellar concentration and adding an aliquot of 100 ul to each well. The wells were all allowed to incubate overnight at 4 °C, and then aspirated to dyness. Additional sites were blocked by the addition of bovine serum albumin (BSA) at 35 mg/ml in the above buffer for two hours at 30 °C to prevent nonspecific binding. The wells were then washed once with binding buffer (50 nM Tris-HCl, 100 mM NaCI 2 mM CaCl₂, 1 mg/ml BSA).

The corresponding ligands (fibrinogen, von Willebrand Factor, or vitronectin) were conjugated to biotin using commercially available reagents and standard protocols. The labeled ligands were added to the receptor-coated wells at final concentration of 10 nM (100 ul/well) and incubated for 3 hours at 25 °C in the presence or absence of the test samples. After incubation, the wells are aspirated to dryness and bound ligand is quantitated.

The bound protein is detected by the addition of antibiotin antibody conjugated to alkaline phosphatase followed by addition of substrate (p-nitrophenyl phosphate), and determination of the optical density of each well at 405 nM. Decreased color development is observed in wells incubated with test samples which inhibit binding of ligand to receptor.

### No. 2 - The Platelet Aggregation Assay

In addition to the ELISA IIb-IIIa assay previously described the Aggregation-Human PRP/ADP Assay is useful for evaluating therapeutic compounds.

Platelet-rich plasma was prepared from healthy human volunteers for use in determining inhibition of platelet aggregation by the compounds. Blood was collected via a 21-gauge butterfly cannula, using a two-syringe technique into 1/10 volume of 3.8% trisodium citrate.

Platelet-rich plasma was prepared at room temperature by centrifugation of the citrated whole blood at 100 x g for twelve minutes. The platelet rich plasma contained approximately 200-400,000 platelets/µl.

Platelet-poor plasma was prepared by centrifugation of citrated whole blood at 12,000 x g for 2 minutes.

Platelet aggregation was assayed in a 4-channel platelet aggregation profiler (PAP-4, Biodata, Hatboro, PA) according to the manufacturers directions. Inhibition of platelet aggregation was studied by adding varying amounts of adenosine diphosphate (ADP) to stirred human platelet-rich plasma. Specifically, the human platelet-rich plasma was incubated with the compound being tested for 1 minute at 37 °C prior to the addition of a variety of aggregating agents most often ADP 5 µM to 20 µM, but also 1 µg/ml collagen, 1 µM U46619 and 0.3 µM platelet activating factor.

### Pharmaceutical Compositions

Pharmaceutical formulations containing compounds of the invention can be administered orally in the form of tablets, capsules, solutions, emulsions or suspensions, inhaled liquid or solid particles, as a spray, through the skin by an appliance such a transdermal patch (such as described in US Patents No. 5,296,222 and 5,271,940, the disclosures of which are incorporated herein by reference) or rectally, for example, in the form of suppositories. The lipophilic prodrug derivatives of the invention are particularly well suited for transdermal absorption administration and delivery systems. Administration can also take place parenterally, for example in the form of injectable solutions.

Tablets are prepared by mixing the Active Ingredient ("Active Ingredient" is one or more spirocyclic bicyclic compounds of the invention inclusive of those corresponding to formulae I) with pharmaceutically inert, inorganic or organic carriers, diluents, and/or excipients. Examples of such excipients which can be used for tablets, are lactose, maize starch or derivatives thereof, talc, stearic acid or salts thereof. Examples of suitable excipients for soft gelatin capsules are vegetable oils, waxes, fats, semisolid and liquid polyols.

Suitable excipients for the preparation of solutions and syrups are water, polyols, sucrose, invert sugar and glucose.

Suitable excipients for injectable solutions are water, alcohols, polyols, glycerol and vegetable oils.

These pharmaceutical products can additionally contain preservatives, solubilizers, stabilizers, wetting agents, emulsifiers, sweeteners, colorants, flavorings, buffers, coating agents and antioxidants.

Pharmaceutical compositions of this invention for parenteral injection comprise pharmaceutically-acceptable sterile aqueous or nonaqueous solutions, dispersions, suspensions or emulsions as well as sterile powders for reconstitution into sterile injectable solutions or dispersions just prior to use.

The Active Ingredient can also be made in micro-encapsulated form.

### Exemplary formulations using the Active Ingredient are described below:

### Formulation 1

Hard gelatin capsules are prepared using the following ingredients:

| | (mg/capsule) |
|---|---|
| Active Ingredient | 250.0 |
| Starch | 305.0 |
| Magnesium stearate | 5.0 |

The above ingredients are mixed and filled into hard gelatin capsules in 560 mg quantities.

### Formulation 2

A tablet formula is prepared using the ingredients below:

| | (mg/tablet) |
|---|---|
| Active Ingredient | 250.0 |
| Cellulose, microcrystalline | 400.0 |
| Colloidal silicon dioxide | 10.0 |
| Stearic acid | 5.0 |

The components are blended and compressed to form tablets, each weighing 665 mg.

### Formulation 3

A dry powder inhaler formulation is prepared containing the following components:

| | Weight % |
|---|---|
| Active ingredient | 5 |
| Lactose | 95 |

The active mixture is mixed with the lactose and the mixture is added to a dry powder inhaling appliance.

### Formulation 4

Tablets, each containing 60 mg of active ingredient, are prepared as follows:

| | (milligrams) |
|---|---|
| Active ingredient | 60.0 |
| Starch | 45.0 |
| Microcrystalline cellulose | 35.0 |
| Polyvinylpyrrolidone (10% solution in water) | 4.0 |
| Sodium carboxymethyl starch | 4.5 |
| Magnesium stearate | 0.5 |
| Talc | 1.0 |
| Total | 150.0 |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules as produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 150mg.

### Formulation 5

Capsules, each containing 80 mg of medicament are made as follows:

| | (milligrams) |
|---|---|
| Active ingredient | 80.0 |
| Starch | 109.0 |
| Magnesium stearate | 1.0 |
| Total | 190.0 |

The active ingredient, cellulose, starch, and magnesium stearate are blended passed through a No. 20 mesh U.S. sieve, and filled into hard gelatin capsules in 190 mg quantities.

### Formulation 6

Suppositories, each containing 225 mg of active ingredient are made as follows:

| | |
|---|---|
| Active Ingredient | 225 mg |
| Saturated fatty acid glycerides to | 2000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Formulation 7

Suspensions, each containing 50 mg of medicament per 5.0 mL dose are made as follows:

| | |
|---|---|
| Active ingredient | 50.0 mg |
| Xanthan gum | 4.0 mg |
| Sodium carboxymethyl cellulose | (11%) |
| Microcrystalline cellulose | (89%) 50.0 mg |
| Sucrose | 1.75 g |
| Sodium benzoate | 10.0 mg |
| Flavor | q.v. |
| Color | q.v. |
| Purified water to | 5.0mL |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Formulation 8

Capsules, each containing 150 mg of medicament, are made as follows:

| | (milligrams) |
|---|---|
| Active ingredient | 150.0 |
| Starch | 407.0 |
| Magnesium stearate | 3.0 |
| Total | 560.0 |

The active ingredient, cellulose, starch, and magnesium stearate are blended, passed through a No. 20 mesh U.S. sieve, and filled into hard gelatin capsules in 560 mg quantities.

### Methods of Treatment

This invention provides a method of preventing or treating thrombosis in mammals, especially humans, which method comprises administering to the human or mammal a therapeutically effective amount of the compounds of this invention. The platelet aggregation inhibitors of the invention are useful therapeutically to prevent thrombus formation. Indications appropriate to such treatment include, without limitation, atherosclerosis and arteriosclerosis, acute myocardial infarction, chronic unstable angina, transient ischemic attacks and strokes, peripheral vascular disease, arterial thrombosis, preeclampsia, embolism, restenosis and/or thrombosis following angioplasty, carotid endarterectomy, anastomosis of vascular grafts, and chronic cardiovascular devices (e.g., in-dwelling catheters or shunts "extracorporeal circulating devices"). These syndromes represent a variety of stenotic and occlusive vascular disorders thought to be initiated by platelet activation on vessel walls.

The PAIs may be used for prevention or abortion of arterial thrombus formation, in unstable angina and arterial emboli or thrombosis, as well as treatment or prevention of myocardial infarction (MI) and mural thrombus formation post MI. For brain-related disorders, treatment or prevention of transient ischemic attack and treatment of thrombotic stroke or stroke-in-evolution are included.

The PAIs may also be used for prevention of platelet aggregation, embolization, or consumption in extracorporeal circulations, including improving renal dialysis, cardiopulmonary bypasses, hemoperfusions, and plasmapheresis.

PAIs prevent platelet aggregation, embolization, or consumption associated with intravascular devices, and administration results in improved utility of intraaortic balloon pumps, ventricular assist devices, and arterial catheters.

The PAIs will also be useful in treatment or prevention of venous thrombosis as in deep venous thrombosis, IVC, renal vein or portal vein thrombosis, and pulmonary venous thrombosis.

Various disorders involving platelet consumption, such as thrombotic thrombocytopenic purpura are also treatable.

In addition, the PAIs of the present invention may be used in numerous nontherapeutic applications where inhibiting platelet aggregation is desired. For example, improved platelet and whole blood storage can be obtained by adding sufficient quantities of the compounds, the amount of which will vary depending upon the length of proposed storage time, the conditions of storage, the ultimate use of the stored material, etc.

Preferably, the compounds of this invention are administered in the form of a pharmaceutical formulation. Thus, the compounds of this invention may be administered orally, parenterally, topically, rectally and etc., in, appropriate dosage units, as desired.

The term parenteral as used herein includes subcutaneous, intravenous, intraarterial, injection or infusion techniques, without limitation. The term, "topically" encompasses administration rectally and by inhalation spray, as well as the more common routes of the skin and the mucous membranes of the mouth and nose.

Actual dosage levels of active ingredients in the pharmaceutical compositions of this invention may be varied so as to administer an amount of the active compound(s) that is effective to achieve the desired therapeutic response for a particular patient.

The selected dosage level will depend upon the activity of the particular compound, the route of administration, the severity of the condition being treated, and the condition and prior medical history of the patient being treated. However, it is within the skill of the art to start doses of the compound at levels lower than required to achieve the desired therapeutic effect and to gradually increase the dosage until the desired effect is achieved. If desired, the effective daily dose may be divided into multiple doses for purposes of administration, e.g., two to four separate doses per day. It will be understood, however, that the specific dose level for any particular patient will depend upon a variety of factors including the body weight, general health, diet, time and route of administration, combination with other drugs and the severity of the particular disease being treated.

The range of therapeutic dosages is from about 0.01 to about 10,000 milligrams per day, with from 1 to 300 milligrams being preferred.

Many modifications and variations of this invention may be made without departing from its scope, as is apparent to those skilled in the art. The specific embodiments described herein are offered by way of example only, and the invention is to be limited only by the terms of the appended claims.

## Claims

1. A compound of formula (I) or a pharmaceutically acceptable salt, solvate, or prodrug thereof: 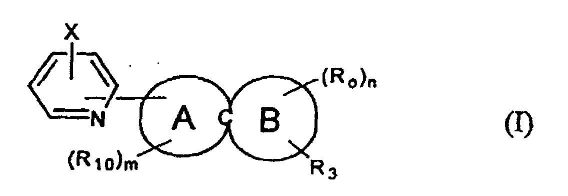 wherein;
the spirocycle nucleus A/B is selected from the group consisting of: 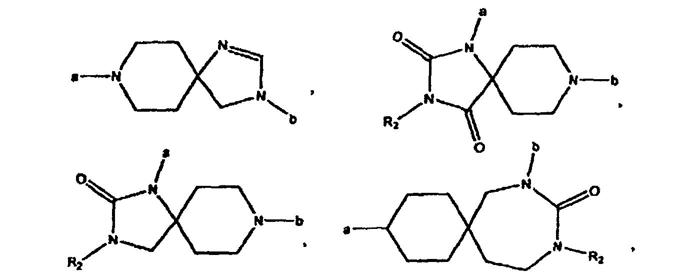 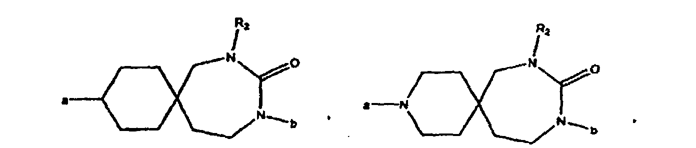 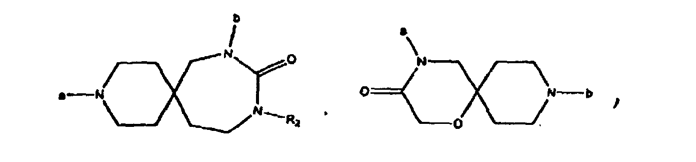 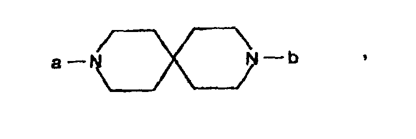 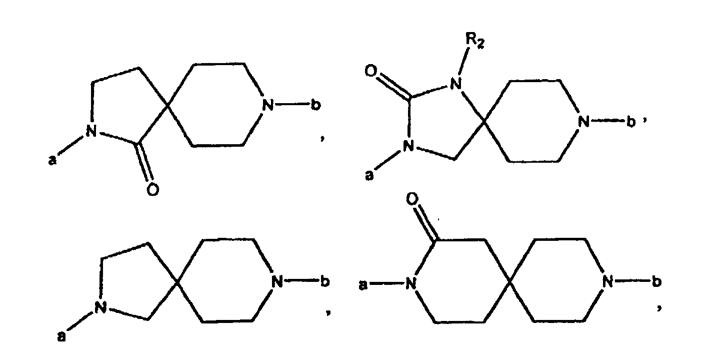 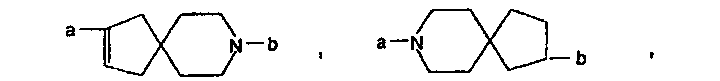 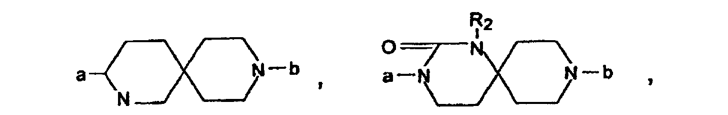 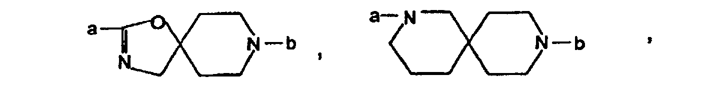 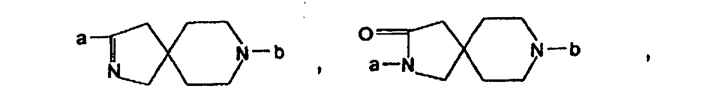 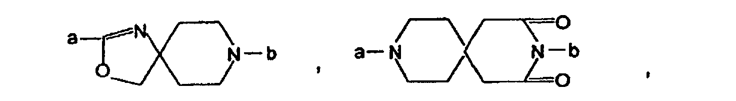 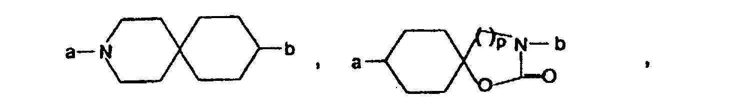 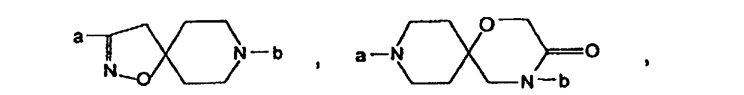 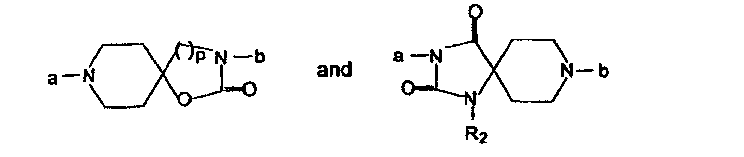 wherein
p is a number from 1 to 2,
one of the "a" and "b" attachment points of the spirocyclic nucleus is attached to a
ring carbon atom on the pyridyl group while the other is attached to the R₃ group, and
R₂ is a R₁₀ group if the "a" attachment point is attached to the pyridyl group, otherwise R₂ is a R₀ group;
R₁₀ is the same or different and is a non-interfering substituent independently selected from hydrogen, alkyl, halosubstituted alkyl, alkenyl, alkynyl, cycloalkyl, aryl, arylalkyl, hydroxy, alkoxy, arylalkoxy, amino, substituted amino, carbamoyl, carboxy, acyl, cyano, halo, nitro, sulfo, =O, or =S, with the proviso that only one R₁₀ may be =O or =S;
m is a number from zero to 9;
R₀ is the same or different and is a non-interfering substituent independently selected from hydrogen, alkyl, halosubstituted alkyl, alkenyl, alkynyl, cycloalkyl, aryl, arylalkyl, hydroxy, alkoxy, arylalkoxy, amino, substituted amino, carbamoyl, carboxy, acyl, cyano, halo, nitro, sulfo, =O or =S, with the proviso that only one R₀ may be =O or =S;
n is a number from zero to 9;
X is a substituent selected from the group consisting of hydrogen, halo, -C₁₋₆alkyl, C₁₋₆alkoxy, C₂₋₆alkenyl, C₂₋₆alkynyl, C₃₋₈cycloalkyl, C₀₋₄alkylC₃₋₈cycloalkyl, -CN, -NO₂, -(CH₂)ⱼ-N(-R^{a},-R^{b}), -C(=O)-N(-R^{a},-R^{b}), -S(=O)₂-N(-R^{a},-R^{b}), -S(=O)₂-R^{a}, -CF₃, and -(CH₂)ⱼ-O-R^{a};
wherein
R^{a} and R^{b} are independently selected from the group consisting of H, -C₁₋₆alkyl, C₁₋₆alkoxy, C₂₋₆akenyl, C₂₋₆alkynyl, C₃₋₈cycloalkyl, C₀₋₆alkylC₃₋₈cycloalkyl, and -C₀₋₆alkyl-(carbocyclic aryl), wherein from 0-4 hydrogen atoms on the ring atoms of the carbocyclic aryl moiety may be independently replaced with a member selected from the group consisting of halo, C₁₋₄alkyl, C₂₋₆alkenyl, C₂₋₆alkynyl, C₃₋₈cycloalkyl, C₀₋₄alkylC₃₋₈cycloalkyl, -CN, -CF₃ and -NO₂; and
j is a number from 0 to 2;
and
R₃ is an acidic group containing one or more acid radicals.

2. A spirocyclic compound according to Claim 1, wherein the A/B nucleus is selected from the group consisting of:

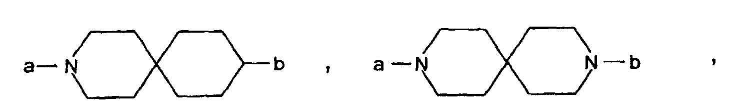

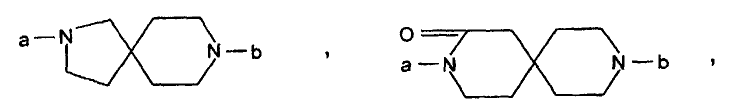

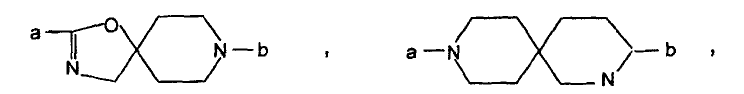

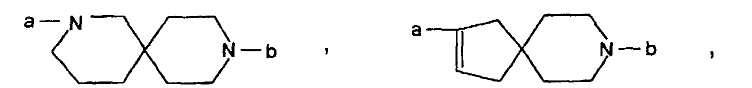

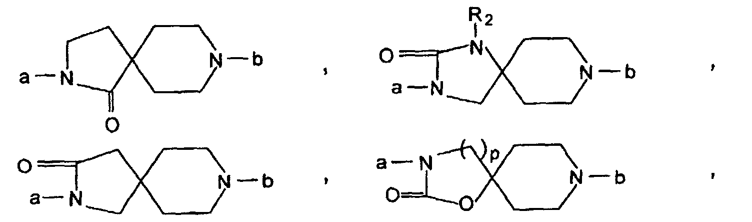

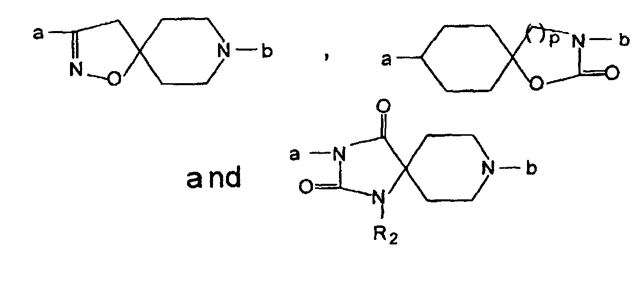

3. A spirocyclic compound according to Claim 1, wherein the A/B nucleus is selected from the group consisting of:

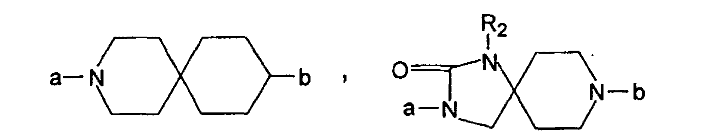

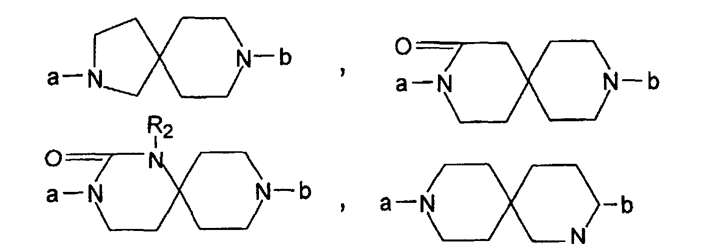

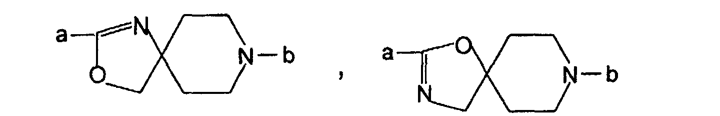

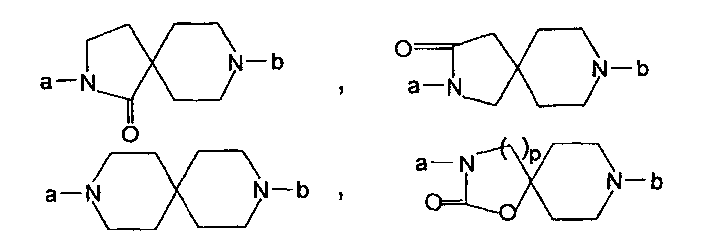

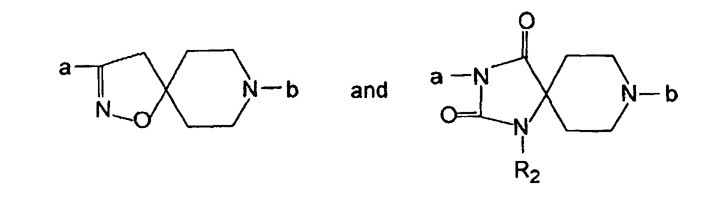

4. A spirocyclic compound according to Claim 1, wherein the A/B nucleus is selected from the group consisting of:

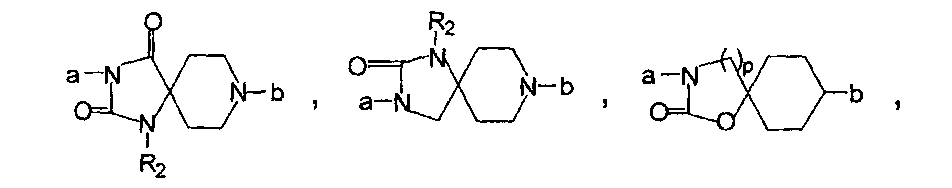

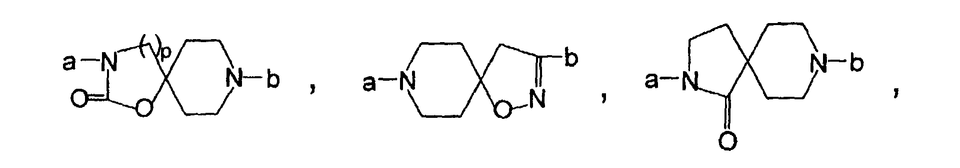

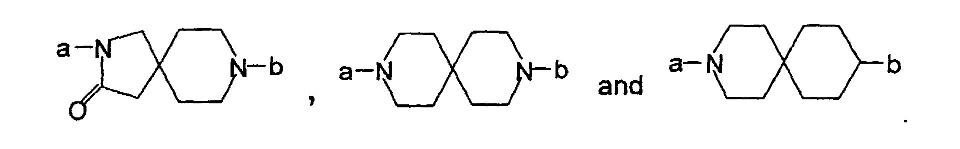

5. The compound of claim 4 wherein the "a" attachment point of the spirocyclic nucleus is attached to the pyridyl group.

6. The compound of claim 4 wherein the "b" attachment point of the spirocyclic nucleus is attached to the pyridyl group.

7. The compound of Claim 1 wherein:
the pyridyl radical is a member selected from the group consisting of: 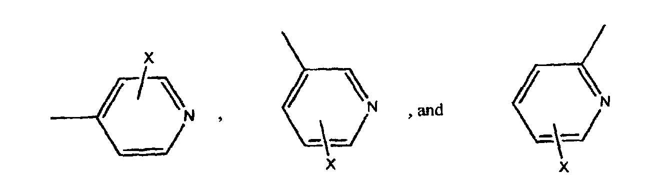
and X is a member selected from the group consisting of hydrogen, lower alkyl, halogen and trihalomethyl.

8. The compound of Claim 2 wherein:
the pyridyl radical is a member selected from the group consisting of: 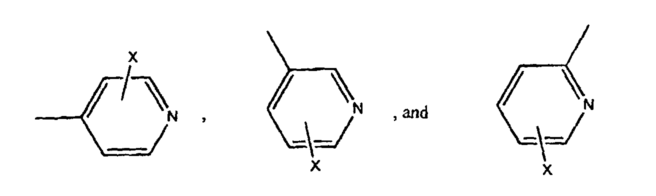
and X is a member selected from the group consisting of hydrogen, lower alkyl, halogen and trihalomethyl.

9. The compound of Claim 3 wherein:
the pyridyl radical is a member selected from the group consisting of: 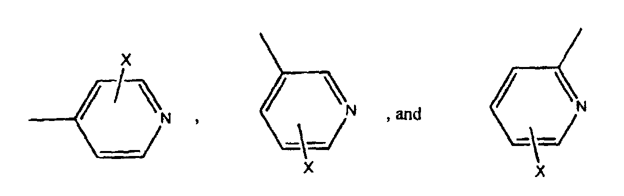
and X is a member selected from the group consisting of hydrogen, lower alkyl, halogen and trihalomethyl.

10. The compound of Claim 4 wherein:
wherein the pyridyl radical is a member selected from the group consisting of: 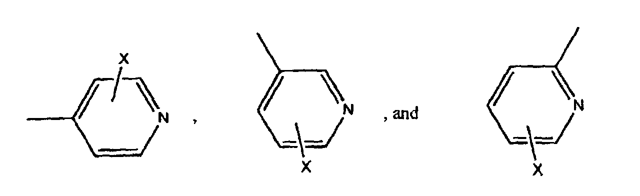 and X is a member selected from the group consisting of hydrogen, lower alkyl, halogen and trihalomethyl.

11. The compound of Claim 10 wherein the pyridyl radical is:

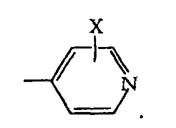

12. The compound of Claim 11 wherein the pyridyl radical is:

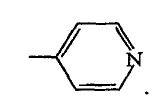

13. The compound of Claim 1 wherein R₃ is CO₂R_{5,} (C₁-C₆ alkyl)CO₂R₅, CO(C₁-C₆ alkyl)CO₂R₅, or CONH(C₁-C₆ alkyl)CO₂R₅, wherein R₅ is hydrogen, C₁-C₆ alkyl, aryl, or substituted aryl.

14. The compound of Claim 1 wherein R₃ is (C₁-C₆ alkyl)CH(NHR₄)CO₂R₅, CO(C₁-C₆ alkyl)CH(NHR₄)CO₂R₅, or CONH(C₁-C₆ alkyl)CH(NHR₄)CO₂R₅,
wherein R₄ is SO₂(C₁-C₆ alkyl), SO₂ aryl, or SO₂ substituted aryl; and R₅ is hydrogen, C₁-C₆ alkyl, aryl, or substituted aryl.

15. The compound of Claim 4 wherein R₃ is CO₂R₅, (C₁-C₆ alkyl)CO₂R₅, CO(C₁-C₆ alkyl)CO₂R₅, or CONH(C₁-C₆ alkyl)CO₂R₅ wherein R₅ is hydrogen, C₁-C₆ alkyl, aryl, or substituted aryl.

16. The compound of Claim 4 wherein R₃ is (C₁-C₆ alkyl)CH(NHR₄)CO₂R₅, CO(C₁-C₆ alkyl)CH(NHR₄)CO₂R₅, or CONH(C₁-C₆ alkyl)CH(NHR₄)CO₂R₅,
wherein R₄ is SO₂(C₁-C₆ alkyl), SO₂ aryl, or SO₂ substituted aryl; and R₅ is hydrogen, C₁-C₆ alkyl, aryl, or substituted aryl.

17. The compound of Claim 5 wherein R₃ is CO₂R₅, (C₁-C₆ alkyl)CO₂R₅, CO(C₁-C₆ alkyl)CO₂R₅ or CONH(C₁-C₆ alkyl)CO₂R₅ wherein R₅ is hydrogen, C₁-C₆ alkyl, aryl, or substituted aryl.

18. The compound of Claim 5 wherein R₃ is (C₁-C₆ alkyl)CH(NHR₄)CO₂R₅, CO(C₁-C₆ alkyl)CH(NHR₄)CO₂R₅, or CONH(C₁-C₆ alkyl)CH(NHR₄)CO₂R₅,
wherein R₄ is SO₂(C₁-C₆ alkyl), SO₂ aryl, or SO₂ substituted aryl; and R₅ is hydrogen, C₁-C₆ alkyl, aryl, or substituted aryl.

19. The compound of Claim 13 wherein R₅ is hydrogen.

20. The compound of Claim 14 wherein R₅ is hydrogen.

21. The compound of Claim 15 wherein R₅ is hydrogen.

22. The compound of Claim 16 wherein R₅ is hydrogen.

23. The compound of Claim 17 wherein R₅ is hydrogen.

24. The compound of Claim 18 wherein R₅ is hydrogen.

25. A compound of Claim 1 selected from the group consisting of: 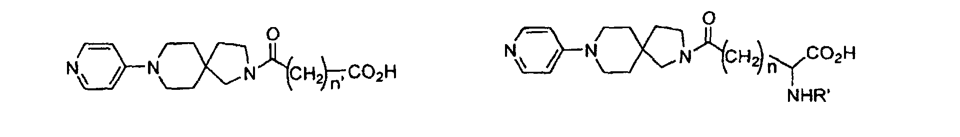 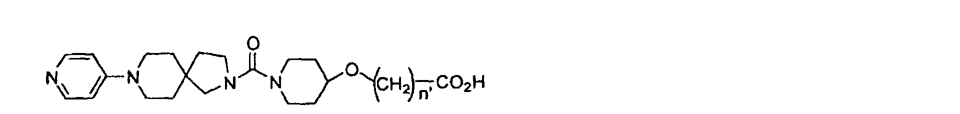 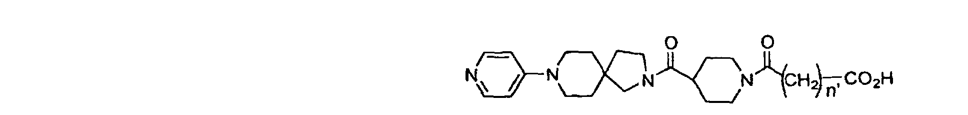 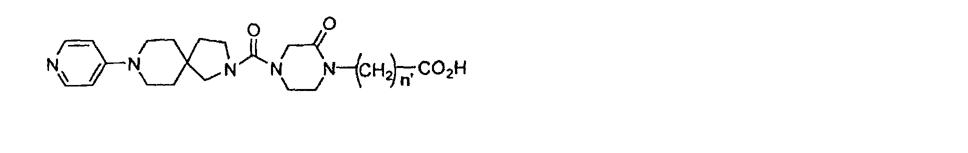 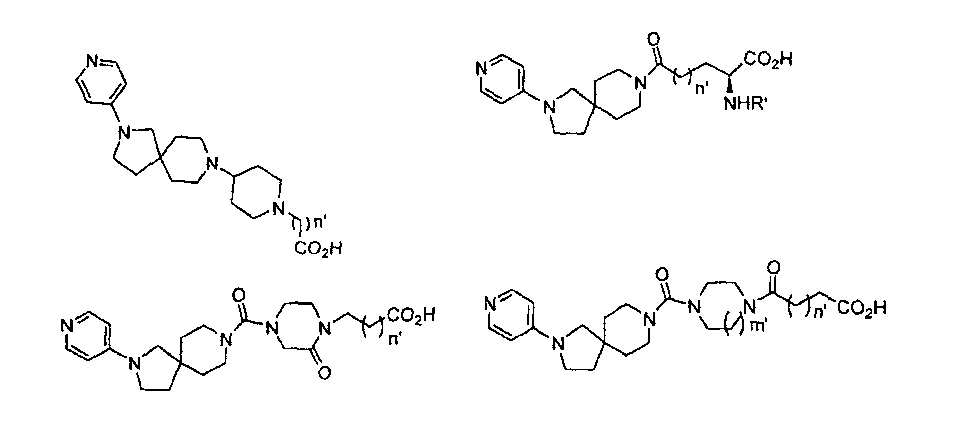 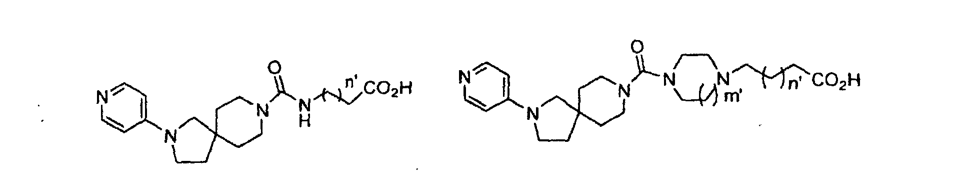 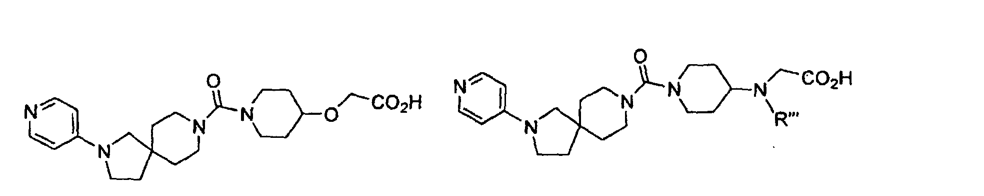 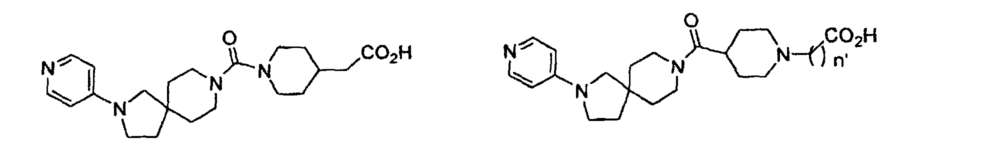 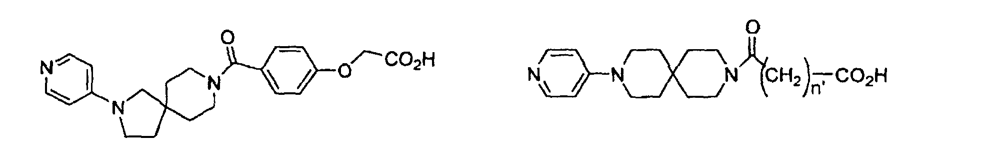 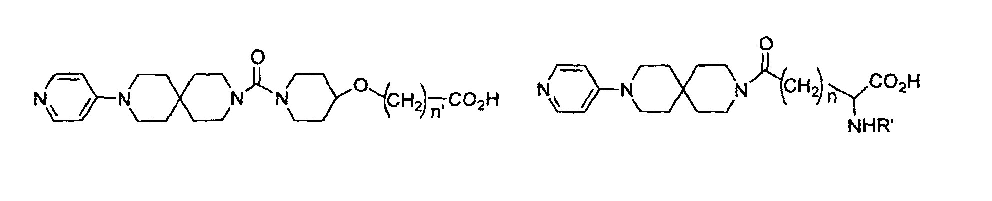 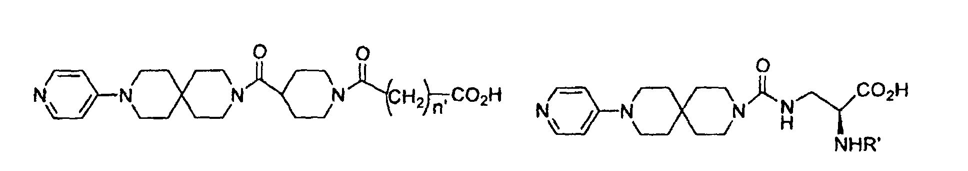 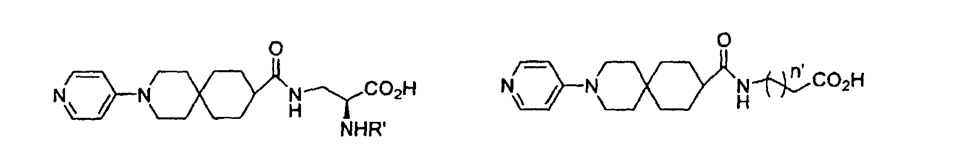 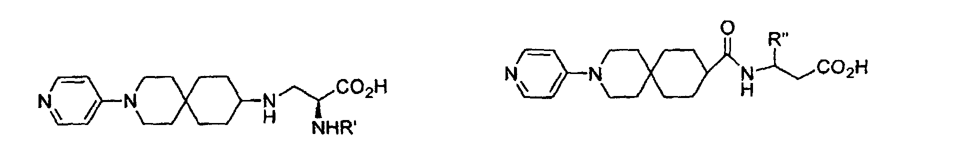 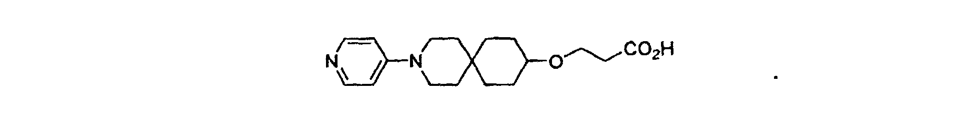 wherein n' is a number from 1 to 6; m' is a number from 1 to 2; R' is selected from the group consisting of CO₂-alkyl, CO₂-aryl, SO₂-alkyl, SO₂-aryl and SO₂-heteroaryl; R" is selected from the group consisting of alkyl, alkenyl, alkoxy, acyl, cycloalkyl, aryl, heteroaryl, which is substituted or unsubstituted; and R"' is selected from the group consisting of hydrogen, hydroxy, alkyl or substituted alkyl,
or a pharmaceutically acceptable salt, solvate, or prodrug thereof.

26. A composition for inhibiting the binding of fibrinogen to blood platelets in a mammal, comprising a compound of any one of Claims 1 to 25 and a pharmaceutically-acceptable carrier.

27. A composition for inhibiting the aggregation of blood platelets in a mammal, comprising a compound of any one of Claims 1 to 25 and a pharmaceutically-acceptable carrier.

28. A composition for preventing or treating thrombosis in a mammal, comprising a compound of any one of Claims 1 to 25 and a pharmaceutically-acceptable carrier.

29. A composition for treating a mammal, including man, to alleviate the pathological effects of atherosclerosis, arteriosclerosis, acute myocardial infarction, chronic stable angina, unstable angina, transient ischemic attacks and strokes, peripheral vascular disease, arterial thrombosis, preeclampsia, embolism, restenosis following angioplasty, carotid endarterectomy, and anastomosis of vascular grafts, comprising a compound of any one of Claims 1 to 25 and a pharmaceutically-acceptable carrier.

30. A pharmaceutical formulation containg as an active ingredient a compound as claimed in any one of Claims 1 to 25, associated with one or more pharmaceutically-acceptable carriers therefor.

31. The use of a compound as claimed in any one of Claims 1 to 25 for the manufacture of a medicament for the treatment of atherosclerosis, arteriosclerosis, acute myocardial infarction, chronic stable angina, unstable angina, transient ischemic attacks and strokes, peripheral vascular disease, arterial thrombosis, preeclampsia, embolism, restenosis following angioplasty, carotid endarterectomy, and anastomosis of vascular grafts.

32. The use of a compound as claimed in any one of Claims 1 to 25 for the manufacture of a medicament for the treatment of thrombosis.

## Patentansprüche

1. Eine Verbindung gemäß der Formel (I) oder ein pharmazeutisch annehmbares Salz, Solvat oder Pro-Pharmakon davon: 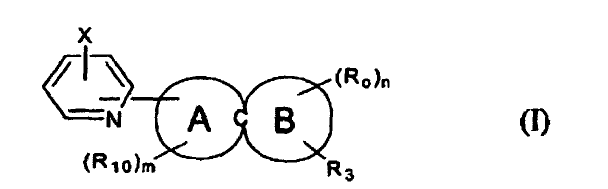 wobei:
der spirocyclische Kern A/B ausgesucht ist aus der Gruppe bestehend aus: 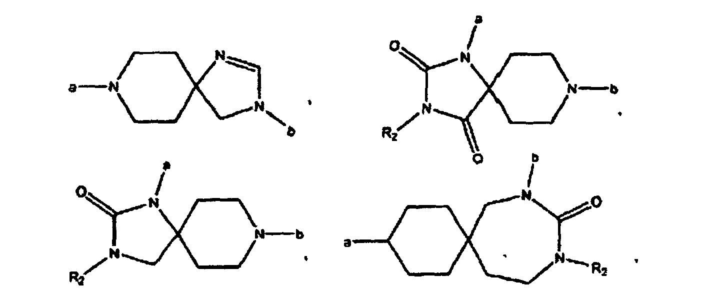 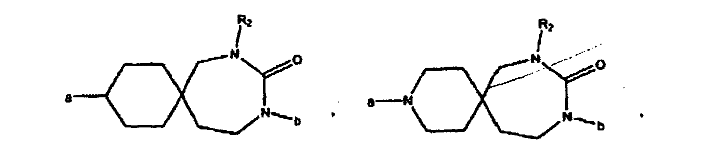 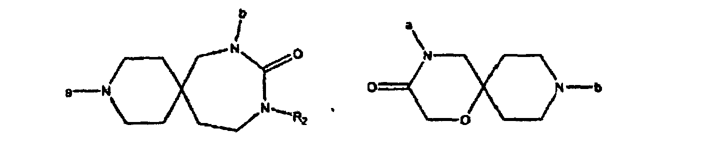 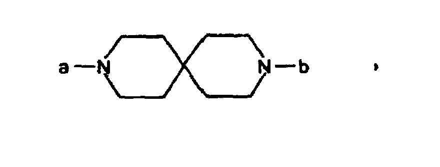 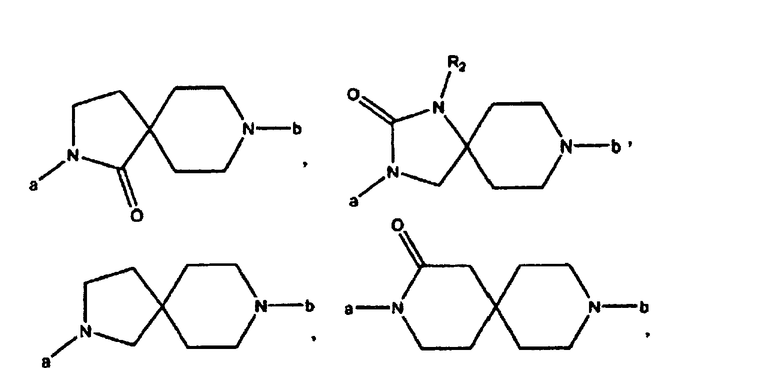 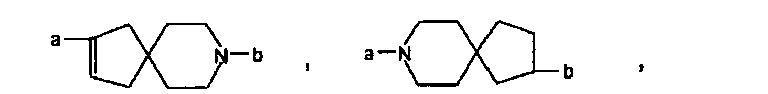 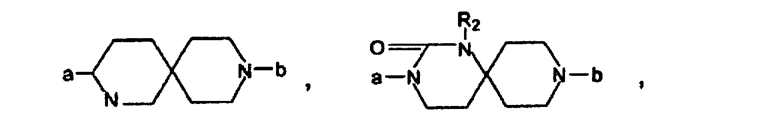 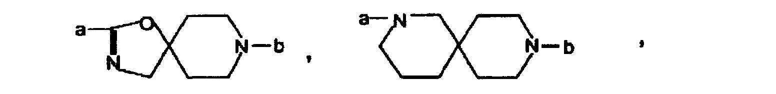 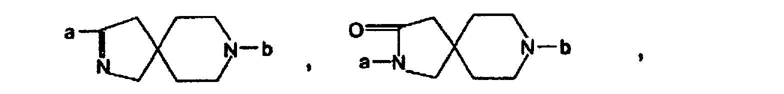 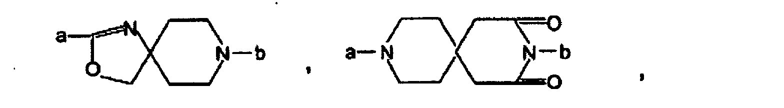 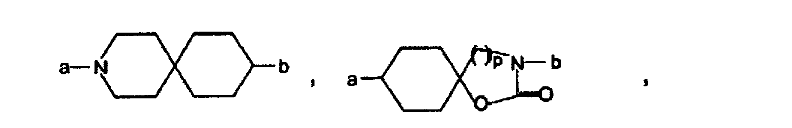 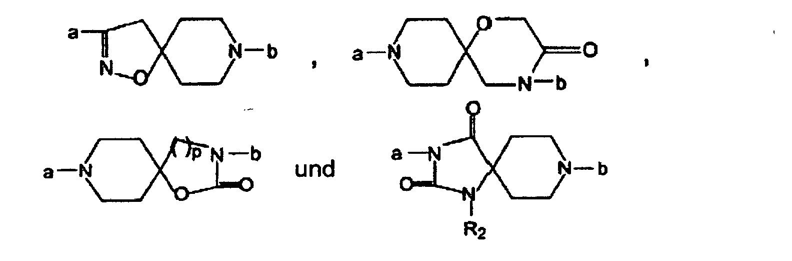
wobei
p eine Zahl von 1 bis 2 ist,
einer der "a" und "b" Anbindungspunkte des spirocyclischen Kerns an ein Ringkohlenstoffatom der Pyridylgruppe gebunden ist während die andere an die R₃-Gruppe gebunden ist, und
R₂ eine R₁₀-Gruppe ist, wenn der "a" Anbindungspunkt an die Pyridylgruppe gebunden ist, andernfalls ist R₂ eine R₀-Gruppe,
R₁₀ gleich oder unterschiedlich ist und ein nicht interferierender Substituent ist, der unabhängig ausgewählt ist aus Wasserstoff, Alkylreste, halogensubstituierten Alkylresten, Alkenylresten, Alkinylresten, Cycloalkylresten, Arylresten, Arlyalkylresten, Hydroxylresten, Alkoxyresten, Arylalkoxyresten, Aminogruppe, substituierten Aminoresten, Carbamoylresten, Carboxylresten, Acylresten, Cyanogruppe, Halogen, Nitrogruppe, Sulforesten, =O oder =S, mit der Bedingung, dass nur ein R₁₀ =O oder =S sein darf,
m eine Zahl von Null bis 9 ist,
R₀ gleich oder unterschiedlich ist und ein nicht interferierender Substituent ist, der unabhängig ausgesucht ist aus Wasserstoff, Alkylresten, halogensubstituierten Alkylresten, Alkenylresten, Alkinylresten, Cycloalkylresten, Arylresten, Arlyalkylresten, Hydroxylresten, Alkoxyresten, Arylalkoxyresten, Aminoresten, substituierten Aminoresten, Carbamoylresten, Carboxylresten, Acylresten, Cyanoresten, Halogen, Nitroresten, Sulforesten, =O oder =S, mit der Bedingung, dass nur ein R₀ =O oder =S sein darf,
n eine Zahl von Null bis 9 ist,
X ein Substituent ist ausgesucht aus der Gruppe bestehend aus Wasserstorf, Halogen, C₁₋₆-Alkylresten, C₁₋₆-Alkoxyresten, C₂₋₆-Alkenylresten, C₂₋₆-Alkinylresten,C₃₋₈-Cycloalkylresten, C₀₋₄-Alkyl-C₃₋₈-Cycloalkylresten, -CN, -NO₂, -(CH₂)ⱼ-N(-R^{a},-R^{b}),-C(=O)-N(-R^{a},-R^{b}), -S(=O)₂-N(-R^{a},-R^{b}), -S(=O)₂-R^{a}, -CF₃, und -(CH₂)ⱼ-O-R^{a},
wobei
R^{a} und R^{b} unabhängig ausgesucht sind aus der Gruppe bestehend aus H, -C₁₋₆-Alkylresten, C₁₋₆-Alkoxyresten, C₂₋₆-Alkenylresten, C₂₋₆-Alkinylresten, C₃₋₈-Cycloalkylresten, C₀₋₆-Alkyl-C₃₋₈-Cycloalkylresten und -C₀₋₄-Alkyl-(carbocyclisches Aryl)resten, wobei von 0-4 Wasserstoffatome an den Ringatomen der carbocyclischen Arylgruppe unabhängig voneinander durch ein Mitglied ausgesucht aus der Gruppe bestehend aus Halogen, C₁₋₄-Alkylresten, C₂₋₆-Alkenylresten, C₂₋₆-Alkinylresten, C₃₋₈-Cycloalkylresten, C₀₋₄-Alkyl-C₃₋₈-Cycloalkylresten, -CN, -CF₃ und -NO₂ ausgetauscht sein können,
und
j eine Zahl von 0 bis 2 ist,
und R₃ eine saure Gruppe ist, die ein oder mehrere Säureradikale umfasst.

2. Spirocyclische Verbindung gemäß Anspruch 1, wobei der A/B-Kern ausgesucht ist aus der Gruppe bestehend aus:

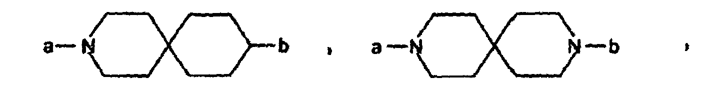

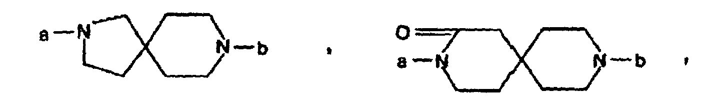

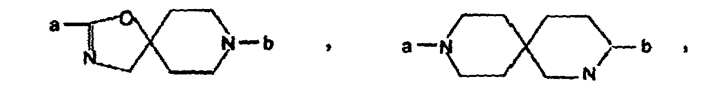

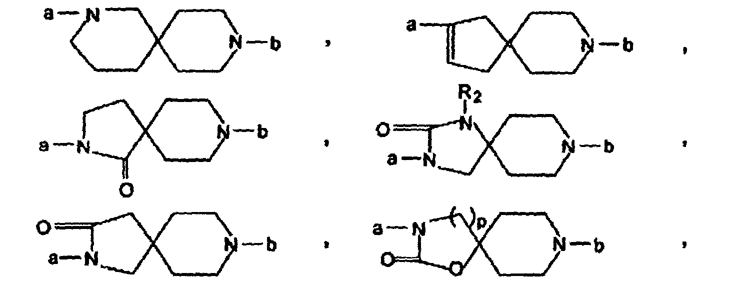

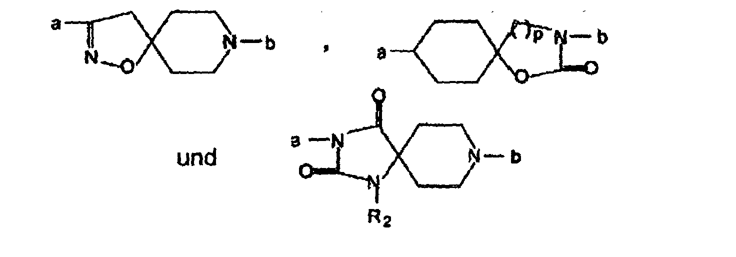

3. Spirocyclische Verbindung gemäß Anspruch 1, wobei der A/B-Kern ausgesucht ist aus der Gruppe bestehend aus:

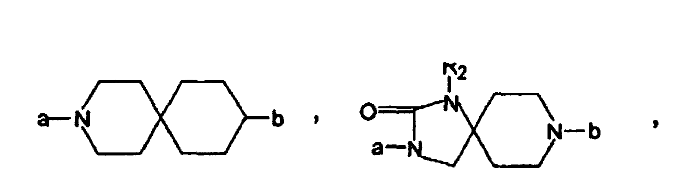

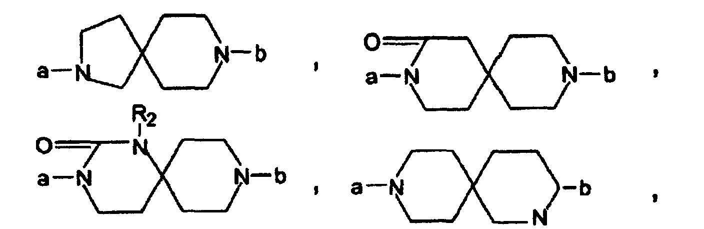

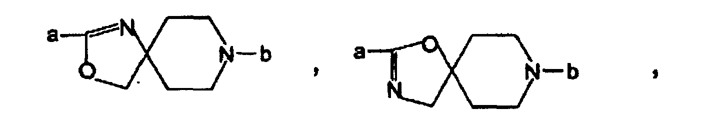

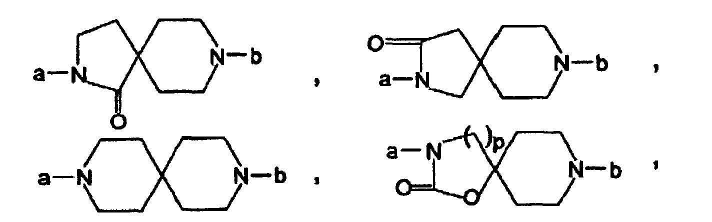

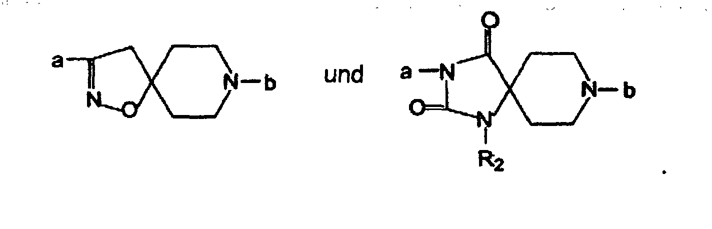

4. Spirocyclische Verbindung gemäß Anspruch 1, wobei der A/B-Kern ausgesucht ist aus der Gruppe bestehend aus:

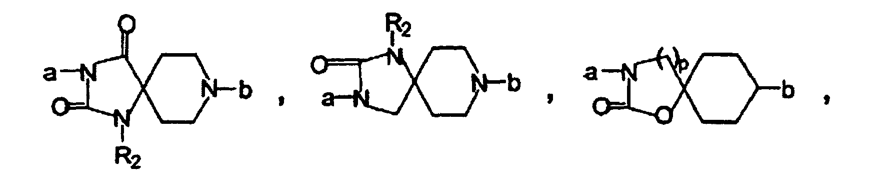

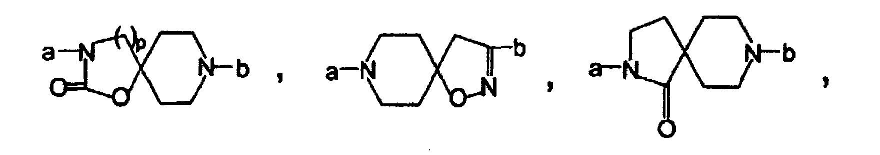

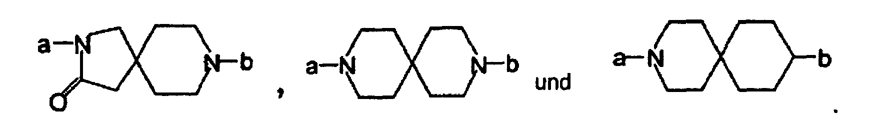

5. Verbindung gemäß Anspruch 4, wobei der "a"-Anbindungspunkt des spyrocyclischen Kerns mit der Pyridylgruppe verbunden ist.

6. Verbindung gemäß Anspruch 4, wobei der "b"-Anbindungspunkt des spyrocyclischen Kerns mit der Pyridylgruppe verbunden ist.

7. Verbindung gemäß Anspruch 1, wobei das Pyridylradikal ein Mitglied ist ausgesucht aus der Gruppe bestehend aus 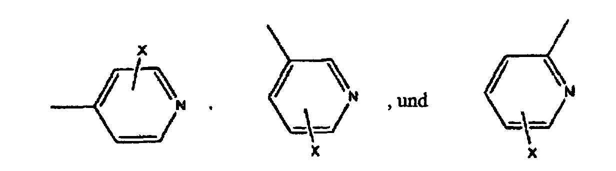 und X ein Mitglied ausgesucht aus der Gruppe bestehend aus Wasserstoff,
Niederalkylresten, Halogen und Trihalogenmethlyresten ist.

8. Verbindung gemäß Anspruch 2, wobei das Pyridylradikal ein Mitglied ist aus de Gruppe bestehend aus: 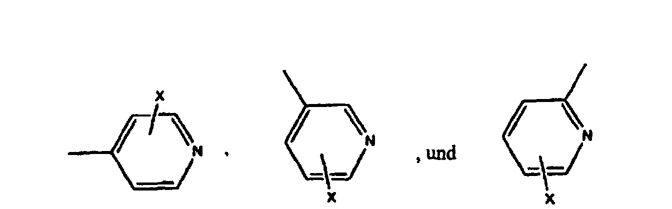 und X ein Mitglied ausgesucht aus der Gruppe bestehend aus Wasserstoff, Niederalkylresten, Halogen und Trihalogenmethlyresten ist.

9. Verbindung gemäß Anspruch 3, wobei das Pyridylradikal ein Mitglied ist aus der Gruppe bestehend aus 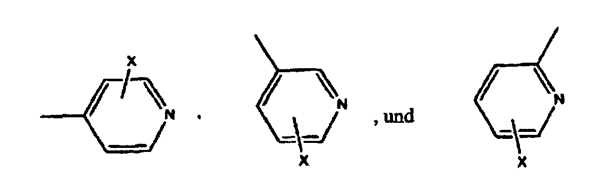 und X ein Mitglied ausgesucht aus der Gruppe bestehend aus Wasserstoff, Niederalkykesten, Halogen und Trihalogenmethlyresten ist.

10. Verbindung gemäß Anspruch 4, wobei das Pyridylradikal ein Mitglied ist aus der Gruppe bestehend aus 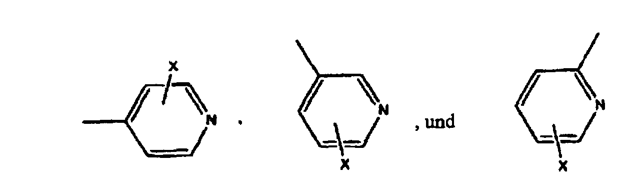 und X ein Mitglied ausgesucht aus der Gruppe bestehend aus Wasserstoff, Niederalkylresten, Halogen und Trihalogenmethlyresten ist.

11. Verbindung gemäß Anspruch 10, wobei das Pyridylradikal 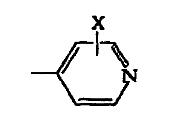 ist.

12. Verbindung gemäß Anspruch 11, wobei das Pyridylradikal 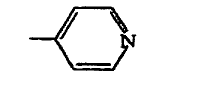 ist.

13. Verbindung gemäß Anspruch 1, wobei R₃ CO₂R₅, (C₁-C₆-Alkyl)CO₂R₅, CO(C₁-C₆-Alkyl)CO₂R₅ oder CONH(C₁-C₆-Alkyl)CO₂R₅ ist, wobei R₅ Wasserstoff, ein C₁-C₆-Alkylrest, Arylrest oder substituierter Arylrest ist.

14. Verbindung gemäß Anspruch 1, wobei R₃ (C₁-C₆-Alkyl)CH(NHR₄)CO₂R₅, CO(C₁-C₆-Alkyl)CH(NHR₄)CO₂R₅ oder CONH(C₁-C₆-Alkyl)CH(NHR₄)CO₂R₅ ist, wobei R₄ SO₂(C₁-C₆-Alkyl), SO₂-Aryl, oder SO₂-substituiertes Aryl und R₅ Wasserstoff, ein C₁-C₆-Alkylrest, ein Arylrest oder ein substituierter Arylrest ist.

15. Verbindung gemäß Anspruch 4, wobei R₃ CO₂R₅, (C₁-C₆-Alkyl)CO₂R₅, CO(C₁-C₆-Alkyl)CO₂R₅ oder CONH(C₁-C₆-Alkyl)CO₂R₅ ist, wobei R₅ Wasserstoff, ein C₁-C₆-Alkylrest, Arylrest oder substituierter Arylrest ist.

16. Verbindung gemäß Anspruch 4, wobei R₃ (C₁ -C₆ -Alkyl)CH(NHR₄)CO₂R₅, CO(C₁-C₆-Alkyl)CH(NHR₄)CO₂R₅ oder CONH(C₁-C₆ -Alkyl)CH(NHR₄)CO₂R₅ ist, wobei R₄ SO₂(C₁-C₆-Alkyl), SO₂-Aryl, oder SO₂-substituiertes Aryl und R₅ Wasserstoff, ein C₁-C₆-Alkylrest, ein Arylrest oder ein substituierter Arylrest ist.

17. Verbindung gemäß Anspruch 5, wobei R₃ CO₂R₅, (C₁-C₆-Alkyl)CO₂R₅, CO(C₁-C₆-Alkyl)CO₂R₅ oder CONH(C₁ -C₆ -Alkyl)CO₂R₅ ist, wobei R₅ Wasserstoff, ein C₁-C₆-Alkylrest, Arylrest oder substituierter Arylrest ist.

18. Verbindung gemäß Anspruch 5, wobei R₃ (C₁ -C₆ -Alkyl)CH(NHR₄)CO₂R₅, CO(C₁-C₆-Alkyl)CH(NHR₄)CO₂R₅ oder CONH(C₁-C₆ -Alkyl)CH(NHR₄)CO₂R₅ ist, wobei R₄ SO₂(C₁-C₆-Alkyl), SO₂-Aryl, oder SO₂-substituiertes Aryl und R₅ Wasserstoff, ein C₁-C₆-Alkylrest, ein Arylrest oder ein substituierter Arylrest ist.

19. Verbindung gemäß Anspruch 13, wobei R₅ Wasserstoffist.

20. Verbindung gemäß Anspruch 14, wobei R₅ Wasserstoff ist.

21. Verbindung gemäß Anspruch 15, wobei R₅ Wasserstoff ist.

22. Verbindung gemäß Anspruch 16, wobei R₅ Wasserstoff ist.

23. Verbindung gemäß Anspruch 17, wobei R₅ Wasserstoff ist.

24. Verbindung gemäß Anspruch 18, wobei R₅ Wasserstoff ist.

25. Verbindung gemäß Anspruch 1, die ausgewählt ist aus der Gruppe bestehend aus: 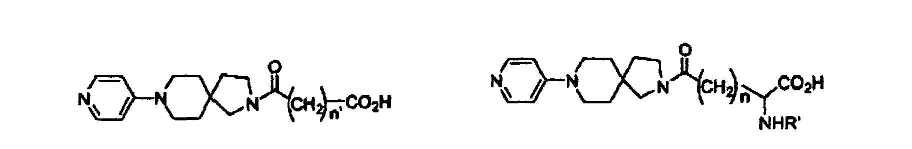 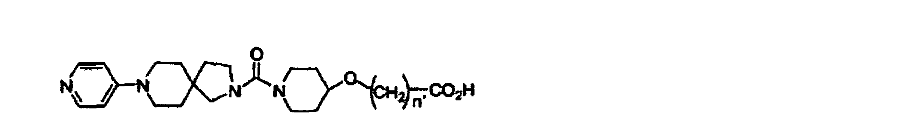 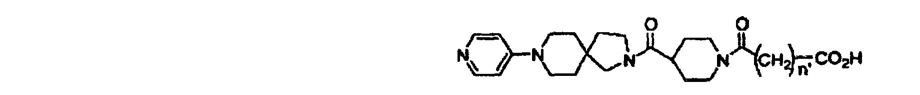 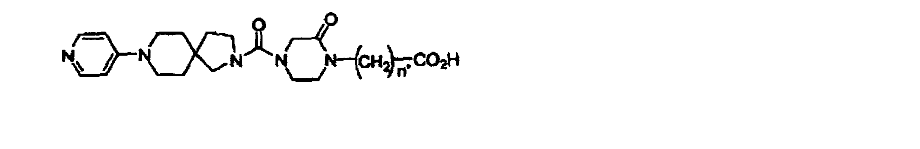 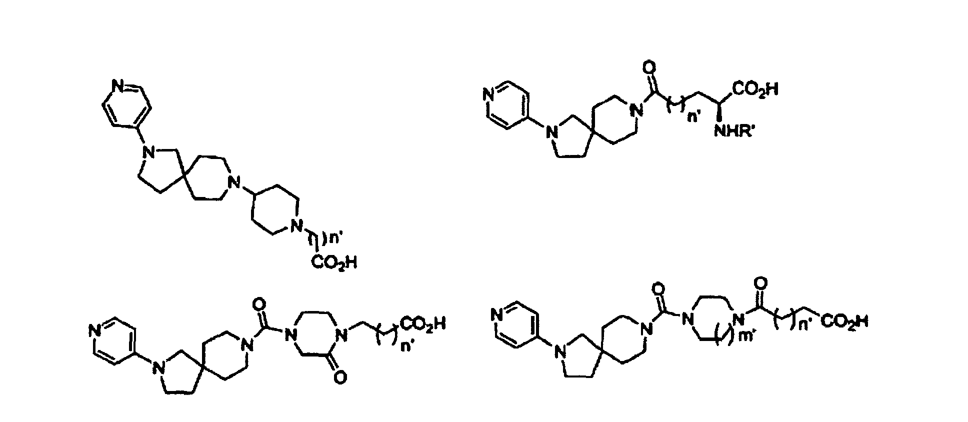 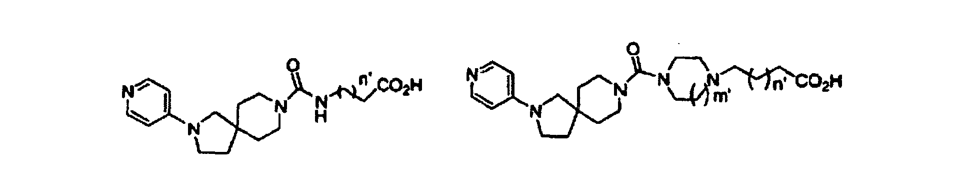 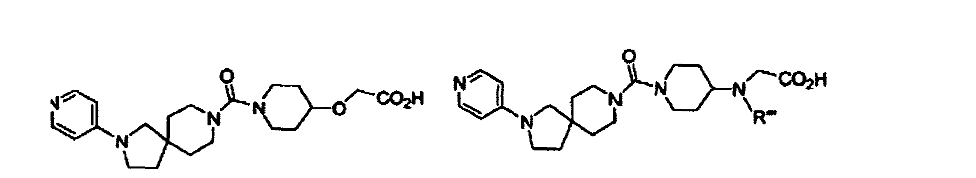 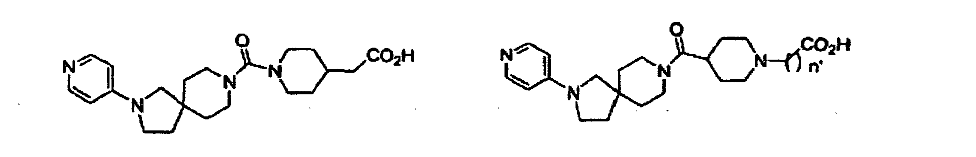 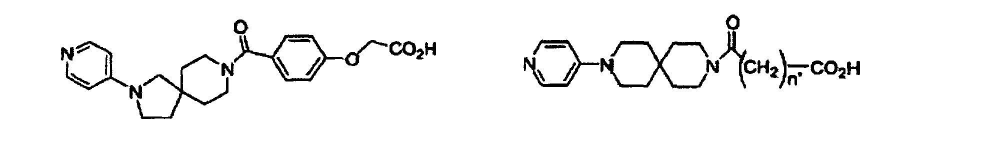 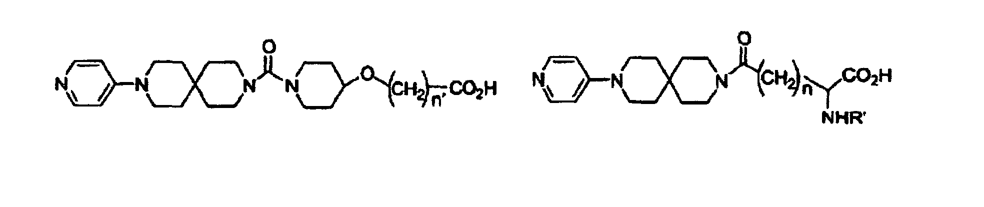 wobei n' eine Zahl von 1 bis 6 ist, m' eine Zahl von 1 bis 2 ist, R' ausgesucht ist aus der 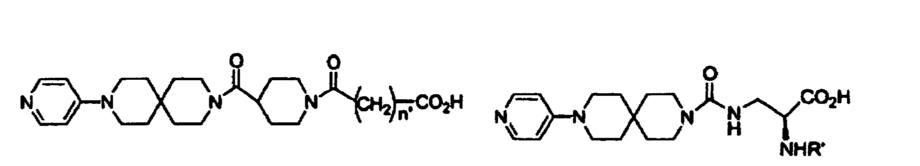 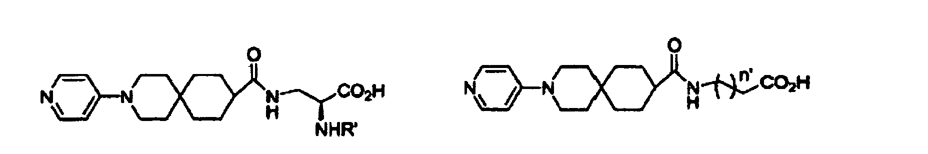 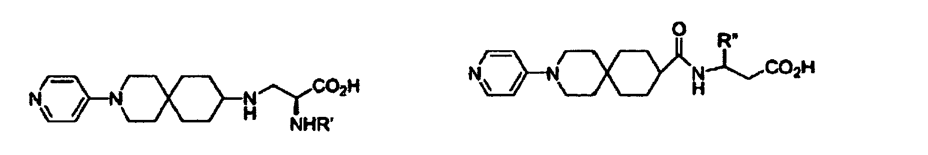 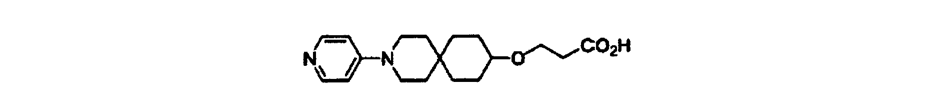 Gruppe bestehend aus CO₂-Alkyl, CO₂-Aryl, SO₂-Alkyl, SO₂-Aryl und SO₂-Heteroaryl, R" ausgesucht ist aus der Gruppe bestehend aus Alkyl-, Alkenyl-, Alkoxy-, Acyl-, Cycloalkyl-, Aryl-, Heteroarylresten, die substituiert oder unsubstituiert sein können, und R"' ausgesucht ist aus der Gruppe bestehend aus Wasserstoff, Hydroxyl-, Alkyl- oder substituierten Alkylresten,
oder ein pharmazeutisch annehmbares Salz, Solvat oder Pro-Pharmakon dieser Verbindungen.

26. Zusammensetzung zur Inhibierung der Bindung von Fibrinogen an Blutplättchen in einem Säugetier, umfassend eine Verbindung gemäß einem der Ansprüche 1 bis 25 und ein pharmazeutisch annehmbares Trägermittel.

27. Zusammensetzung zur Inhibierung der Blutplättchenaggregation in einem Säugetier, umfassend eine Verbindung gemäß einem der Ansprüche 1 bis 25 und ein pharmazeutisch annehmbares Trägermittel.

28. Zusammensetzung zur Verhinderung oder Behandlung von Thrombose in einem Säugetier, umfassend eine Verbindung gemäß einem der Ansprüche 1 bis 25 und ein pharmazeutisch annehmbares Trägermittel.

29. Zusammensetzung zur Behandlung eines Säugetiers, einschließlich eines Menschen, um die pathologischen Auswirkungen von Atherosklerose, Arterioskelrose, akutem myokardialem Infarkt, chronischer stabiler Angina, instabiler Angina, transiente ischämischer Anfälle und Schlaganfälle, periphere Gefäßerkrankungen, arterieller Thrombose, Präeklampsie, Embolie, Re-Stenose nach Angioplastie, Karotin-Endarterektomie und Anastomose vaskulärer Transplantate zu lindern, umfassend eine Verbindung gemäß einem der Ansprüche 1 bis 25 und ein pharmazeutisch annehmbares Trägermittel.

30. Pharmazeutische Formulierung, enthaltend als Wirkstoff eine Verbindung wie in einem der Ansprüche 1 bis 25 beansprucht in Verbindung mit einem oder mehreren pharmazeutisch annehmbaren Trägermittel für dieser Verbindung.

31. Verwendung einer der in einem der Ansprüche 1 bis 25 beanspruchten Verbindung zur Herstellung eines Arzneimittels zur Behandlung von Atherosklerose, Arterioskelrose, akutem myokardialem Infarkt, chronischer stabiler Angina, instabiler Angina, transiente ischämischer Anfälle und Schlaganfälle, periphere Gefäßerkrankungen, arterieller Thrombose, Präeklampsie, Embolie, Re-Stenose nach Angioplastie, Karotis-Endarterektomie und Anastomose vaskulärer Transplantate.

32. Verwendung einer der in einem der Ansprüche 1 bis 25 beanspruchten Verbindungen 1 bis 25 zur Herstellung eines Arzneimittels zur Behandlung von Thrombose.

## Revendications

1. Composé de formule (I) ou sel, solvate ou promédicament d'un tel composé : 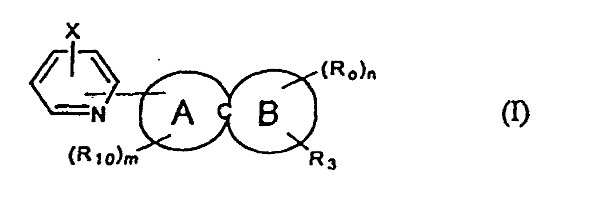 dans laquelle le noyau spirocyclique A/B est sélectionné dans le groupe consistant en : 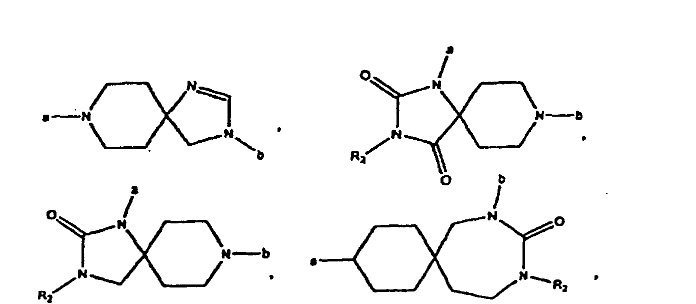 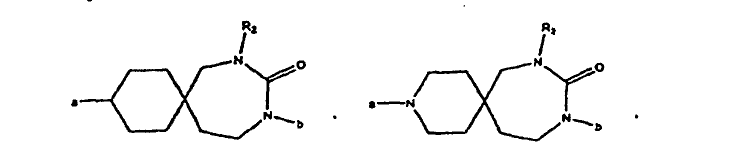 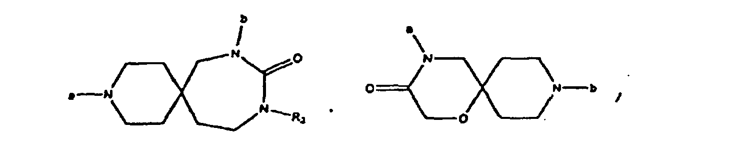 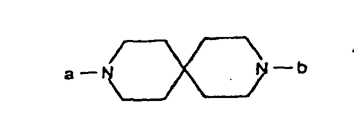 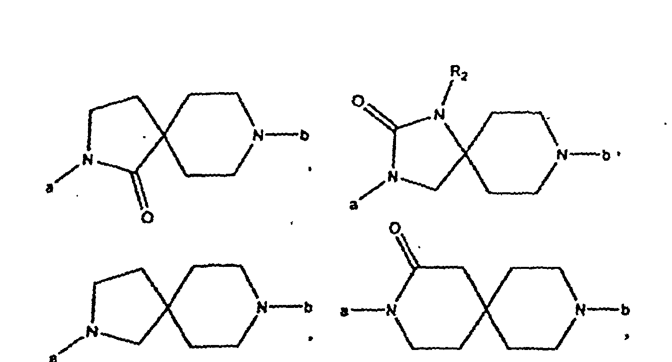 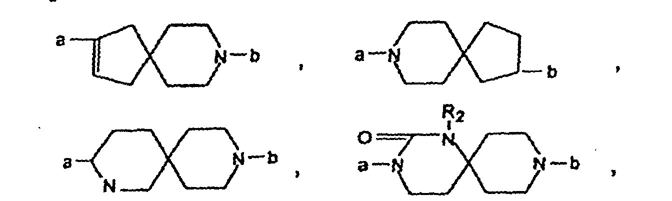 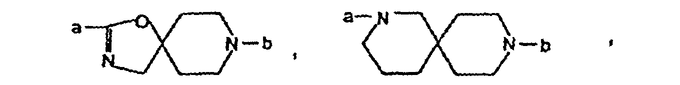 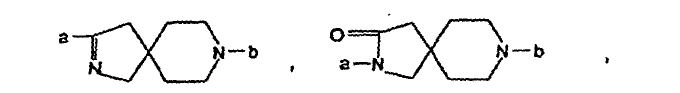 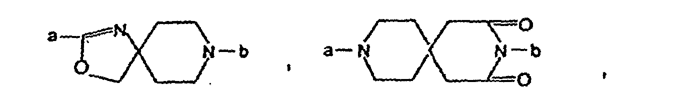 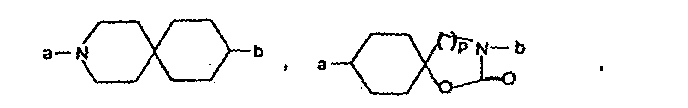 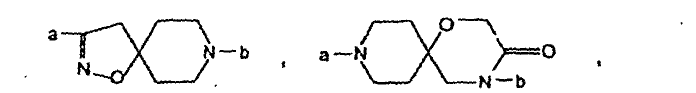 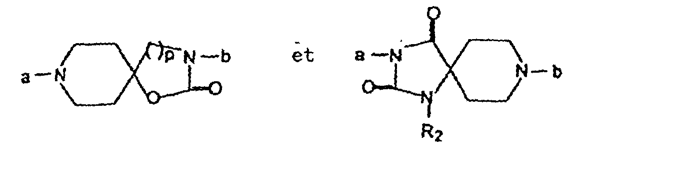
où p est un nombre allant de 1 à 2 ;
l'un des points de fixation "a" et "b" du noyau spirocyclique est fixé à un atome de carbone du cycle sur le groupe pyridyle tandis que l'autre est fixé au groupe R₃ ; et
R₂ est un groupe R₁₀ si le point de fixation "a" est fixé au groupe pyridyle ou, sinon, R₂ est un groupe R₀ ;
R₁₀ représente des radicaux identiques ou différents et est un substituant non-interférant sélectionné indépendamment parmi l'hydrogène, alkyle, alkyle halogéno-substitué, alkényle, alkynyle, cycloalkyle, aryle, arylalkyle, hydroxy, alkoxy, arylalkoxy, amino, amino substitué, carbamoyle, carboxy, acyle, cyano, halogéno, nitro, sulfo, =O ou =S, étant entendu que seul un R₁₀ peut représenter =O ou =S ;
m est un nombre allant de zéro à 9 ;
R₀ représente des radicaux identiques ou différents et est un substituant non-interférant, indépendamment sélectionné parmi l'hydrogène, alkyle, alkyle halogéno-substitué, alkényle, alkynyle, cycloalkyle, aryle, arylalkyle, hydroxy, alkoxy, arylalkoxy, amino, amino substitué, carbamoyle, carboxy, acyle, cyano, halogéno, nitro, sulfo, =O ou =S, étant entendu que un seul R₀peut représenter =O ou =S ;
n est un nombre allant de zéro à 9 ;
X est un substituant sélectionné dans le groupe consistant en l'hydrogène, halogéno, alkyle en C₁₋₆, alkoxy en C₁₋₆, alkényle en C₂₋₆, alkynyle en C₂₋₆, cycloalkyle en C₃₋₈, (alkyle en C₀₋₄)cycloalkyle en C₃₋₈, -CN, -NO₂, (CH₂)ⱼ-N(-R^{a}, -R^{b}), -C (=O) -N (-R^{a},-R^{b}), -S (=O)₂-N (-R^{a}, -R^{b}) , -S (=O)₂-R^{a}, -CF₃ et -(CH₂)ⱼ-O-R^{a};
où
R^{a} et R^{b} sont indépendamment sélectionnés dans le groupe consistant en H, alkyle en C₁₋₆, alkoxy en C₁₋₆, alkényle en C₂₋₆, alkynyle en C₂₋₆, cycloalkyle en C₃₋₈, (alkyle en C₀₋₆)cycloalkyle en C₃₋₈, et (alkyle en C₀₋₆)-(aryle carbocyclique) , où de 0 à 4 atomes d'hydrogène sur les atomes du cycle du motif aryle carbocyclique peuvent être indépendamment remplacés par un élément sélectionné dans le groupe consistant en halogéno, alkyle en C₁₋₄, alkényle en C₂₋₆, alkynyle en C₂₋₆, cycloalkyle en C₃₋₈, (alkyle en C₀₋₄)cycloalkyle en C₃₋₈, -CN, -CF₃ et -NO₂ ; et
j est un nombre allant de 0 à 2 ;
et
R3 est un groupe acide contenant un ou plusieurs radicaux acides.

2. Composé spirocyclique selon la revendication 1, dans lequel le noyau A/B est sélectionné dans le groupe consistant en :

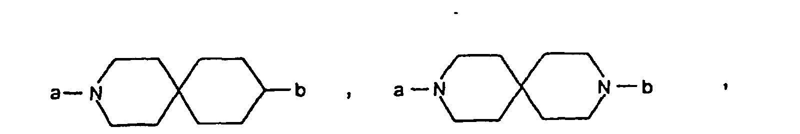

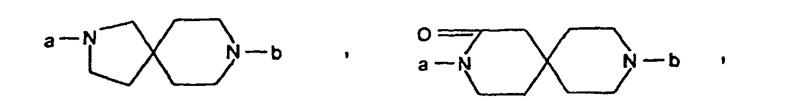

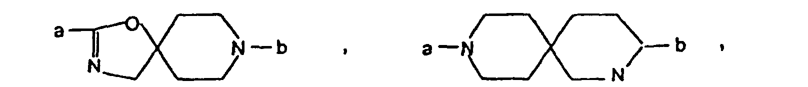

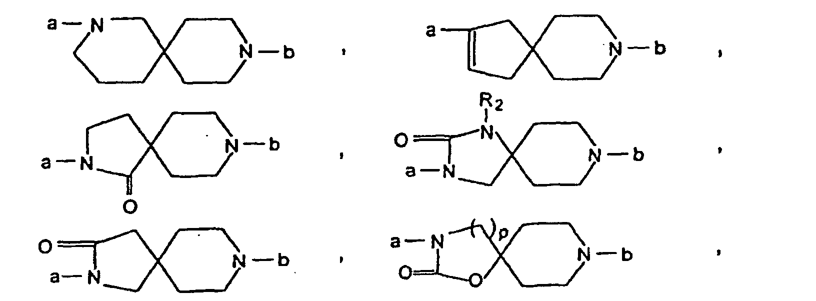

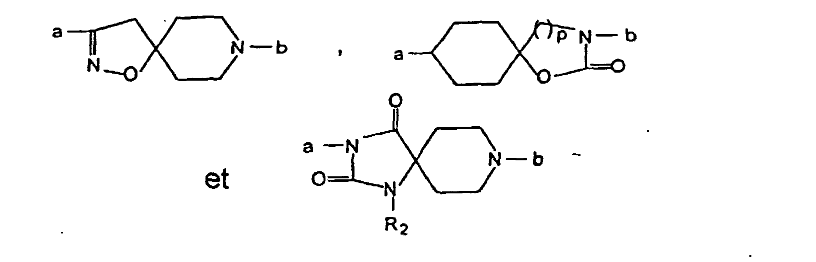

3. Composé spirocyclique selon la revendication 1, dans lequel le noyau A/B est sélectionné dans le groupe consistant en :

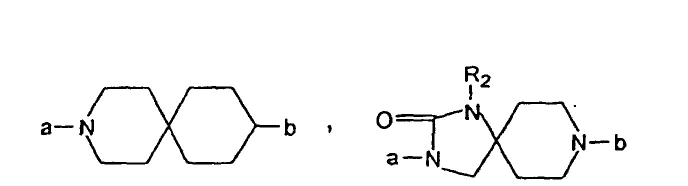

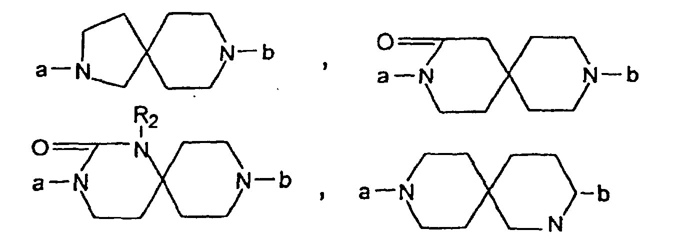

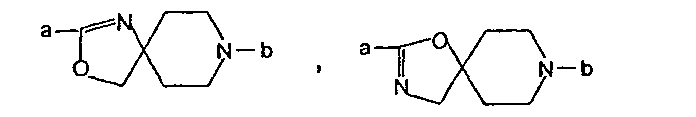

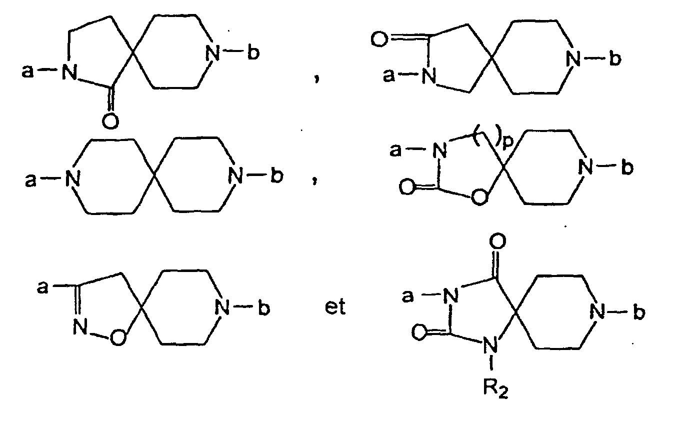

4. Composé spirocyclique selon la revendication 1, dans lequel le noyau A/B est sélectionné dans le groupe consistant en :

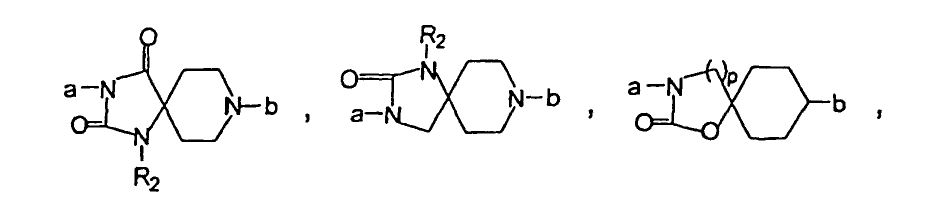

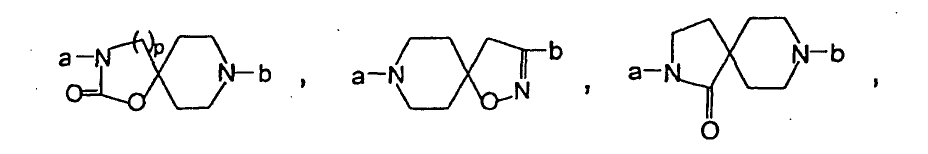

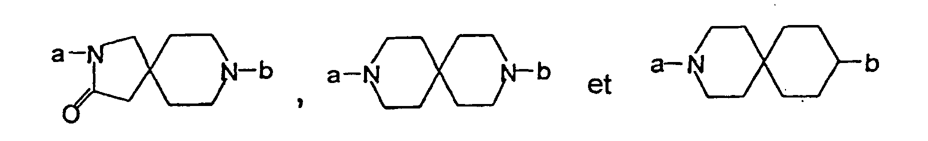

5. Composé selon la revendication 4, dans lequel le point de fixation "a" du noyau spirocyclique est fixé au groupe pyridyle.

6. Composé selon la revendication 4, dans lequel le point de fixation "b" du noyau spirocyclique est fixé au groupe pyridyle.

7. Composé selon la revendication 1, dans lequel :
le radical pyridyle est un élément sélectionné dans le groupe consistant en : 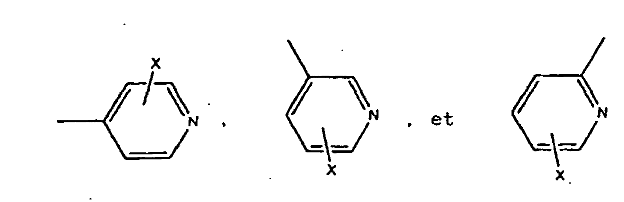
et X est un élément sélectionné dans le groupe consistant en l'hydrogène, alkyle inférieur, halogéno et trihalogénométhyle.

8. Composé selon la revendication 2, dans lequel :
le radical pyridyle est un élément sélectionné dans le groupe consistant en : 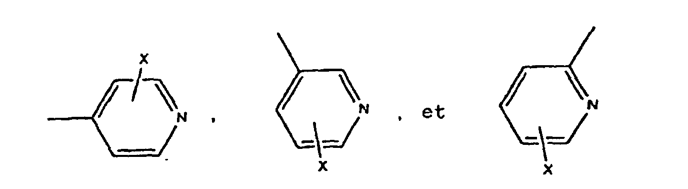
et X est un élément sélectionné dans le groupe consistant en l'hydrogène, alkyle inférieur, halogéno et trihalogénométhyle.

9. Composé selon la revendication 3 dans lequel :
le radical pyridyle est un élément sélectionné dans le groupe consistant en : 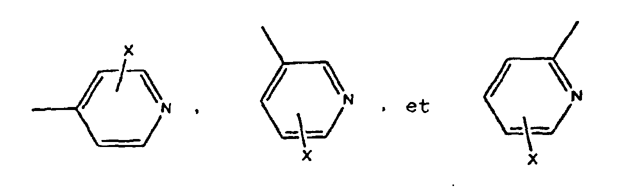
et X est un élément sélectionné dans le groupe consistant en l'hydrogène, alkyle inférieur, halogéno et trihalogénométhyle.

10. Composé selon la revendication 4, dans lequel :
le radical pyridyle est un élément sélectionné dans le groupe consistant en : 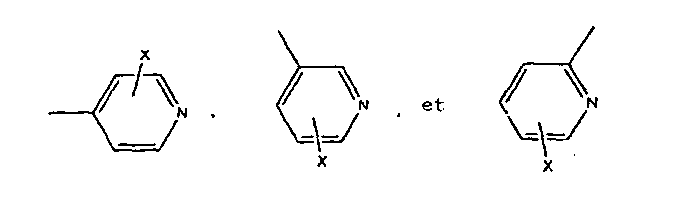
et X est un élément sélectionné dans le groupe consistant en l'hydrogène, alkyle inférieur, halogéno et trihalogénométhyle.

11. Composé selon la revendication 10, dans lequel le radical pyridyle est :

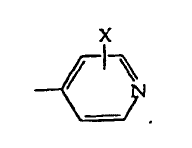

12. Composé selon la revendication 11, dans lequel le radical pyridyle est :

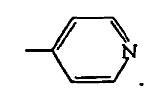

13. Composé selon la revendication 1, dans lequel R₃ représente CO₂R₅, (alkyle en C₁-C₆) CO₂R₅, CO(alkyle en C₁-C₆)CO₂R₅ ou CONH(alkyle en C₁-C₆) CO₂R₅, où R₅ représente l'hydrogène, alkyle en C₁-C₆, aryle ou aryle substitué.

14. Composé selon la revendication 1, dans lequel R₃ représente (alkyle en C₁-C₆) CH(NHR₄) CO₂R₅, CO (alkyle en C₁-C₆) CH (NHR₄) CO₂R₅ ou CONH (alkyle en C₁-C₆) CH (NHR₄) CO₂R₅,
où R₄ représente SO₂ (alkyle en C₁-C₆) , SO₂ aryle ou SO₂ aryle substitué ; et R₅ représente l'hydrogène, alkyle en C₁-C₆, aryle ou aryle substitué.

15. Composé selon la revendication 4, dans lequel R₃ représente CO₂R₅, (alkyle en C₁-C₆) CO₂R₅, CO (alkyle en C₁-C₆)CO₂R₅ ou CONH(alkyle en C₁-C₆)CO₂R₅, où R₅ représente l'hydrogène, alkyle en C₁-C₆, aryle ou aryle substitué.

16. Composé selon la revendication 4, dans lequel R₃ représente (alkyle en C₁-C₆) CH (NHR₄) CO₂R₅, CO(alkyle en C₁-C₆) CH (NHR₄) CO₂R₅ ou CONH (alkyle en C₁-C₆) CH (NHR₄) CO₂R₅,
où R₄ représente SO₂ (alkyle en C₁-C₆), SO₂-aryle ou SO₂-aryle substitué ; et R₅ représente l'hydrogène, alkyle en C₁-C₆, aryle ou aryle substitué.

17. Composé selon la revendication 5, dans lequel R₃ représente CO₂R₅, (alkyle en C₁-C₆)CO₂R₅, CO(alkyle en C₁-C₆)CO₂R₅ ou CONH (alkyle en C₁-C₆) CO₂R₅, où R₅ représente l'hydrogène, alkyle en C₁-C₆, aryle ou aryle substitué.

18. Composé selon la revendication 5, dans lequel R₃ représente (alkyle en C₁-C₆)CH(NHR₄) CO₂R₅, CO (alkyle en C₁-C₆) CH (NHR₄) CO₂R₅ ou CONH (alkyle en C₁-C₆) CH (NHR₄) CO₂R₅,
où R₄ représente SO₂ (alkyle en C₁-C₆), SO₂₋aryle ou SO₂₋aryle substitué ; et R₅ représente l'hydrogène, alkyle en C₁-C₆, aryle ou aryle substitué.

19. Composé selon la revendication 13, dans lequel R₅ représente l'hydrogène.

20. Composé selon la revendication 14, dans lequel R₅ représente l'hydrogène.

21. Composé selon la revendication 15, dans lequel R₅ représente l'hydrogène.

22. Composé selon la revendication 16, dans lequel R₅ représente l'hydrogène.

23. Composé selon la revendication 17, dans lequel R₅ représente l'hydrogène.

24. Composé selon la revendication 18, dans lequel R₅ représente l'hydrogène.

25. Composé selon la revendication 1, sélectionné dans le groupe consistant en : 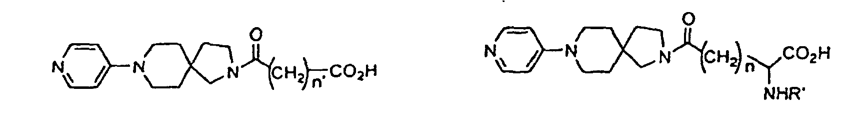 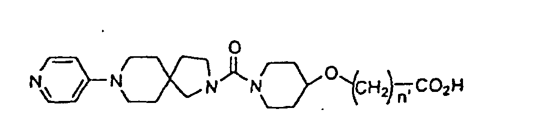 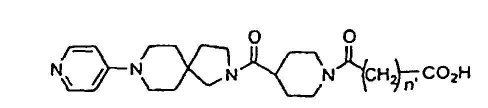 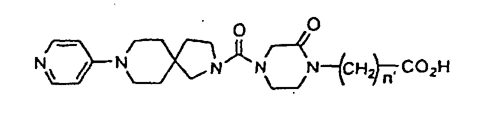 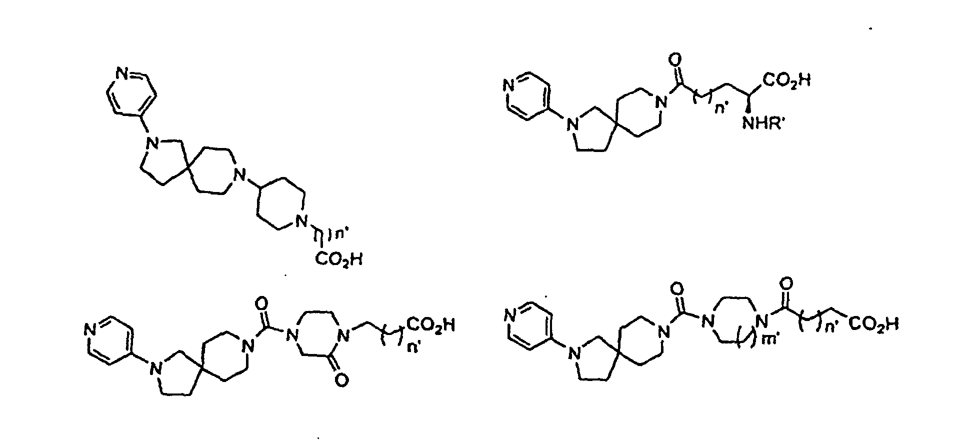 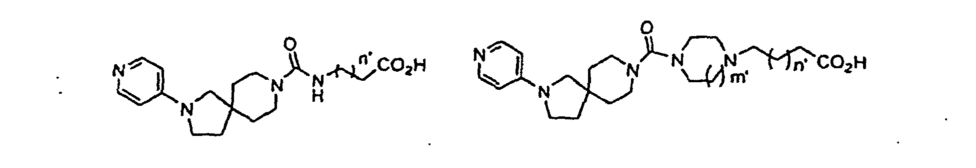 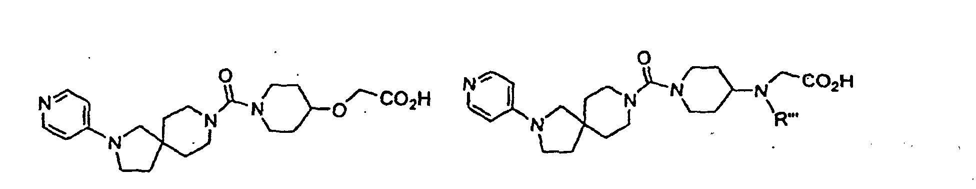 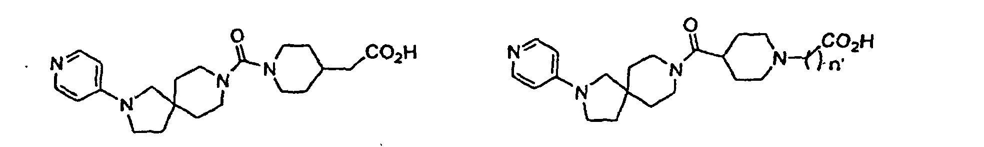 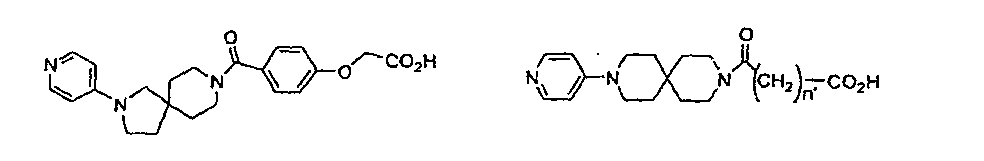 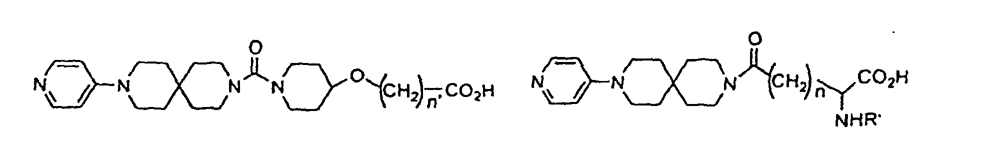 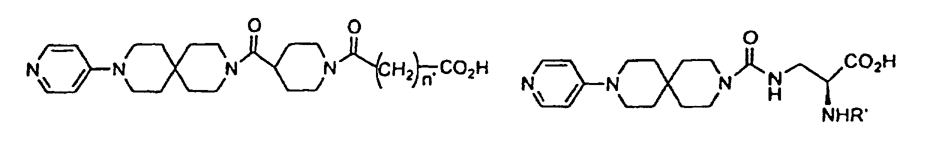 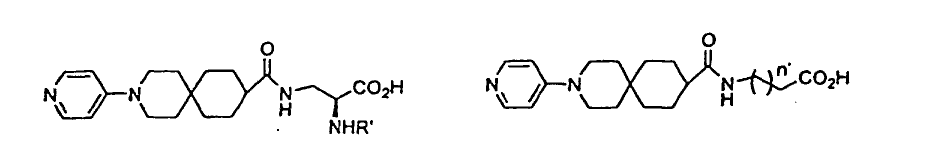 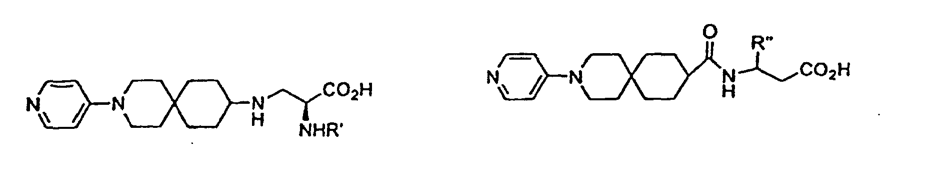 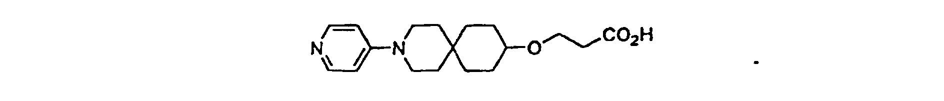 dans lesquelles n' est un nombre allant de 1 à 6 ; m' est un nombre allant de 1 à 2 ; R' est sélectionné dans le groupe consistant en CO₂-alkyle, CO₂-aryle, SO₂-alkyle, SO₂-aryle et SO₂-hétéroaryle ; R' ' est sélectionné dans le groupe consistant en alkyle, alkényle, alkoxy, acyle, cycloalkyle, aryle, hétéroaryle, qui est substitué ou non-substitué ; et R' ' ' est sélectionné dans le groupe consistant en l'hydrogène, hydroxy, alkyle ou alkyle substitué,
ou un sel, un solvate ou un promédicament pharmaceutiquement acceptable d'un tel composé.

26. Composition pour inhiber la liaison du fibrinogène aux plaquettes sanguines chez un mammifère, comprenant un composé selon l'une quelconque des revendications 1 à 25 et un support pharmaceutiquement acceptable.

27. Composition pour inhiber l'agrégation des plaquettes sanguines chez un mammifère, comprenant un composé selon l'une quelconque des revendications 1 à 25 et un support pharmaceutiquement acceptable.

28. Composition pour prévenir ou traiter la thrombose chez un mammifère, comprenant un composé selon l'une quelconque des revendications 1 à 25 et un support pharmaceutiquement acceptable.

29. Composition pour traiter un mammifère, y compris l'homme, pour soulager les effets pathologiques de l'athérosclérose, l'artériosclérose, l'infarctus aigu du myocarde, l'angor chronique stable, l'angor instable, les attaques ischémiques passagères, la maladie vasculaire périphérique, la thrombose artérielle, la prééclampsie, l'embolie, la resténose suivant une angioplastie, l'endartérectomie de la carotide, et l'anastomose des greffes vasculaires, comprenant un composé selon l'une quelconque des revendications 1 à 25 et un support pharmaceutiquement acceptable.

30. Formulation pharmaceutique contenant comme principe actif un composé selon l'une quelconque des revendications 1 à 25, associée à un ou plusieurs supports pharmaceutiquement acceptables.

31. Utilisation d'un composé selon l'une quelconque des revendications 1 à 25, pour la fabrication d'un médicament pour le traitement de l'athérosclérose, l'artériosclérose, l'infarctus aigu du myocarde, l'angor chronique stable, l'angor instable, les attaques ischémiques passagères, la maladie vasculaire périphérique, la thrombose artérielle, la prééclampsie, l'embolie, la resténose suivant une angioplastie, l'endartérectomie de la carotide, et l'anastomose des greffes vasculaires.

32. Utilisation d'un composé selon l'une quelconque des revendications 1 à 25 pour la fabrication d'un médicament pour le traitement de la thrombose.
